# EUROPEAN PATENT SPECIFICATION

(11) **EP 1 487 486 B1**
(45) Date of publication and mention of the grant of the patent: **14.01.2009**
(21) Application number: 03712391.6
(22) Date of filing: 19.03.2003
(51) Int. Cl.: A61K 39/39

(54) **IMIDAZOQUINOLINEAMINES AS ADJUVANTS IN HIV DNA VACCINATION**
IMIDAZOQUINOLINAMINE ALS ADJUVANTIEN FÜR HIV DNA VAKZINE
IMIDAZOQUINOLINEAMINES COMME ADJUVANTS DANS LA VACCINATION D'ADN D'HIV

(30) Priority: 19.03.2002 US 102622; 19.03.2002 US 366058 P
(43) Date of publication of application: 22.12.2004
(73) Proprietor: PowderJect Research Limited, Abington, Oxfordshire OX14 4SH (GB); GLAXO GROUP LIMITED, Greenford, Middlesex UB6 ONN (GB)
(72) Inventor: BRAUN, Ralph, Patrick, Middleton, WI 53562 (US); THOMSEN, Lindy, Hertfordshire SG1 2NY (GB); VAN-WELY, Catherine, Hertfordshire SG1 2NY (GB); ERTL, Peter, Hertfordshire SG1 2NY (GB)
(74) Representative: Woods, Geoffrey Corlett
(86) International application number: PCT/GB2003/001213
(87) International publication number: WO 2003/080112

(56) References cited:
- WO-A-01/54719
- WO-A-02/24225
- WO-A-93/20847
- WO-A-03/025003
- BILLAUT-MULOT O ET AL: "Modulation of cellular and humoral immune responses to a multiepitopic HIV-1 DNA vaccine by interleukin-18 DNA immunization/viral protein boost" VACCINE, BUTTERWORTH SCIENTIFIC. GUILDFORD, GB, vol. 19, no. 20-22, 6 April 2001 (2001-04-06), pages 2803-2811, XP004231795 ISSN: 0264-410X
- BERNSTEIN D I ET AL: "Effect of imiquimod as an adjuvant for immunotherapy of genital HSV in guinea-pigs" VACCINE, BUTTERWORTH SCIENTIFIC. GUILDFORD, GB, vol. 13, no. 1, 1995, pages 72-76, XP004057704 ISSN: 0264-410X
- VASILAKOS J P ET AL: "ADJUVANT ACTIVITIES OF IMMUNE RESPONSE MODIFIER R-848: COMPARISON WITH CPG ODN" CELLULAR IMMUNOLOGY, ACADEMIC PRESS, SAN DIEGO, CA, US, vol. 204, no. 1, 25 August 2000 (2000-08-25), pages 64-74, XP001037913 ISSN: 0008-8749

## Description

### Field of the invention

The invention relates to the fields of vaccines, vaccine adjuvants, molecular biology and immunology, and generally relates to adjuvants and nucleic acid immunization techniques. More specifically, the invention relates to certain adjuvant compositions, and to vaccine and/or nucleic acid immunization strategies employing such compositions. The invention in particular relates to DNA vaccines that are useful in the prophylaxis and treatment of HIV infections, more particularly when administered by particle mediated delivery.

### Background of the invention

HIV-1 is the primary cause of the acquired immune deficiency syndrome (AIDS) which is regarded as one of the world's major health problems. Although extensive research throughout the world has been conducted to produce a vaccine, such efforts thus far have not been successful.

Non-envelope proteins of HIV-1 have been described and include for example internal structure proteins such as the products of the *gag* and *pol* genes and, other non-structural proteins such as Rev, Nef, Vif and Tat (Green et al., New England J. Med, 324, 5, 308 et seq (1991) and Bryant et al. (Ed. Pizzo), Pediatr. Infect. Dis. J., 11, 5, 390 et seq (1992).

The Gag gene is translated from the full-length RNA to yield a precursor polyprotein which is subsequently cleaved into 3 - 5 capsid proteins; the matrix protein, capsid protein and nucleic acid binding protein and protease. (1. Fundamental Virology, Fields BN, Knipe DM and Howley M 1996 2. Fields Virology vol 2 1996).

The gag gene gives rise to the 55-kilodalton (kD) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the pol gene) during the process of viral maturation into four smaller proteins designated MA (matrix [p17]), CA (capsid [p24]), NC (nucleocapsid [p9]), and p6.(4)

In addition to the 3 major Gag protein, all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus.(5) These MA molecules facilitate the nuclear transport of the viral genome because a karyophilic signal on MA is recognized by the cellular nuclear import machinery. This phenomenon allows HIV to infect nondividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporine A inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell.

The Pol gene encodes two proteins containing the two activities needed by the virus in early infection, the RT and the integrase protein needed for integration of viral DNA into cell DNA. The primary product of Pol is cleaved by the virion protease to yield the amino terminal RT peptide which contains activities necessary for DNA synthesis (RNA and DNA directed DNA polymerase, ribouclease H) and carboxy terminal integrase protein. HIV RT is a heterodimer of full-length RT (p66) and a cleavage product (p51) lacking the carboxy terminal Rnase integrase domain.

RT is one of the most highly conserved proteins encoded by the retroviral genome. Two major activities of RT are the DNA Pol and Ribonuclease H. The DNA Pol activity of RT uses RNA and DNA as templates interchangeably and like all DNA polymerases known is unable to initiate DNA synthesis de novo, but requires a preexisting molecule to serve as a primer (RNA).

The Rnase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains with the Pol covering the amino two thirds of the Pol.

The p66 catalytic subunit is folded into 5 distinct subdomains. The amino terminal 23 of these have the portion with RT activity. Carboxy term to these is the Rnase H Domain.

After infection of the host cell, the retroviral RNA genome is copied into linear ds DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the c terjminal domain. The catalytic core domain contains all of the requirements for the chemistry of polynucleotidyl transfer.

The Nef protein is known to cause the removal of CD4, the HIV receptor, from the cell surface, but the biological importance of this function is debated. Additionally Nef interacts with the signal pathway of T cells and induces an active state, which in turn may promote more efficient gene expression. Some HIV isolates have mutations in this region, which cause them not to encode functional protein and are severely compromised in their replication and pathogenesis in vivo.

DNA vaccines usually consist of a bacterial plasmid vector into which is inserted a strong promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example E.coli and then isolated and prepared in an appropriate medium, depending upon the intended route of administration, before being administered to the host. Following administration the plasmid is taken up by cells of the host where the encoded peptide is produced. The plasmid vector will preferably be made without an origin of replication which is functional in eukaryotic cells, in order to prevent plasmid replication in the mammalian host and integration within chromosomal DNA of the animal concerned.

There are a number of advantages of DNA vaccination relative to traditional vaccination techniques. First, it is predicted that because of the proteins which are encoded by the DNA sequence are synthesised in the host, the structure or conformation of the protein will be similar to the native protein associated with the disease state. It is also likely that DNA vaccination will offer protection against different strains of a virus, by generating cytotoxic T lymphocyte response that recognise epitopes from conserved proteins. Furthermore, because the plasmids are taken up by the host cells where antigenic protein can be produced, a long-lasting immune response will be elicited. The technology also offers the possibility of combing diverse immunogens into a single preparation to facilitate simultaneous immunisation in relation to a number of disease states.

Techniques for the injection of DNA and mRNA into mammalian tissue for the purposes of immunization against an expression product have been described in the art. The techniques, termed "nucleic acid immunization" herein, have been shown to elicit both humoral and cell-mediated immune responses. For example, sera from mice immunized with a DNA construct encoding the envelope glycoprotein, gp160, were shown to react with recombinant gp160 in immunoassays, and lymphocytes from the injected mice were shown to proliferate in response to recombinant gp120. Wang et al. (1993) Proc. Natl. Acad. Sci. USA 90:4156-4160. Similarly, mice immunized with a human growth hormone (hGH) gene demonstrated an antibody-based immune response. Tang et al. (1992) Nature 356:152-154. Intramuscular injection of DNA encoding influenza nucleoprotein driven by a mammalian promoter has been shown to elicit a CD8+ CTL response that can protect mice against subsequent lethal challenge with virus. Ulmer et al. (1993) Science 259:1745-1749. Immunohistochemical studies of the injection site revealed that the DNA was taken up by myeloblasts, and cytoplasmic production of viral protein could be demonstrated for at least 6 months.

The use of imiquimod as adjuvant with protein-based HIV-vaccines was shown in WO93/20847.

### Summary of the invention

The inventors have found that an imidazo quinoline amine compound acts as an effective adjuvant when administered topically 12 to 36 hours after a primer or booster immunisation. In addition the compound was found to be effective in stimulating cell-mediated immunity. The compound is from among a series of related compounds known to be capable of modifying immune responses.

Further the inventors have made a construct which may be used in nucleic acid vaccines for the prophylaxis and treatment of HIV infections and AIDS.

Accordingly the invention provides for enhancing in an individual an immune response generated by a nucleic acid vaccine, by administering a compound which is an imidazoquinoline amine, imidazopyridine amine, 6,7-fused cycloalkylimidazopyridine amine, 1,2-bridged imidazoquinoline amine, thiazolo- or oxazolo-quinolinamine or pyridinamines, imidazonaphthyridine or tetrahydroimidazonaphthyridine amine, wherein the compound is administered topically or transdermally to the individual 12 to 36 hours after the nucleic acid vaccine is administered, and wherein the nucleic acid vaccine comprises a nucleotide sequence that encodes an HIV-1 gag protein or fragment containing a gag epitope thereof and a second HIV antigen or a fragment encoding an epitope of said second HIV antigen, operably linked to a heterologous promoter.

### Brief description of the Figures

Figures 1 to 17 show the adjuvant activity of the compound of the invention in a series of vaccination experiments.
Figure 16 shows IFN-γ sAg Elispots 2 weeks post prime when imiquimod is used as adjuvant with pdpsc18.1. The administrations for columns 1 to 13 of the Figure are as follows:

| | |
|---|---|
| 1. | 2.0 ug pdpsc 18 |
| 2. | 0.2 ug pdpsc 18 |
| 3. | 0.02 ug pdpsc 18 |
| 4. | 2.0 ug pdpsc 18 IMQ before |
| 5. | 0.2 ug pdpsc 18 IMQ before |
| 6. | 0.02 ug pdpsc 18 IMQ before |
| 7. | 2.0 ug pdpsc 18 IMQ right after |
| 8. | 0.2 ug pdpsc 18 IMQ right after |
| 9. | 0.02 ug pdpsc 18 IMQ light after |
| 10. | 2.0 ug pdpsc 18 IMQ 24 hr pp |
| 11. | 0.2 ug pdpsc 18 IMQ 24 hr pp |
| 12. | 0.02 ug pdpsc 18 IMQ 24 hr pp |
| 13. | 2 ug p7313plc |

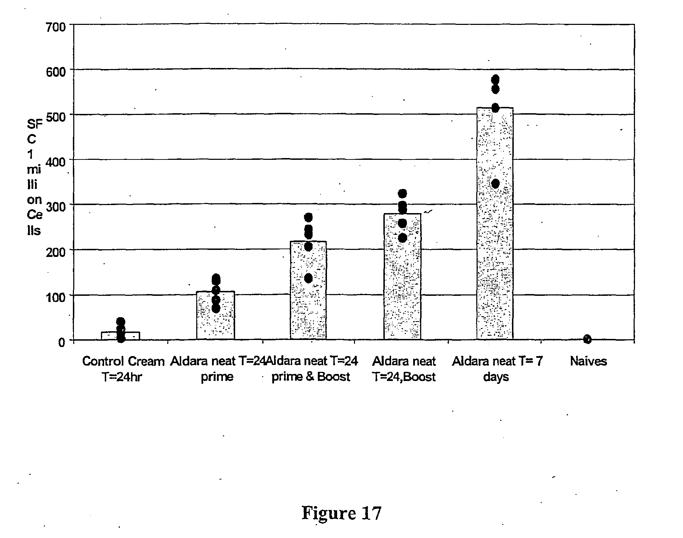

Figure 17 shows IFN-γ Elispots for HBV sAg peptide.

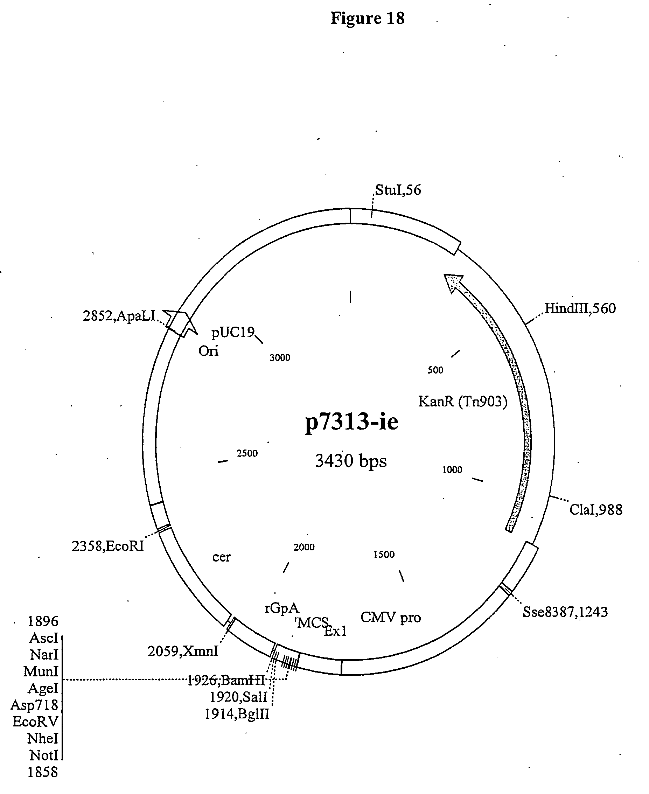

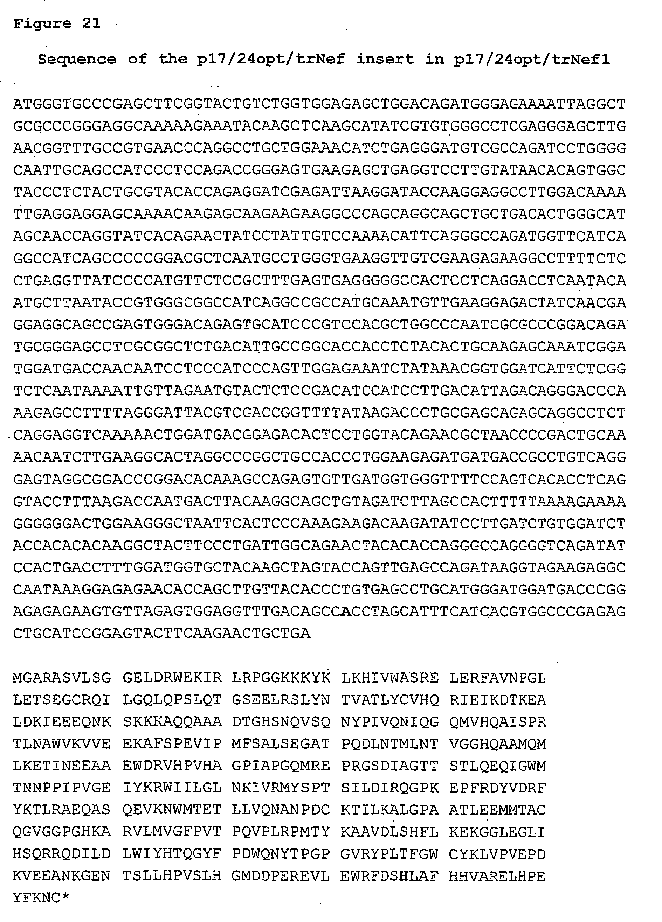

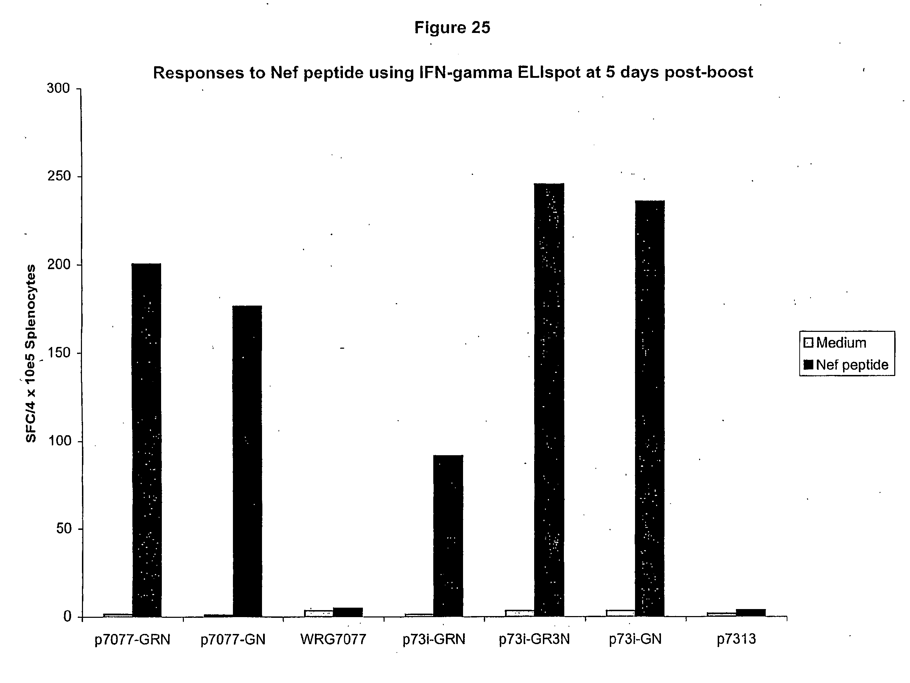

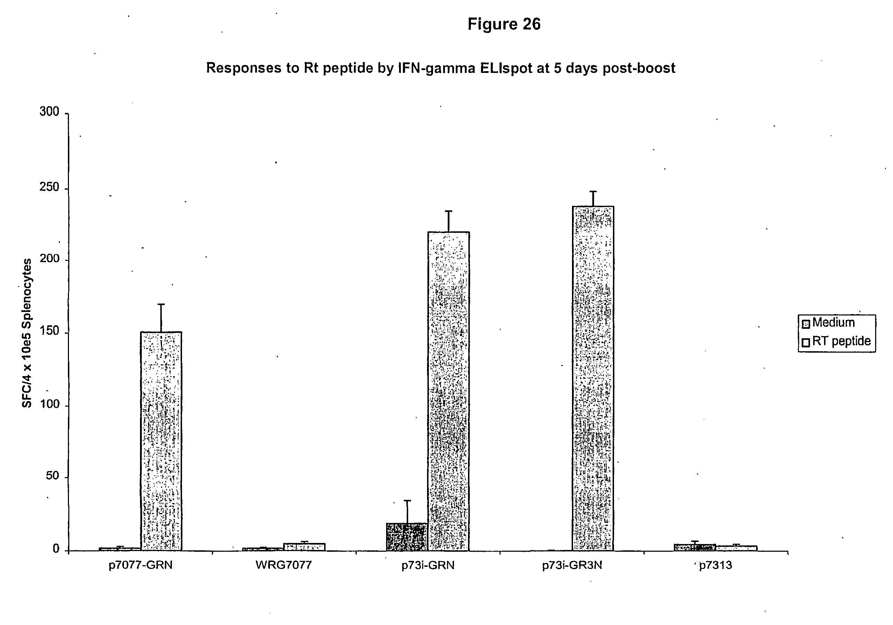

Figures 18 to 26 relate to the construct of the invention.

### Detailed description of the invention

It is to be understood that this invention is not limited to particular antigens or to antigen-coding nucleotide sequences. It is also to be understood that different applications of the disclosed methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an antigen" includes a mixture of two or more such agents, reference to "a particle" includes reference to mixtures of two or more particles, reference to "a recipient cell" includes two or more such cells, and the like.

The invention provides for enhancing an immune response to a vaccine. A compound described herein is administered topically or transdermally to an individual 12 to 36 hours after administration of the vaccine. Typically therefore the compound is administered 16 to 32, 20 to 28 or preferably 22 to 26 hours after administration of the vaccine. Thus the compound may be administered about 24 hours after administration of the vaccine.

The administration of the vaccine (after which the compound of the invention is administered) may be one of a series of administrations of polynucleotide or antigen which occur in an administration regimen. Therefore the administration of the vaccine may be a priming or boosting administration. Thus in one embodiment of the invention the compound of the invention is administered 12 to 36 hours after a priming administration and/or 12 to 36 hours after a booster administration.

In one embodiment nothing further is administered to the individual in the 12 to 36 hour (or any of the other time periods mentioned above) period between administration of the vaccine and the administration of the compound. Alternatively in some embodiments in this time period no vaccine is administered, or preferably at least not the same vaccine which was administered before. In one embodiment no further compound of the invention is administered in the time period.

In other words in some embodiments after administration of the vaccine no further vaccine and/or no compound of the invention is administered until after any of the specified time periods. Such a vaccine may be the same as or different from the vaccine which was administered earlier.

The aim of the invention is to enhance the immune response to the vaccine. Preferably cell-mediated immunity is enhanced, and in particular the CD8 T cell response is enhanced. In this case the administration of the compound of the invention increases the level of CD8 T cell response, for example increases the level of antigen experienced CD8 T cells. The increase in the CD8 T cell response may be measured using any suitable assay (and thus may be capable of being detected in such an assay), such as an ELISPOT assay, preferably an IFN-γ ELISPOT assay.

In one embodiment the CD4 T cell response is also enhanced, such as the CD4 Th1 response. Thus the levels of antigen experienced CD4 T cells may also be increased. Such increased levels of CD4 T cells may be detected using a suitable assay, such as a proliferation assay.

Administration of the compound may cause the immune response to shift to a cell mediated response. Thus the immune response may shift towards a Th1 response and/or the ratio of IgG1 to IgG2a antibody may decrease. In one embodiment the administration of the compound causes a decrease in antibody response.

The compound is selected from an imidazoquinoline amine, imidazopyridine amine, 6,7-fused cycloalkylimidazopyridine amine, 1,2-bridged imidazoquinoline amine, thiazolo- and oxazolo-quinolinamines and pyridinamines, imidazonaphthyridine and tetrahydroimidazonaphthyridine amine.

Preferred compounds include 1H-imidazo[4,5-c]quinolin-4-amines defined by one of Formulas I-V below: 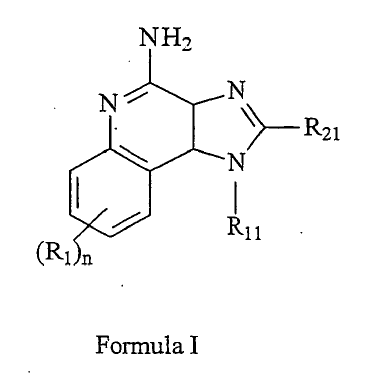 wherein:
R11 is selected from the group consisting of alkyl of one to ten carbon atoms, hydroxyalkyl of one to six carbon atoms, acyloxyalkyl wherein the acyloxy moiety is alkanoyloxy of two to four carbon atoms or benzoyloxy, and the alkyl moiety contains one to six carbon atoms, benzyl, (phenyl)ethyl and phenyl, said benzyl, (phenyl)ethyl or phenyl substituent being optionally substituted on the benzene ring by one or two moieties independently selected from the group consisting of alkyl of one to four carbon atoms, alkoxy of one to four carbon atoms and halogen, with the proviso that if said benzene ring is substituted by two of said moieties, then said moieties together contain no more than six carbon atoms;
R21 is selected from the group consisting of hydrogen, alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to four carbon atoms, alkoxy of one to four carbon atoms and halogen, with the proviso that when the benzene ring is substituted by two of said moieties, then the moieties together contain no more than six carbon atoms; and
each R1 is independently selected from the group consisting of alkoxy of one to four carbon atoms, halogen, and alkyl of one to four carbon atoms, and n is an integer from 0 to 2, with the proviso that if n is 2, then said R1 groups together contain no more than six carbon atoms; 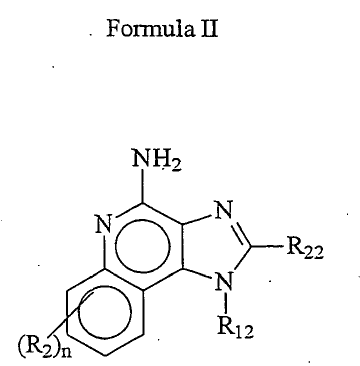
wherein:
R12 is selected from the group consisting of straight chain or branched chain alkenyl containing two to ten carbon atoms and substituted straight chain or branched chain alkenyl containing two to ten carbon atoms, wherein the substituent is selected from the group consisting of straight chain or branched chain alkyl containing one to four carbon atoms and cycloalkyl containing three to six carbon atoms; and cycloalkyl containing three to six carbon atoms substituted by straight chain or branched chain alkyl containing one to four carbon atoms; and
R22 is selected from the group consisting of hydrogen, straight chain or branched chain alkyl containing one to eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl containing one to four carbon atoms, straight chain or branched chain alkoxy containing one to four carbon atom, and halogen, with the proviso that when the benzene ring is substituted by two such moieties, then the moieties together contain no more than six carbon atoms; and
each R2 is independently selected from the group consisting of straight chain or branched chain alkoxy containing one to four carbon atoms, halogen, and straight chain or branched chain alkyl containing one to four carbon atoms, and n is an integer from zero to 2, with the proviso that if n is 2, then said R2 groups together contain no more than six carbon atoms; 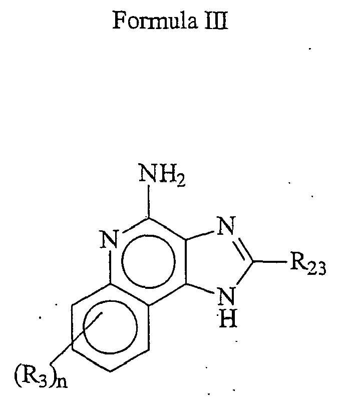
wherein:
R23 is selected from the group consisting of hydrogen, straight chain or branched chain alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl of one to four carbon atoms, straight chain or branched chain alkoxy of one to four carbon atoms, and halogen, with the proviso that when the benzene ring is substituted by two such moieties, then the moieties together contain no more than six carbon atoms; and
each R3 is independently selected from the group consisting of straight chain or branched chain alkoxy of one to four carbon atoms, halogen, and straight chain or branched chain alkyl of one to four carbon atoms, and n is an integer from zero to 2, with the proviso that if n is 2, then said R3 groups together contain no more than six carbon atoms; 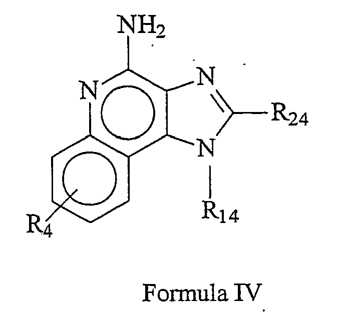
wherein:
R14 is -CHRₓR_{y} wherein R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when R_{y} is a carbon-carbon bond R_{y} and Rₓ together and a tetrahydrofuranyl group optionally substituted with one or more substituents independently selected from the group consisting of hydroxy and hydroxyalkyl of one to four carbon atoms;
R24 is selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen; and
R4 is selected from the group consisting of hydrogen, straight chain or branched chain alkoxy containing one to four carbon atoms, halogen, and straight chain or branched chain alkyl containing one to four carbon atoms; 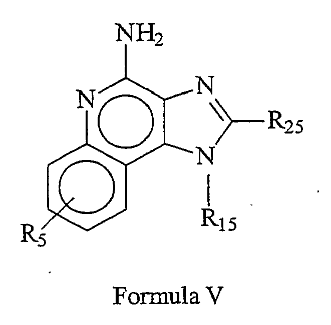
wherein:
R15 is selected from the group consisting of: hydrogen; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; straight chain or branched chain alkenyl containing two to ten carbon atoms and substituted straight chain or branched chain alkenyl containing two to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;
R15 is preferably a C1-2 alkyl group which is substituted by a hydroxyalkyl of 1 to 4 carbon atoms, and more preferably R15 is a C1 alkyl group which is substituted by a hydroxyalkyl of 3 carbon atoms;
R25 is 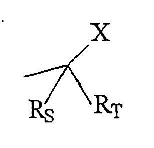
wherein:
R_{S} and R_{T} are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;
X is selected from the group consisting of alkoxy containing one to four carbon atoms, alkoxyalkyl wherein the alkoxy moiety contains one to four carbon atoms and the alkyl moiety contains one to four carbon atoms, hydroxyalkyl of one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, chloro hydroxy, 1-morpholino, 1-pyrrolidino, alkylthio of one to four carbon atoms; and
R5 is selected from the group consisting of hydrogen, straight chain or branched chain alkoxy containing one to four carbon atoms, halogen, and straight chain or branched chain alkyl containing one to four carbon atoms; or a pharmaceutically acceptable salt of any of the foregoing.

Preferred 6,7 fused cycloalkylimidazopyridine amine compounds are defined by Formula VI below: 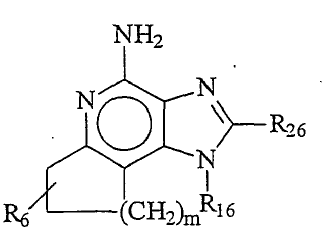 wherein m is 1, 2, or 3;
R16 is selected from the group consisting of hydrogen; cyclic alkyl of three, four, or five carbon atoms; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; fluoro- or chloroalkyl containing from one to ten carbon atoms and one or more fluorine or chlorine atoms; straight chain or branched chain alkenyl containing two to ten carbon atoms and substituted straight chain or branched chain alkenyl containing two to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms, with the proviso that any such alkyl, substituted alkyl, alkenyl, substituted alkenyl, hydroxyalkyl, alkoxyalkyl, or acyloxyalkyl group does not have a fully carbon substituted carbon atom bonded directly to the nitrogen atom; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;
and -CHRₓR_{y}
   wherein:
   R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when R_{y} is a carbon-carbon bond R_{y} and Rₓ together form a tetrahydrofuranyl group optionally substituted with one or more substituents independently selected from the group consisting of hydroxy and hydroxyalkyl of one to four carbon atoms,
   R26 is selected from the group consisting of hydrogen, straight chain or branched chain alkyl containing one to eight carbon atoms, straight chain or branched chain hydroxyalkyl containing one to six carbon atoms, morpholinoalkyl, benzyl, (phenyl)ethyl and phenyl, the benzyl, (phenyl)ethyl or phenyl substituent being optionally substituted on the benzene ring by a moiety selected from the group consisting of methyl, methoxy, and halogen; and
   -C(R_{S})(R_{T})(X) wherein R_{S} and R_{T} are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;
   X is selected from the group consisting of alkoxy containing one to four carbon atoms, alkoxyalkyl wherein the alkoxy moiety contains one to four carbon atoms and the alkyl moiety contains one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, alkylthio of one to four carbon atoms, and morpholinoalkyl wherein the alkyl moiety contains one to four carbon atoms, and
   R6 is selected from the group consisting of hydrogen, fluoro, chloro, straight chain or branched chain alkyl containing one to four carbon atoms, and straight chain or branched chain fluoro- or chloroalkyl containing one to four carbon atoms and at least one fluorine or chlorine atom;
   and pharmaceutically acceptable salts thereof.

Preferred imidazopyridine amine compounds are defined by Formula VII 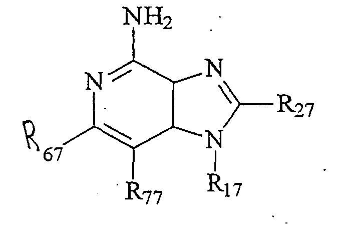 below:
wherein
R17 is selected from the group consisting of hydrogen; -CH₂R_{W} wherein R_{W} is selected from the group consisting of straight chain, branched chain, or cyclic alkyl containing one to ten carbon atoms, straight chain or branched chain alkenyl containing two to ten carbon atoms, straight chain or branched chain hydroxyalkyl containing one to six carbon atoms, alkoxyalkyl wherein the alkoxy moiety contains one to four carbon atoms and the alkyl moiety contains one to six carbon atoms, and phenylethyl; and -CH=CR_{Z}R_{Z} wherein each R_{Z} is independently straight chain, branched chain, or cyclic alkyl of one to six carbon atoms;
R27 is selected from the group consisting of hydrogen, straight chain or branched chain alkyl containing one to eight carbon atoms, straight chain or branched chain hydroxyalkyl containing one to six carbon atoms, alkoxyalkyl wherein the alkoxy moiety contains one to four carbon atoms and the alkyl moiety contains one to six carbon atoms, benzyl, (phenyl)ethyl and phenyl, the benzyl, (phenyl)ethyl or phenyl substituent being optionally substituted on the benzene ring by a moiety selected from the group consisting of methyl, methoxy, and halogen; and morpholinoalkyl wherein the alkyl moiety contains one to four carbon atoms;
R67 and R77 are independently selected from the group consisting of hydrogen and alkyl of one to five carbon atoms, with the proviso that R67 and R77 taken together contain no more than six carbon atoms, and with the further proviso that when R77 is hydrogen then R67 is other than hydrogen and R27 is other than hydrogen or morpholinoalkyl, and with the further proviso that when R67 is hydrogen then R77 and R27 are other than hydrogen;
and pharmaceutically acceptable salts thereof.

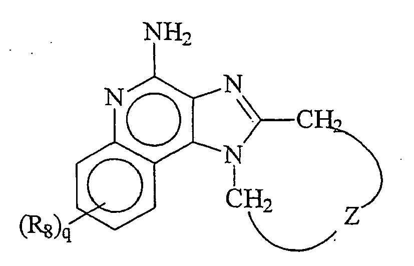

Preferred 1,2-bridged imidazoquinoline amine compounds are defined by Formula VIII below:
wherein:
Z is selected from the group consisting of:
   - (CH₂)p- wherein p is 1 to 4;
   - (CH₂)a -C(R_{D}R_{E})(CH₂)_{b}-, wherein a and b are integers and a+b is 0 to 3, R_{D} is hydrogen or alkyl of one to four carbon atoms, and R_{E} is selected from the group consisting of alkyl of one to four carbon atoms, hydroxy, -OR_{F} wherein R_{F} is alkyl of one to four carbon atoms, and -NR_{G} R'_{G} wherein R_{G} and R'_{G} are independently hydrogen or alkyl of one to four carbon atoms; and
   - (CH₂)a -(Y)-(CH₂)_{b}- wherein a and b are integers and a+b is 0 to 3, and Y is O, S, or - NR_{J}- wherein R_{J} is hydrogen or alkyl of one to four carbon atoms;
   and wherein q is 0 or 1 and R8 is selected from the group consisting of alkyl of one to four carbon atoms, alkoxy of one to four carbon atoms, and halogen,
   and pharmaceutically acceptable salts thereof.

Suitable thiazolo- and oxazolo-quinolinamine and pyridinamine compounds include compounds of Formula IX and Formula IXa: 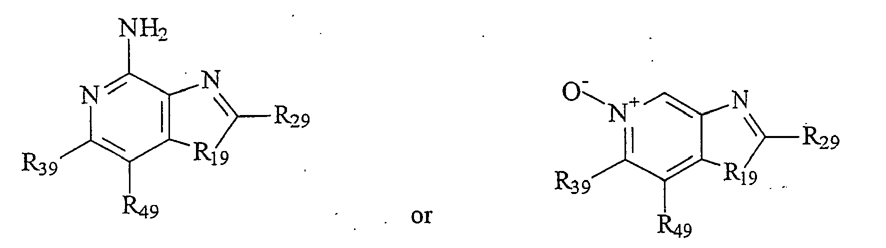 wherein:
R19 is selected from the group consisting of oxygen, sulfur and selenium;
R29 is selected from the group consisting of
   - hydrogen;
   - alkyl;
   - alkyl-OH;
   - haloalkyl;
   - alkenyl;
   - alkyl-X-alkyl;
   - alkyl-X-alkenyl;
   - alkenyl-X-alkyl;
   - alkenyl-X-alkenyl;
   - alkyl-N(R59)₂ ;
   - alkyl-N_{3 ;}
   - alkyl-O-C(O)-N(R59)₂ ;
   - heterocyclyl;
   - alkyl-X-heterocyclyl;
   - alkenyl-X-heterocyclyl;
   - aryl;
   - alkyl-X-aryl;
   - alkenyl-X-aryl;
   - heteroaryl;
   - alkyl-X-heteroaryl; and
   - alkenyl-X-heteroaryl;
R39 and R49 are each independently:
   - hydrogen;
   - X-alkyl;
   - halo;
   - haloalkyl;
   - N(R59)₂ ;
   or when taken together, R39 and R49 form a fused aromatic, heteroaromatic, cycloalkyl or heterocyclic ring;

X is selected from the group consisting of -O-, -S-, -NR59-, -C(O)-, -C(O)O-, -OC(O)-, and a bond; and
each R59 is independently H or C₁₋₈ alkyl;

For formula IX and IXa the terms "alkyl" and "alkenyl" refer to a straight or branched hydrocarbon group, or a cyclic group (i.e., cycloalkyl and cycloalkenyl) that contains from 1 to 20, preferably 1 to 10, more preferably 1 to 8 carbon atoms, unless otherwise specified. Typical alkyl groups are, for example, methyl, ethyl, n-propyl, isopropyl, n-butyl, isobutyl, sec-butyl, tert-butyl, n-pentyl, n-hexyl, n-heptyl, n-octyl, and the like. Exemplary cyclic groups include cyclopropyl, cyclopentyl, cyclohexyl, cyclohexenyl and adamantyl. The prefix "alk," when used, e.g. for "alkoxy" and the like, also has the same meaning.

The term "aryl" refers to a carbocyclic aromatic ring or ring system. The aryl group is preferably a six-membered ring, such as phenyl, or an aromatic polycyclic ring system, such as naphthyl. The most preferred aryl group is phenyl which may be unsubstituted or substituted by one or more substituents as defined below. Examples of other suitable aryl groups include biphenyl, fluorenyl and indenyl.

The term "heteroaryl" refers to an aromatic ring or ring system that contains one or more heteroatoms, in which the beteroatoms are selected from nitrogen, oxygen and sulfur. Suitable heteroaryl groups include furyl, thienyl, pyridyl, quinolinyl, tetrazolyl, imidazo, and so on. In the case where R3 and R4 are taken together and form a 5- or 6-membered heteroaromatic ring, the heteroatom is nitrogen, oxygen or sulfur and the ring may contain one or more of such atoms. Preferably, the heteroatom is nitrogen or sulfur. Preferred heteroaromatic rings formed by R3 and R4 are illustrated by the following formulae where the two lines indicate where they are fused.

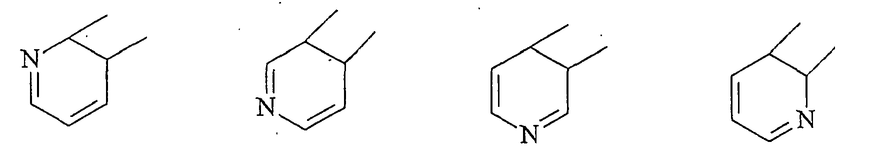

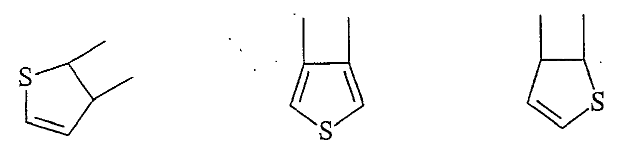

The terms "heterocyclic" and "heterocyclyl" refer to non-aromatic rings or ring systems that contain one or more ring heteroatoms (e.g., O, S, N). Exemplary heterocyclic groups include pyrrolidinyl, tetrahydrofuranyl, morpholinyl, piperidino, piperazino, thiazolidinyl, imidazolidinyl, and the like.

All of the above rings and ring systems can be unsubstituted or substituted by one or more substituents selected from the group consisting of alkyl, alkoxy, alkylthio, hydroxy, halogen, haloalkyl, polyhaloalkyl, perhaloalkyl (e.g., trifluoromethyl), trifluoroalkoxy (e.g., trifluoromethoxy), nitro, amino, alkylamino, dialkylamino, alkylcarbonyl, alkenylcarbonyl, arylcarbonyl, heteroarylcarbonyl, aryl, arylalkyl, heteroaryl, heteroarylalkyl, heterocyclyl, heterocycloalkyl, nitrile and alkoxycarbonyl. Preferred substituents are C₁₋₄ alkyl, C₁₋₄ alkoxy, halo, amino, alkylamino, dialkylamino, hydroxy, C₁₋₄ alkoxymethyl and trifluoromethyl.

The term "halo" refers to a halogen atom, such as, for example, fluorine, chlorine, bromine or iodine.

Suitable imidazonaphthyridine and tetrahydroimidazomaphthyridine compounds are those of Formulae X and XI below: 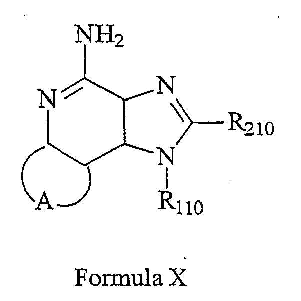 wherein
A is =N-CR=CR-CR=; =CR-N=CR-CR=; =CR-CR=N-CR=; or =CR-CR=CR-N=;
R110 is selected from the group consisting of:
   - hydrogen;
   - C₁₋₂₀ alkyl or C₂₋₂₀ alkenyl that is unsubstituted or substituted by one or more substituents selected from the group consisting of:

   - aryl;
   - heteroaryl;
   - heterocyclyl;
   - O-C₁₋₂₀ alkyl,
   - O-(C₁₋₂₀ alkyl)₀₋₁ -aryl;
   - O-(C₁₋₂₀ akryl)₀₋₁ -heteroaryl;
   - O-(C₁₋₂₀ alkyl)₀₋₁ -heterocyclyl;
   - C₁₋₂₀ alkoxycarbonyl;
   - S(O)0-2-C₁₋₂₀ alkyl;
   - S(O)₀₋₂ -(C₁₋₂₀ alkyl)₀₋₁ -aryl;
   - S(O)₀₋₂ -(C₁₋₂₀ alkyl)₀₋₁ -heteroaryl;
   - S(O)₀₋₂ -(C₁₋₂₀ alkyl)₀₋₁ -heterocyclyl;
   - N(R310)₂ ;
   - N₃ ;
      oxo;
   - halogen;
   - NO₂ ;
   - OH; and
   - SH; and
   - C₁₋₂₀ alkyl-NR310-Q-X-R410 or -C₂₋₂₀ alkenyl-NR310 -Q-X-R410 wherein Q is -CO-or -SO₂-; X is a bond, -O- or -NR310 - and R410 is aryl; heteroaryl; heterocyclyl; or - C₁₋₂₀ alkyl or C₂₋₂₀ alkenyl that is unsubstituted or substituted by one or more substituents selected from the group consisting of:
      - aryl;
      - heteroaryl;
      - heterocyclyl;
      - O-C₁₋₂₀ alkyl,
      - O-(C₁₋₂₀ alkyl)₀₋₁ -aryl;
      - O-(C₁₋₂₀ alkyl)₀₋₁ -heteroaryl;
      - O-(C₁₋₂₀ alkyl)₀₋₁ -heterocyclyl;
      - C₁₋₂₀ alkoxycarbonyl;
      - S(O)₀₋₂ -C₁₋₂₀ alkyl;
      - S(O)₀₋₂ -(C₁₋₂₀ alkyl)₀₋₁ -aryl;
      - S(O)₀₋₂ -(C₁₋₂₀ alkyl)₀₋₁ -heteroaryl;
      - S(O)₀₋₂ -(C₁₋₂₀ alky)₀₋₁ -heterocyclyl;
      - N(R310)₂;
      - NR310 -CO-O-C₁₋₂₀ alkyl;
      - N₃ ;
         oxo;
      - halogen;
      - NO₂ ;
      - OH; and
      - SH; or R410 is 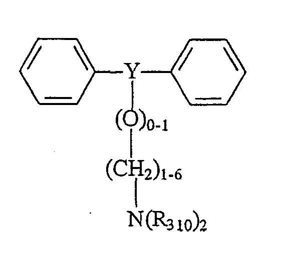
   wherein Y is -N- or -CR-;
R210 is selected from the group consisting of:
   - hydrogen;
   - C₁₋₁₀ alkyl;
   - C₂₋₁₀ alkenyl;
   - aryl;
   - C₁₋₁₀ alkyl -O-C₁₋₁₀ -alkyl;
   - C₁₋₁₀ alkyl-O-C₂₋₁₀ alkenyl; and
   - C₁₋₁₀ alkyl or C₂₋₁₀ alkenyl substituted by one or more substituents selected from the group consisting of:
   - OH;
   - halogen;
   - N(R310)₂ ;
   - CO-N(R310)₂ ;
   - CO-C₁₋₁₀ alkyl;
   - N₃ ;
   - aryl;
   - heteroaryl;
   - heterocyclyl;
   - CO-aryl; and
   - CO-heteroaryl;
   each R310 is independently selected from the group consisting of hydrogen and C₁₋₁₀ alkyl; and
   each R is independently selected from the group consisting of hydrogen, C₁₋₁₀ alkyl, C₁₋₁₀ alkoxy, halogen and trifluoromethyl, or a pharmaceutically acceptable salt thereof. 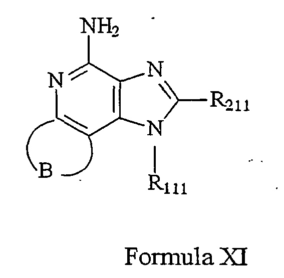 wherein:
B is -NR-C(R)₂ -C(R)₂ -C(R)₂ -; -C(R)₂ -NR-C(R)₂ -C(R)₂ -; -C(R)₂ -C(R)₂-NR-C(R)₂ - or -C(R)₂ -C(R)₂ -C(R)₂-NR-;
R111 is selected from the group consisting of:
   - hydrogen;
   - C₁₋₂₀ alkyl or C₂₋₂₀ alkenyl that is unsubstituted or substituted by one or more substituents selected from the group consisting of:
      - aryl;
      - heteroaryl;
      - heterocyclyl;
      - O-C₁₋₂₀ alkyl;
      - O-(C₁₋₂₀ alkyl)₀₋₁ -aryl;
      - O-(C₁₋₂₀ alkyl)₀₋₁ -heteroaryl;
      - O-(C₁₋₂₀ alkyl)₀₋₁ -heterocyclyl;
      - C₁₋₂₀ alkoxycarbonyl;
      - S(O)₀₋₂ -C₁₋₂₀ alkyl;
      - S(O)₀₋₂ -(C₁₋₂₀ alkyl)₀₋₁ -aryl;
      - S(O)₀₋₂ -(C₁₋₂₀ alkyl)₀₋₁ -heteroaryl;
      - S(O)₀₋₂ -(C₁₋₂₀ alkyl)₀₋₁ -heterocyclyl;
      - N(R311)₂ ;
      - N₃ ;
      - oxo;
      - halogen;
      - NO₂ ;
      - OH; and
      - SH; and
   - C₁₋₂₀ alkyl-NR311 -Q-X-R411 or -C₂₋₂₀ alkenyl-NR311 -Q-X-R411 wherein Q -CO-or -SO₂ -; X is a bond, -O- or -NR311 - and R411 is aryl; heteroaryl; heterocyclyl; or - C₁₋₂₀ alkyl or C₂₋₂₀ alkenyl that is unsubstituted or substituted by one or more substituents selected from the group consisting of:
      - aryl;
      - heteroaryl;
      - heterocyclyl;
      - O-C₁₋₂₀ alkyl,
      - O-(C₁₋₂₀ alkyl)₀₋₁ -aryl;
      - O-(C₁₋₂₀ alkyl)₀₋₁ -heteroaryl;
      - O-(C₁₋₂₀ alkyl)₀₋₁ -heterocyclyl;
      - C₁₋₂₀ alkoxycarbonyl;
      - S(O)o-z -C₁₋₂₀ alkyl;
      - S(O)₀₋₂ -(C₁₋₂₀ alkyl)₀₋₁ -aryl;
      - S(O)₀₋₂ -(C₁₋₂₀ alkyl)₀₋₁ -heteroaryl;
      - S(O)₀₋₂ -(C₁₋₂₀ alkyl)₀₋₁ -heterocyclyl;
      - N(R311)₂ ;
      - NR311 -CO-O-C₁₋₂₀ alkyl;
      - N₃ ;
         oxo;
      - halogen;
      - NO₂;
      - OH; and
      - SH; or R411 is 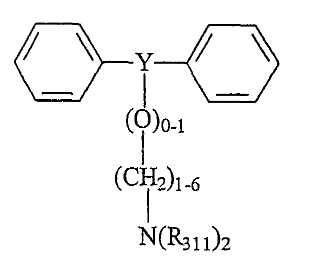
   wherein Y is -N- or -CR-;
R211 is selected from the group consisting of:
   - hydrogen;
   - C₁₋₁₀ alkyl;
   - C₂₋₁₀ alkenyl;
   - aryl
   - C₁₋₁₀ alkyl-O-C₁₋₁₀ -alkyl;
   - C₁₋₁₀ alkyl-O-C₂₋₁₀ alkenyl; and
   - C₁₋₁₀ alkyl or C₂₋₁₀ alkenyl substituted by one or more substituents selected from the group consisting of:
   - OH;
   - halogen;
   - N(R311)₂;
   - CO-N(R311)₂ ;
   - CO-C1-10 alkyl;
   - N₃ ;
   - aryl;
   - heteroaryl;
   - heterocyclyl;
   - CO-aryl; and
   - CO-heteroaryl;
   each R 311 is independently selected from the group consisting of hydrogen and C₁₋₁₀ alkyl; and
   each R is independently selected from the group consisting of hydrogen, C₁₋₁₀ alkyl, C₁₋₁₀ alkoxy, halogen and trifluoromethyl, and pharmaceutically acceptable salts thereof.

The substituents R11 -R111 above are generally designated "1-substituents" herein. The preferred 1-substituents are alkyl containing one to six carbon atoms and hydroxyalkyl containing one to six carbon atoms. More preferably the 1- substituent is 2-methylpropyl or 2-hydroxy-2-methylpropyl.

The substituents R21 -R211 above are generally designated "2-substituents" herein. The preferred 2-substituents are hydrogen, alkyl of one to six carbon atoms, alkoxyalkyl wherein the alkoxy moiety contains one to four carbon atoms and the alkyl moiety contains one to four carbon atoms, and hydroxyalkyl of one to four carbon atoms. More preferably the 2-substituent is hydrogen, methyl, butyl, propyl hydroxymethyl, ethoxymethyl or methoxyethyl.

In instances where n can be zero, one, or two, n is preferably zero or one.

The compound may be defined by Formula XII below: wherein 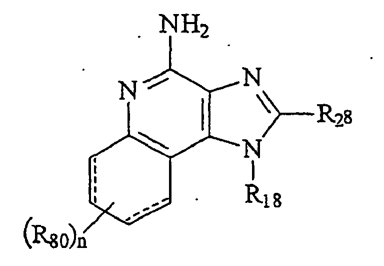
R18 is -alkyl-NR3 -SO₂ -X-R4 or -alkenyl-NR3 -SO₂ -X-R4 ;
X is a bond or -NR5 -;
R4 is aryl, heteroaryl, heterocyclyl, alkyl or alkenyl, each of which may be unsubstituted or substituted by one or more substituents selected from the group consisting of:
   - alkyl;
   - alkenyl;
   - aryl;
   - heteroaryl;
   - heterocyclyl;
   - substituted aryl;
   - substituted heteroaryl;
   - substituted heterocyclyl;
   - O-alkyl;
   - O-(alkyl)₀₋₁ -aryl;
   - O-(alkyl)₀₋₁ -substituted aryl;
   - O-(alkyl)₀₋₁ -heteroaryl;
   - O-(alkyl)₀₋₁ -substituted heteroaryl;
   - O-(alkyl)₀₋₁ -heterocyclyl;
   - O-(alkyl)₀₋₁ -substituted heterocyclyl;
   - COOH;
   - CO-O-alkyl;
   - CO-alkyl;
   - S(O)₀₋₂ -alkyl;
   - S(O)₀₋₂ -(alkyl)₀₋₁ -aryl;
   - S(O)₀₋₂ -(alkyl)₀₋₁ -substituted aryl;
   - S(O)₀₋₂ -(alkyl)₀₋₁ -heteroaryl;
   - S(O)₀₋₂ -(alkyl)₀₋₁ -substituted heteroaryl;
   - S(O)₀₋₂ -(alkyl)₀₋₁ -heterocyclyl;
   - S(O)₀₋₂ -(alkyl)₀₋₁ -substituted heterocyclyl;
   - (alkyl)₀₋₁ -NR3 R3 ;
   - (alkyl)₀₋₁ -NR3 -CO-O-alkyl;
   - (alkyl)₀₋₁ -NR3 -CO-alkyl;
   - (alkyl)₀₋₁-NR3 -CO-aryl;
   - (alkyl)₀₋₁ -NR3 -CO-substituted aryl;
   - (alkyl)₀₋₁ -NR3 -CO-heteroaryl;
   - (alkyl)₀₋₁ -NR3 -CO-substituted heteroaryl;
   - N₃ ;
   - halogen;
   - haloalkyl;
   - haloalkoxy;
   - CO-haloalkoxy;
   - NO₂;
   - CN;
   - OH;
   - SH; and in the case of alkyl, alkenyl, or heterocyclyl, oxo;
R28 is selected from the group consisting of:
   - hydrogen;
   - alkyl;
   - alkenyl;
   - aryl;
   - substituted aryl;
   - heteroaryl;
   - substituted heteroaryl;
   - alkyl-O-alkyl;
   - alkyl-O-alkenyl; and
   - alkyl or alkenyl substituted by one or more substituents selected from the group consisting of:
   - OH;
   - halogen;
   - N(R3)₂;
   - CO-N(R3)₂;
   - CO-C₁₋₁₀ alkyl;
   - CO-O-C₁₋₁₀ alkyl;
   - N₃;
   - aryl;
   - substituted aryl;
   - heteroaryl;
   - substituted heteroaryl;
   - heterocyclyl;
   - substituted heterocyclyl;
   - CO-aryl;
   - CO-(substituted aryl);
   - CO-heteroaryl; and
   - CO-(substituted heteroaryl);
   each R3 is independently selected from the group consisting of hydrogen and C₁₋₁₀ alkyl;
   R5 is selected from the group consisting of hydrogen and C₁₋₁₀ alkyl, or R4 and R5 can combine to form a 3 to 7 membered heterocyclic or substituted heterocyclic ring; n is 0 to 4 and each R80 present is independently selected from the group consisting of C₁₋₁₀ alkyl, C₁₋₁₀ alkoxy, halogen and trifluoromethyl, or a pharmaceutically acceptable salt thereof.

The compounds may be further defined by Formula (XIII) below: 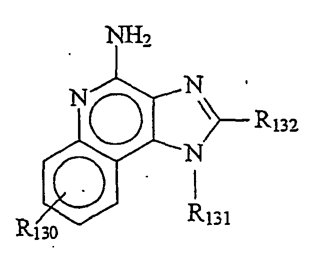 wherein:
R₁₃₁ is selected from the group consisting of straight chain or branched chain alkyl containing one to six carbon atoms and substituted straight chain or branched chain alkyl containing one to six carbon atoms, wherein the substituent is selected from halogen, amino, mono-alkyl amino, di-alkyl amino, alkoxy, alkylthio, hydroxy or hydroxyalkyl, the alkyl groups of the substituents comprising from one to four carbon atoms;
R₁₃₂ is selected from the group consisting of hydrogen, straight chain or branched chain alkyl containing one to six carbon atoms and substituted straight chain or branched chain alkyl containing one to six carbon atoms, wherein the substituent is X-R' wherein X is -NR"-, -O- or -S-, R' is hydrogen or straight chain or branched chain alkyl containing one to four carbon atoms and R" is hydrogen or straight chain or branched chain alkyl containing one to four carbon atoms; and
R₁₃₀ is selected from the group consisting of hydrogen, straight chain or branched chain alkoxy containing one to four carbon atoms, halogen, and straight chain or branched chain alkyl containing one to four carbon atoms.

Preferred compounds of the invention are compounds of Formula (XIII) wherein:
R₁₃₁ is selected from the group consisting of straight chain or branched chain alkyl containing one to six carbon atoms and substituted straight chain or branched chain alkyl containing one to six carbon atoms, wherein the substituent is selected from alkoxy, hydroxy or hydroxyalkyl, the alkyl groups of the substituents comprising from one to four carbon atoms;
R₁₃₂ is selected from the group consisting of hydrogen, straight chain or branched chain alkyl containing one to six carbon atoms and substituted straight chain or branched chain alkyl containing one to six carbon atoms, wherein the substituent is X-R' wherein X is -O- and R' is hydrogen or straight chain or branched chain alkyl containing one to four carbon atoms; and
R₁₃₀ is selected from the group consisting of hydrogen, straight chain or branched chain alkoxy containing one to four carbon atoms and straight chain or branched chain alkyl containing one to four carbon atoms;

Further preferred compounds of the invention are compounds of Formula (XIII) wherein:
R₁₃₁ is selected from the group consisting of straight chain or branched chain alkyl containing one to four carbon atoms and substituted straight chain or branched chain alkyl containing one to four carbon atoms, wherein the substituent is hydroxy or hydroxyalkyl of one to four carbon atoms;
R₁₃₂ is selected from the group consisting of hydrogen, straight chain or branched chain alkyl containing one to four carbon atoms and substituted straight chain or branched chain alkyl containing one to four carbon atoms, wherein the substituent is X-R' wherein X is -O- and R' is straight or branched chain alkyl containing one or two carbon atoms; and
R₁₃₀ is hydrogen.

As used herein (and in particular with reference to Formula XII), unless defined otherwise the terms "alkyl", "alkenyl", "alkynyl" and the prefix "-alk" are inclusive of both straight chain and branched chain groups and of cyclic groups, i.e. cycloalkyl and cycloalkenyl. Unless otherwise specified, these groups contain from 1 to 20 carbon atoms, with alkenyl and alkynyl groups containing from 2 to 20 carbon atoms. Preferred groups have a total of up to 10 carbon atoms. Cyclic groups can be monocyclic or polycyclic and preferably have from 3 to 10 ring carbon atoms. Exemplary cyclic groups include cyclopropyl, cyclopentyl, cyclohexyl and adamantyl.

The term "haloalkyl" is inclusive of groups that are substituted by one or more halogen atoms, including groups wherein all of the available hydrogen atoms are replaced by halogen atoms. This is also true of groups that include the prefix "haloalk-". Examples of suitable haloalkyl groups are chloromethyl, trifluoromethyl, and the like.

The term "aryl" as used herein includes carbocyclic aromatic rings or ring systems. Examples of aryl groups include phenyl, naphthyl, biphenyl, fluorenyl and indenyl. The term "heteroaryl" includes aromatic rings or ring systems that contain at least one ring hetero atom (e.g., O, S, N). Suitable heteroaryl groups include furyl, thienyl, pyridyl, quinolinyl, tetrazolyl, imidazo, pyrazolo, thiazolo, oxazolo, and the like.

"Heterocyclyl" includes non-aromatic rings or ring systems that contain at least one ring hetero atom (e.g., O, S, N). Exemplary heterocyclic groups include pyrrolidinyl, tetrahydrofuranyl, morpholinyl, thiomorpholinyl, piperidinyl, piperazinyl, thiazolidinyl, imidazolidinyl, and the like.

Unless otherwise specified, the terms "substituted cycloalkyl", "substituted aryl", "substituted heteroaryl" and "substituted heterocyclyl" indicate that the rings or ring systems in question are further substituted by one or more substituents independently selected from the group consisting of alkyl, alkoxy, alkylthio, hydroxy, halogen, haloalkyl, haloalkylcarbonyl, haloalkoxy (e.g., trifluoromethoxy), nitro, alkylcarbonyl, alkenylcarbonyl, arylcarbonyl, heteroarylcarbonyl, aryl, arylalkyl, heteroaryl, heteroarylalkyl, heterocyclyl, heterocycloalkyl, nitrile, alkoxycarbonyl, alkanoyloxy, alkanoylthio, and in the case of cycloalkyl and heterocyclyl, oxo. A preferred compound of the invention is imiquimod.

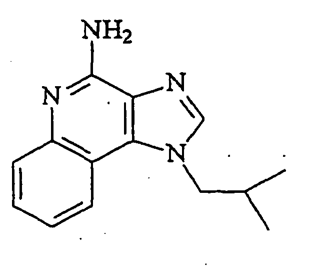

Imiquimod is 1-(2-methyl-propyl)-1H-imidazo [4,5-c] quinolin-4-amine. It has a molecular formula of C₁₄H₁₆N₄ and a molecular weight of 240.3.

A preferred compound of Formula (XIII) is resiquimod:

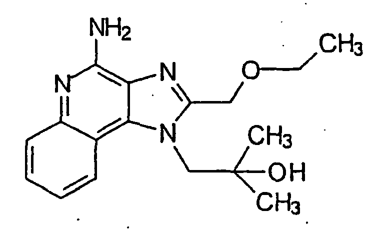

Resiquimod is 4-amino-2-ethoxymethyl-alpha, alpha-dimethyl-1H-imidazo [4,5-c] quinoline-1-ethanol. (R-848; S-28463)

The compound of the invention may be prepared by known means, for example as described in US-B1-6,245,776, US-A-4,689,338, US-A-5,389,640, US-A-5,268,376, US-A-4,929,624, US-A-5,266,575, US-A-5,352,784, US-A-5,494,916, US-A-5,482,936, US-A-5,346,905, US-A-5,395,937, US-A-5,238,944, US-A-5,525,612,US-B1-6,323,200, US-B1-6,331,539 and WO 99/29693.

The nucleic acid vaccine used in the invention comprises a nucleic acid molecule that comprises a nucleotide sequence encoding HIV gag protein or fragment thereof linked to a nucleotide sequence encoding a further HIV antigen or fragment thereof and operably linked to a heterologous promoter. The fragment of said nucleotide sequence will encode an HIV epitope and typically encode a peptide of at least 8 amino acids. The nucleotide sequence is preferably a DNA sequence and is preferably contained within a plasmid without an origin of replication.

In a preferred embodiment of the invention the coding sequence of the nucleic acid is optimised to resemble the codon usage of highly expressed genes in mammalian cells. In particular, the gag protein is optimised to resemble that of highly expressed human genes.

The DNA code has 4 letters (A, T, C and G) and uses these to spell three letter "codons" which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilization of codons may be markedly different in a single species between genes which are expressed at high and low levels. This bias is different in viruses, plants, bacteria and mammalian cells, and some species show a stronger bias away from a random codon selection than others.

For example, humans and other mammals are less strongly biased than certain bacteria or viruses. For these reasons, there is a significant probability that a mammalian gene expressed in E.coli or a viral gene expressed in mammalian cells will have an inappropriate distribution of codons for efficient expression. It is believed that the presence in a heterologous DNA sequence of clusters of codons which are rarely observed in the host in which expression is to occur, is predictive of low heterologous expression levels in that host.

In the polynucleotides, the codon usage pattern may be altered from that typical of human immunodeficiency viruses to more closely represent the codon bias of the target organism, e.g. a mammal, especially a human. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude codons with an RSCU value of less than 0.2 in highly expressed genes of the target organism. A relative synonymous codon usage (RSCU) value is the observed number of codons divided by the number expected if all codons for that amino acid were used equally frequently. A polynucleotide of the present invention will generally have a codon usage coefficient for highly expressed human genes of greater than 0.3, preferably greater than 0.4, most preferably greater than 0.5. Codon usage tables for human can also be found in Genebank.

In comparison, a highly expressed beta action gene has a RSCU of 0.747. The codon usage table for a homo sapiens is set out below:

**Codon Usage Table:**

| ***Homo sapiens* [gbpri]: 27143 CDS's (12816923 codons)** | | | | | | | |
|---|---|---|---|---|---|---|---|
| fields: [triplet] [frequency: per thousand] ([number]) | | | | | | | |
| | | | | | | | |
| UUU | 17.0(217684) | UCU | 14.8(189419) | UAU | 12.1(155645) | UGU | 10.0(127719) |
| UUC | 20.5(262753) | UCC | 17.5(224470) | UAC | 15.8(202481) | UGC | 12.3(157257) |
| UUA | 7.3( 93924) | UCA | 11.9(152074) | UAA | 0.7( 9195) | UGA | 1.3( 16025) |
| UUG | 12.5(159611) | UCG | 4.5( 57572) | UAG | 0.5( 6789) | UGG | 12.9(165930) |
| | | | | | | | |
| CUU | 12.8(163707) | CCU | 17.3(222146) | CAU | 10.5(134186) | CGU | 4.6( 59454) |
| CUC | 19.3(247391) | CCC | 20.0(256235) | CAC | 14.9(190928) | CGC | 10.8(137865) |
| CUA | 7.0( 89078) | CCA | 16.7(214583) | CAA | 12.0(153590) | CGA | 6.3( 80709) |
| CUG | 39.7(509096) | CCG | 7.0( 89619) | CAG | 34.5(441727) | CGG | 11.6(148666) |
| | | | | | | | |
| AUU | 15.8(202844) | ACU | 12.9(165392) | AAU | 17.0(218508) | AGU | 12.0(154442) |
| AUC | 21.6(277066) | ACC | 19.3(247805) | AAC | 19.8(253475) | AGC | 19.3(247583) |
| AUA | 7.2( 92133) | ACA | 14.9(191518) | AAA | 24..0(308123) | AGA | 11.5(147264) |
| AUG | 22.3(285776) | ACG | 6.3( 80369) | AAG | 32.6(418141) | AGG | 11.3(145276) |
| | | | | | | | |
| GUU | 10.9(139611) | GCU | 18.5(236639) | GAU | 22.4(286742) | GGU | 10.8(138606) |
| GUC | 14.6(187333) | GCC | 28.3(362086) | GAC | 26.1(334158) | GGC | 22.7(290904) |
| GUA | 7.0( 89644) | GCA | 15.9(203310) | GAA | 29.1(373151) | GGA | 16.4(210643) |
| GUG | 28.8(369006) | GCG | 7.5( 96455) | GAG | 40.2(515485) | GGG | 16.4(209907) |

Coding GC 52.51% 1st letter GC 56.04% 2nd letter GC 42.35% 3rd letter GC 59.13%

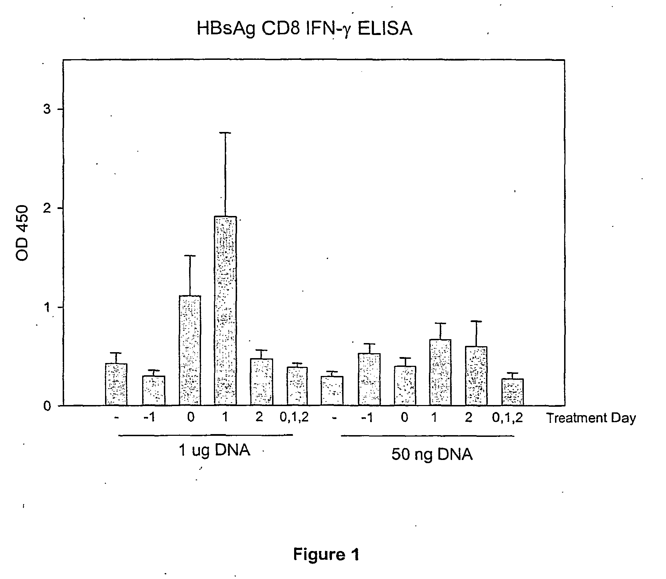

The nucleic acid of the vaccine is generally in the form of a vector. The vector may be suitable for driving expression of heterologous DNA in bacterial insect or mammalian cells, particularly human cells. In one embodiment, the expression vector is p7313 (see figure 1).

In one embodiment in the nucleic acid the gag gene is under the control of a heterologous promoter fused to a DNA sequence encoding NEF, a fragment thereof, or HIV Reverse Transcriptase (RT) or fragment thereof.

In a preferred embodiment, the gag gene does not encode the gag p6 peptide. Preferably the NEF gene is truncated to remove the sequence encoding the N terminal 81 amino acids.

In a further embodiment the RT gene is also optimised to resemble a highly expressed human gene.

In embodiments of the invention fragments of gag, nef or RT proteins are contemplated. For example, a polynucleotide of the invention may encode a fragment of an HIV gag, nef or RT protein. A polynucleotide which encodes a fragment of at least 8, for example 8-10 amino acids or up to 20, 50, 60, 70, 80, 100 150 or 200 amino acids in length is considered to fall within the scope usable in the invention as long as the encoded oligo or polypeptide demonstrates HIV antigenicity. In particular, but not exclusively, this aspect of the invention encompasses the situation when the polynucleotide encodes a fragment of a complete HIV protein sequence and may represent one or more discrete epitopes of that protein. Such fragments may be codon optimised such that the fragment has a codon usage pattern which resembles that of a highly expressed mammalian gene.

Preferred constructs include:

| | |
|---|---|
| 1. | p17, p24, fused to truncated NEF (devoid of nucleotides encoding terminal amino-acids 1-85). |
| 2. | p17, p24, RT, truncated NEF (devoid of nucleotides encoding terminal amino-acids 1-85). |
| 3. | p17, p24 (optimised gag) truncated NEF (devoid of nucleotides encoding terminal amino-acids 1-85). |
| 4. | p17, p24 (optimised gag) RT (optimised) truncated NEF (devoid of nucleotides encoding terminal amino-acids 1-85). |
| 5. | p17, p24, RT (optimised) truncated NEF (devoid of nucleotides encoding terminal amino-acids 1-85). |

As mentioned above the nucleic acid is administered in the form of a vaccine (or vaccine composition). The term "vaccine" or "vaccine composition" intends any pharmaceutical composition containing the nucleic acid, which composition can be used to prevent or treat a disease or condition in a subject (generally HIV and/or AIDS). The term thus encompasses both subunit vaccines, i. e., vaccine compositions containing polynucleotides and/or antigens which are separate and discrete from a whole organism with which they are associated in nature, as well as compositions containing whole killed, attenuated or inactivated bacteria, viruses, parasites or other microbes.

In the invention the compound of the invention is delivered topically or transdermally. The vaccine may also be delivered topically or transdermally. The term "transdermal" delivery intends intradermal (e..g., into the dermis or epidermis), transdermal (e. g.,"percutaneous") and transmucosal administration, i. e., delivery by passage of an agent into or through skin or mucosal tissue. See, e. g., Transdermal Drug Delivery : Developmental Issues and Research Initiatives, Hadgraft and Guy (eds.), Marcel Dekker, Inc., (1989); Controlled Drug Delivery : Fundamentals and Applications, Robinson and Lee (eds.), Marcel Dekker Inc., (1987); and Transdermal Delivery of Drugs, Vols. 1- 3, Kydonieus and Berner (eds.), CRC Press, (1987).

Thus, topical or transdermal delivery encompasses delivery of particles from a particle delivery device (e.g. needleless syringe) as described in U. S. Patent No. 5,630,796, as well as particle-mediated delivery of coated core carriers as described in U. S. Patent No. 5,865,796.

By"core carrier"is meant a carrier particle on which the compound or nucleic acid is coated in order to impart a defined particle size as well as a sufficiently high density to achieve the momentum required for cell membrane penetration, such that the compound or nucleic acid can be delivered using particle-mediated delivery techniques, for example those described in U. S. Patent No. 5,100,792. Core carriers typically include materials such as tungsten, gold, platinum, ferrite, polystyrene and latex. See, for example, Particle Bombardment Technology for Gene Transfer, (1994) Yang, N. ed., Oxford University Press, New York, NY pages 10-11.

By"particle delivery device,"or"needleless syringe,"is meant an instrument which delivers a particulate composition transdermally, without a conventional needle that pierces the skin. Particle delivery devices for use with the present invention are discussed throughout this document.

By"antigen"is meant a molecule which contains one or more epitopes that will stimulate a host's immune system to make a cellular antigen-specific immune response, or a humoral antibody response. Thus, antigens include proteins, polypeptides, antigenic protein fragments, oligosaccharides, polysaccharides, and the like. Similarly, an oligonucleotide or polynucleotide which expresses an antigen, such as in DNA immunization applications, is also included in the definition of antigen.

Synthetic antigens are also included, for example, polyepitopes, flanking epitopes, and other recombinant or synthetically derived antigens (Bergmann et al. (1993) Eur. J. Immunol. 23: 2777-2781; Bergmann et al. (1996) J. Immunol.157: 3242-3249 ; Suhrbier, A. (1997) Immunol. and Cell Biol. 75: 402-408; Gardner et al. (1998) 12th World AIDS Conference, Geneva, Switzerland, June 28-July 3, 1998).

The term"peptide"is used in it broadest sense to refer to a compound of two or more subunit amino acids, amino acid analogs, or other peptidomimetics. The subunits may be linked by peptide bonds or by other bonds, for example ester, ether, etc.

As used herein, the term"amino acid"refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is typically called a polypeptide or a protein.

The antigen or fragments of proteins mentioned herein typically comprise one or more T cell epitopes. "T cell epitopes"are generally those features of a peptide structure capable of inducing a T cell response. In this regard, it is accepted in the art that T cell epitopes comprise linear peptide determinants that assume extended conformations within the peptide-binding cleft of MHC molecules, (Unanue et al. (1987) Science 236: 551-557). As used herein, a T cell epitope is generally a peptide having about 8-15, preferably 5-10 or more amino acid residues.

The compound used in the invention acts as adjuvant. However, further adjuvants may also be used in the invention. Typically such further adjuvants are co-administered with the vaccine. As used herein the term"adjuvant"refers to any material that enhances the action of a drug, antigen, polynucleotide, vector or the like.

Thus, one example of an adjuvant is a"cytokine."As used herein, the term"cytokine" refers to any one of the numerous factors that exert a variety of effects on cells, for example, inducing growth, proliferation or maturation. Certain cytokines, for example TRANCE, flt-3L, and CD40L, enhance the immunostimulatory capacity of APCs. Non-limiting examples of cytokines which may be used alone or in combination include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1 a), interleukin-11 (IL-11), MIP-1a, leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Genentech (South San Francisco, CA), Amgen (Thousand Oaks, CA), R & D Systems and Immunex (Seattle, WA).

The sequence of many of these molecules are also available, for example, from the GenBank database. It is intended, although not always explicitly stated, that molecules having similar biological activity as wild-type or purified cytokines (e. g., recombinantly produced or mutants thereof) and nucleic acid encoding these molecules are intended to be used within the spirit and scope of the invention.

A composition which contains the nucleic acid and an adjuvant (such as the compound usable in the invention), or a vaccine composition which is co-administered with an adjuvant, displays "enhanced immunogenicity"when it possesses a greater capacity to elicit an immune response than the immune response elicited by an equivalent amount of the vaccine administered without the adjuvant.

Such enhanced immunogenicity can be determined by administering the adjuvant composition and antigen controls to animals and comparing antibody titers and/or cellular-mediated immunity between the two using standard assays such as radioimmunoassay, ELISAs, CTL assays, and the like, well known in the art.

The terms"nucleic acid molecule"and"polynucleotide"are used interchangeably and refer to a polymeric form of nucleotides of any length, either deoxyribonucleotides or ribonucleotides, or analogs thereof. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers.

A polynucleotide is typically composed of a specific sequence of four nucleotide bases: adenine (A); cytosine (C); guanine (G); and thymine (T) (uracil (U) is substituted for thymine (T) when the polynucleotide is RNA). Thus, the term polynucleotide sequence is the alphabetical representation of a polynucleotide molecule. This alphabetical representation can be input into databases in a computer having a central processing unit and used for bioinformatics applications such as functional genomics and homology searching.

A"gene"as used in the context of the present invention is a sequence of nucleotides in a genetic nucleic acid (chromosome, plasmid, etc.) with which a genetic function is associated. A gene is a hereditary unit, for example of an organism, comprising a polynucleotide sequence (e. g., a DNA sequence for mammals) that occupies a specific physical location (a"gene locus"or"genetic locus") within the genome of an organism. A gene can encode an expressed product, such as a polypeptide or a polynucleotide (e. g., tRNA). Alternatively, a gene may define a genomic location for a particular event/function, such as the binding of proteins and/or nucleic acids (e. g., phage attachment sites), wherein the gene does not encode an expressed product. Typically, a gene includes coding sequences, such as polypeptide encoding sequences, and non-coding sequences, such as promoter sequences, poly-adenlyation sequences, transcriptional regulatory sequences (e. g., enhancer sequences). Many eucaryotic genes have"exons" (coding sequences) interrupted by"introns" (non-coding sequences). In certain cases, a gene may share sequences with another gene (s) (e. g., overlapping genes).

A"coding sequence"or a sequence which"encodes"a selected polypeptide, is a nucleic acid molecule which is transcribed (in the case of DNA) and translated (in the case of mRNA) into a polypeptide in vivo when placed under the control of appropriate regulatory sequences (or"control elements").

The boundaries of the coding sequence are determined by a start codon at the 5' (amino) terminus and a translation stop codon at the 3' (carboxy) terminus. A coding sequence can include, but is not limited to, cDNA from viral, procaryotic or eucaryotic mRNA, genomic DNA sequences from viral or procaryotic DNA, and even synthetic DNA sequences. A transcription termination sequence may be located 3'to the coding sequence. Transcription and translation of coding sequences are typically regulated by" control elements,"including, but not limited to, transcription promoters, transcription enhancer elements, transcription termination signals, polyadenylation sequences (located 3' to the translation stop codon), sequences for optimization of initiation of translation (located 5' to the coding sequence), and translation termination sequences.

A"promoter"is a nucleotide sequence which initiates transcription of a polypeptide-encoding polynucleotide. Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is repressed by an analyte, cofactor; regulatory protein, etc.), and constitutive promoters. In addition, such promoters can also have tissue specificity.

It is intended that the term"promoter"or"control element"includes full-length promoter regions and functional (e. g., controls transcription or translation) segments of these regions. The promoter present in the polynucleotide used in the invention may be capable of causing expression of the coding sequence in a cell which is found in the vicinity of the skin.

The polynucleotide may be delivered in the form of a vector. A"vector"is capable of transferring gene sequences to target cells (e. g., viral vectors, non-viral vectors, particulate carriers, and liposomes). Typically, "vector construct,""expression vector,"and"gene transfer vector,"mean any nucleic acid construct capable of directing the expression of a gene of interest and which can transfer gene sequences to target cells. Thus, the term includes cloning and expression vehicles, as well as viral vectors.

An"isolated polynucleotide"molecule is a nucleic acid molecule separate and discrete from the whole organism with which the molecule is found in nature; or a nucleic acid molecule devoid, in whole or part, of sequences normally associated with it in nature; or a sequence, as it exists in nature, but having heterologous sequences (as defined below) in association therewith.

"Operably linked"refers to an arrangement of elements wherein the components so described are configured so as to perform their usual function. Thus, a given promoter that is operably linked to a coding sequence (e. g., an antigen or interest) is capable of effecting the expression of the coding sequence when the regulatory proteins and proper enzymes are present. In some instances, certain control elements need not be contiguous with the coding sequence, so long as they function to direct the expression thereof. For example, intervening untranslated yet transcribed sequences can be present between the promoter sequence and the coding sequence and the promoter sequence can still be considered"operably linked"to the coding sequence.

"Recombinant"as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term"recombinant"as used with respect to a protein or polypeptide means a polypeptide produced by expression of a recombinant polynucleotide.

Proteins (including protein antigens), such as Gag, nef and/or RT, as used in the invention (as encoded by the nucleic acid of the invention) may have homology and/or sequence identity with naturally occurring forms. Similarly polynucleotide coding sequences capable of expressing such proteins will generally have homology and/or sequence identity with naturally occurring sequences. Techniques for determining nucleic acid and amino acid"sequence identity"also are known in the art. Typically, such techniques include determining the nucleotide sequence of the mRNA for a gene and/or determining the amino acid sequence encoded thereby, and comparing these sequences to a second nucleotide or amino acid sequence.

In general,"identity"refers to an exact nucleotide-to-nucleotide or amino acid-to-amino acid correspondence of two polynucleotides or polypeptide sequences, respectively. Two or more sequences (polynucleotide or amino acid) can be compared by determining their "percent identity. "The percent identity of two sequences, whether nucleic acid or amino acid sequences, is the number of exact matches between two aligned sequences divided by the length of the shorter sequences and multiplied by 100.

An approximate alignment for nucleic acid sequences is provided by the local homology algorithm of Smith and Waterman, Advances in Applied Mathematics 2: 482-489 (1981). This algorithm can be applied to amino acid sequences by using the scoring matrix developed by Dayhoff, Atlas of Protein Sequences and Structure, M. O. Dayhoff ed., 5 suppl. 3: 353-358, National Biomedical Research Foundation, Washington, D. C., USA, and normalized by Gribskov, Nucl. Acids Res. 14 (6): 6745-6763 (1986). An exemplary implementation of this algorithm to determine percent identity of a sequence is provided by the Genetics Computer Group (Madison, WI) in the"BestFit"utility application. The default parameters for this method are described in the Wisconsin Sequence Analysis Package Program Manual, Version 8 (1995) (available from Genetics Computer Group, Madison, WI). A preferred method of establishing percent identity in the context of the present invention is to use the MPSRCH package of programs copyrighted by the University of Edinburgh, developed by John F. Collins and Shane S.

Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match"value reflects"sequence identity."Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff= 60; expect = 10; Matrix = BLOSUM62 ; Descriptions = 50 sequences; sort by = HIGH SCORE; Databases = non-redundant, GenBank +EMBL + DDBJ + PDB + GenBank CDS translations + Swiss protein + Spupdate + PIR. Details of these programs can be found at the following internet address: http://www. ncbi. nlm. gov/cgi-bin/BLAST.

Alternatively, homology can be determined by hybridization of polynucleotides under conditions which form stable duplexes between homologous regions, followed by digestion with single-stranded-specific nuclease (s), and size determination of the digested fragments. Two DNA, or two polypeptide sequences are"substantially homologous"to each other when the sequences exhibit at least about 80%-85%, preferably at least about 90%, and most preferably at least about 95%-98% sequence identity over a defined length of the molecules, as determined using the methods above.

As used herein, substantially homologous also refers to sequences showing complete identity to the specified DNA or polypeptide sequence. DNA sequences that are substantially homologous can be identified in a Southern hybridization experiment under, for example, stringent conditions, as defined for that particular system. For example, stringent hybridization conditions can include 50% formamide, 5x Denhardt's Solution, 5x SSC, 0.1% SDS and 100 pg/ml denatured salmon sperm DNA and the washing conditions can include 2x SSC, 0.1% SDS at 37 C followed by lx SSC, 0.1% SDS at 68 C. Defining appropriate hybridization conditions is within the skill of the art. See, e. g., Sambrook et al., supra; DNA Cloning, supra; Nucleic Acid Hybridization, supra.

As used herein, the term"treatment"includes any of following: the prevention of infection or reinfection; the reduction or elimination of symptoms; and the reduction or complete elimination of a pathogen (e.g. HIV). Treatment may be effected prophylactically (prior.to infection) or therapeutically (following infection, such as before or after the development of AIDS). An"effective amount"is an amount sufficient to effect beneficial or desired results. An effective amount can be administered in one or more administrations, applications of dosages. The term"coadministering"or"coadministration"refers to administration of at least two substances. Coadministration can be achieved by administering the substances concurrently or at different times. In addition, co-administration includes delivery using one or more delivery means.

The invention is carried out for the purpose of stimulating a suitable immune response. By suitable immune response, it is meant that the invention can bring about in an immunized subject an immune response characterized by the production of B and/or T lymphocytes specific for an antigen, wherein the immune response can protect the subject against subsequent infection with homologous or heterologous strains of HIV, reduce viral burden, bring about resolution of infection in a shorter amount of time relative to a non-immunized subj ect, or prevent or reduce clinical manifestation of disease symptoms, such as AIDS symptoms.

The subject on which the invention is performed is generally a vertebrate subject, typically capable of being infected by HIV. By"vertebrate subject"is meant any member of the subphylum cordata, particularly mammals, including, without limitation, humans and other primates (such as chimpanzees and macaques (e.g. rhesus macaques)), as well as rodents, such as mice and rats. The term does not denote a particular age. Thus, both adult and newborn individuals are intended to be covered. In one embodiment the subject is susceptible to or at risk from HIV.

As mentioned above in the invention a nucleic acid is administered which encodes particular HIV proteins or proteins fragments from HIV. Such a nucleic acid which is capable of expressing such antigens is also termed a DNA-vaccine (although as is clear from the disclosure herein this may consist of a polynucleotide other than DNA). DNA-vaccines generally consist of a plasmid that encodes a relevant antigen for de novo synthesis by cells present in a targeted tissue. Viral promoters, e. g., the promoter from Cytomegalovirus (CMV), are generally used in the nucleic acid (including DNA-vaccine plasmid construct) to drive antigen expression. A preferred promoter element is the CMV immediate early promoter devoid of intron A, but including exon 1. Thus the polynucleotide of the invention may be under the control of HCMV IE early promoter.

Delivery of these DNA-vaccine plasmids, both in "naked" form and attached to particles, has been shown to elicit both humoral and cell-mediated immune responses. (See, e. g., Wang et al. (1993) Proc. Natl. Acad. Sci. USA 90 : 4156-4160; Tang et al. (1992) Nature 356: 152-154; Fynan, supra).

The polynucleotides of the present invention may be introduced into cells in vitro or in vivo, for example by transfection or by coating the polynucleotides onto particles and administering the coated particles to the cells. Alternatively, the polynucleotides and/or peptides may be provided in a particulate (e. g., powder) form, discussed more fully below and in the disclosure of International Publication Numbers WO 97/48485 and WO 98/10750, which are incorporated by reference herein.

Thus, the invention includes eliciting an immune response, including a CTL response (typically CD8 T cell response), in a vertebrate subject by administering a polynucleotide where the antigen encoding sequence is operably linked to a regulatory element capable of causing expression of the coding sequence.

### Antigens encoded by the nucleic acid used in the invention

The invention described herein elicits an immune response against particular antigens for the treatment and/or prevention of HIV infection and/or any condition which is caused by or exacerbated by HIV infection, such as AIDS.

The antigens may derive from any available HIV isolates (typically HIV-1), such as any strain mentioned herein. The antigens include gag antigens (or fragments thereof which contain an epitope) such as p24gag and p55gag, as well as proteins derived from the pol, env, tat, vif, rev, nef, vpr, vpu and LTR regions of HIV (or fragments thereof which contain an epitope).

In a preferred embodiment the nucleic acid comprises sequence encoding at least three HIV antigens, preferably Gag, nef and RT (or instead of the whole protein a fragment of any of these proteins which contains an epitope). These coding sequences may be in any order, but in a preferred embodiment are in the order Nef-RT-Gag, RT-Nef, Gag or RT-Gag-Nef. ,

In one embodiment the HTV protein which are expressed are fusion proteins, such as a fusion protein containing sequence from (including the above-mentioned fragments) Nef, RT and Gag.

Typically, a nucleotide sequence corresponding to (encoding) one or more of the above-listed antigen (s) is used in the production of the polynucleotides, as described below.

### Isolation of Genes and Construction of Polynucleotides

The polynucleotides encode at least two HIV antigens operably linked to a promoter, such as a viral, non-viral, cell-or tissue-specific promoter (e. g., a promoter derived from a regulatory element which controls transcription of a sequence in cells of the species of subject to be vaccinated).

These polynucleotides are useful in eliciting an immune response to the antigen (s), particularly in activating T-lymphocytes. Nucleotide sequences selected for use in the present invention can be derived from known sources, for example, by isolating the same from cells containing a desired gene or nucleotide sequence using standard techniques.

Similarly, the nucleotide sequences can be generated synthetically using standard modes of polynucleotide synthesis that are well known in the art. See, e. g., Edge et al. (1981) Nature 292: 756-762; Nambair et al. (1994) Science 223: 1299-1301 ; Jay et al. (1984) J. Biol. Chem. 259 : 6311-6317. Generally, synthetic oligonucleotides can be prepared by either the phosphotriester method as described by Edge et al., supra, and Duckworth et al. (1981) Nucleic Acids Res. 9 : 1691-1706, or the phosphoramidite method as described by Beaucage et al.(1981) Tet. Letts. 22: 1859, and Matteucci et al. (1981) J. Am. Chem. Soc. 103: 3185. Synthetic oligonucleotides can also be prepared using commercially available automated oligonucleotide synthesizers. The nucleotide sequences can thus be designed with appropriate codons for a particular amino acid sequence.

In general, one will select preferred codons for expression in the intended host. The complete sequence is assembled from overlapping oligonucleotides prepared by standard methods and assembled into a complete coding sequence. See, e. g., Edge et al. (supra) ; Narnbair et al. (supra) and Jay et al. (supra). Another method for obtaining nucleic acid sequences for use herein is by recombinant means. Thus, a desired nucleotide sequence can be excised from a plasmid carrying the same using standard restriction enzymes and procedures.

Site specific DNA cleavage is performed by treating with the suitable restriction enzyme (or enzymes) under conditions which are generally understood in the art, and the particulars of which are specified by manufacturers of commercially available restriction enzymes. If desired, size separation of the cleaved fragments may be performed by polyacrylamide gel or agarose gel electrophoresis using standard techniques.

Restriction cleaved fragments may be blunt ended by treating with the large fragment of E. coli DNA polymerase I (Klenow) in the presence of the four deoxynucleotide triphosphates (dNTPs) using standard techniques. The Klenow fragment fills in at 5'single-stranded overhangs but digests protruding 3'single strands, even though the four dNTPs are present. If desired, selective repair can be performed by supplying only one, or several, selected dNTPs within the limitations dictated by the nature of the overhang. After Klenow treatment, the mixture can be extracted with e. g. phenol/chloroform, and ethanol precipitated.

Treatment under appropriate conditions with S 1 nuclease or BAL-31 results in hydrolysis of any single-stranded portion.

Yet another convenient method for isolating specific nucleic acid molecules is by the polymerase chain reaction (PCR). Mullis et al. (1987) Methods Enzymol. 155: 335-350. This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. The product of the first round of replication is itself a template for subsequent replication, thus repeated successive cycles of replication result in geometric amplification of the DNA fragment delimited by the primer pair used.

This method also allows for the facile addition of nucleotide sequences onto the ends of the DNA product by incorporating these added sequences onto the oligonucleotide primers (see, e. g., PCR Protocols, A Guide to Methods and Applications, Innis et al (eds) Harcourt Brace Jovanovich Publishers, NY (1994)). PCR conditions used for each amplification reaction are empirically determined. A number of parameters influence the success of a reaction. Among them are annealing temperature and time, extension time, Mg2+ and ATP concentration, pH, and the relative concentration of primers, templates, and deoxyribonucleotides.

Once coding sequences for desired proteins have been prepared or isolated, such sequences can be cloned into any suitable vector or replicon. Numerous cloning vectors are known to those of skill in the art, and the selection of an appropriate cloning vector is a matter of choice. Ligations to other sequences are performed using standard procedures, known in the art.

As described in detail below, selected nucleotide sequences can be placed under the control of regulatory sequences such as a promoter, so that the sequence encoding the desired protein is transcribed into RNA in the host tissue transformed by a vector containing this expression construct.

### Promoters

Expression of a selected antigen in a polynucleotide is driven by a promoter, such as a viral, non-viral, preferably mammalian, cell- (or tissue-) specific promoter. In a preferred embodiment, in addition to promoters, it may be desirable to add other regulatory sequences which allow for regulation of the expression of protein sequences encoded by the delivered nucleotide sequences. Suitable additional regulatory sequences are known to those of skill in the art, and examples include those which cause the expression of a coding sequence to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences such that the positioning and orientation of the coding sequence with respect to the control sequences allows the coding sequence to be transcribed under the"control"of the control sequences (i. e., RNA polymerase, which binds to the DNA molecule at the control sequences, transcribes the coding sequence). Modification of the sequences encoding the particular protein of interest may be desirable to achieve this end. For example, in some cases it may be necessary to modify the sequence so that it is attached to the control sequences with the appropriate orientation; i. e., to maintain the reading frame. The control sequences and other regulatory sequences may be ligated to the coding sequence prior to insertion into a vector.

Alternatively, the coding sequence can be cloned directly into an expression vector which already contains the control sequences and an appropriate restriction site.

Generally, nucleic acid molecules used in the subject invention contain coding regions with suitable control sequences and, optionally, ancillary nucleotide sequences which encode cytokines or other immune enhancing polypeptides. The nucleic acid molecules are generally prepared in the form of vectors which include the necessary elements to direct transcription and translation in a recipient cell.

### Adjuvants

In order to augment an immune response in a subject, the compositions and uses described herein can further include ancillary substances/adjuvants as well as the compound specified in the claims, such as pharmacological agents, cytokines, or the like. Suitable adjuvants include any substance that enhances the immune response of the subject to the antigens encoded by the polynucleotide of the invention. They may enhance the immune response by affecting any number of pathways, for example, by stabilizing the antigen/MHC complex, by causing more antigen/MHC complex to be present on the cell surface, by enhancing maturation of APCs, or by prolonging the life of APCs (e. g., inhibiting apoptosis). As described herein, these cytokines, delivered as either peptides or as polynucleotides encoding functional peptides, are also be useful in eliciting immune responses.

Ancillary nucleic acid sequences coding for peptides known to stimulate, modify, or modulate a host's immune response (e. g., cytokines), can be co-administered as polynucleotides with the above-described antigen-encoding polynucleotides or peptide antigens. These nucleotides can be administered either on the same vector that carries the antigen-encoding sequence, or, alternatively on a separate vector. In some cases, it may be desirable to design a polynucleotide in which both the antigen-encoding sequence and the adjuvant-encoding sequence are under the control of the same promoter.

### Administration of Polynucleotides and Adjuvants

The polynucleotides, adjuvants and ancillary substances (including the compound specified in the claims) described herein may be administered by any suitable method (although the compound specified in the claims will be administered topically or transdermally). In a preferred embodiment, described below, they are administered by coating them onto particles and then administering the particles to the subject or cells. However, they may also be delivered using a viral vector as known in the art, or by using non-viral systems, as described for example in U. S. Patent No. 5,589,466.

### Viral Vectors

A number of viral based systems have been used for gene delivery. For example, retroviral systems are known and generally employ packaging lines which have an integrated defective provirus (the"helper") that expresses all of the genes of the virus but cannot package its own genome due to a deletion of the packaging signal, known as the psi sequence. Thus, the cell line produces empty viral shells. Producer lines can be derived from the packaging lines which, in addition to the helper, contain a viral vector which includes sequences required in cis for replication and packaging of the virus, known as the long terminal repeats (LTRs). The gene of interest can be inserted in the vector and packaged in the viral shells synthesized by the retroviral helper. The recombinant virus can then be isolated and delivered to a subject. (See, e. g., U. S. Patent No. 5,219,740.)

Representative retroviral vectors include but are not limited to vectors such as the LHL, N2, LNSAL, LSHL and LHL2 vectors described in e. g., U. S. Patent No. 5,219,740, incorporated herein by reference in its entirety, as well as derivatives of these vectors, such as the modified N2 vector described herein, Retroviral vectors can be constructed using techniques well known in the art. See, e. g., U. S. Patent No 5,219,740; Mann et al. (1983) Cell 33 : 153-159.

Adenovirus based systems have been developed for gene delivery and are suitable for delivering the polynucleotides described herein. Human adenoviruses are double-stranded DNA viruses which enter cells by receptor mediated endocytosis. These viruses are particularly well suited for gene transfer because they are easy to grow and manipulate and they exhibit a broad host range in vivo and in vitro. For example, adenoviruses can infect human cells of hematopoietic, lymphoid and myeloid origin. Furthermore, adenoviruses infect quiescent as well as replicating target cells. Unlike retroviruses which integrate into the host genome, adenoviruses persist extrachromosomally thus minimizing the risks associated with insertional mutagenesis. The virus is easily produced at high titers and is stable so that it can be purified and stored. Even in the replication-competent form, adenoviruses cause only low level morbidity and are not associated with human malignancies. Accordingly, adenovirus vectors have been developed which make use of these advantages. For a description of adenovirus vectors and their uses see, e. g., Haj-Ahmad and Graham (1986) J. Virol. 57 : 267-274; Bett et al. (1993) J. Virol. 67: 5911-5921; Mittereder et al. (1994) Human Gene Therapy 5: 717-729; Seth et al. (1994) J. Virol. 68: 933-940; Barr et al. (1994) Gene Therapy 1: 51-58 ; Berkner, K. L. (1988) BioTechniques 6 : 616-629; Rich et al. (1993) Human Gene Therapy 4 : 461-476.

Adeno-associated viral vector (AAV) can also be used to administer the polynucleotides described herein. AAV vectors can be derived from any AAV serotype, including without limitation, AAV-1, AAV-2, AAV-3, AAV-4, AAV5, AAVX7, etc. AAV vectors can have one or more of the AAV wild-type genes deleted in whole or part, preferably the rep and/or cap genes, but retain one or more functional flanking inverted terminal repeat (ITR) sequences. Functional ITR sequences are necessary for the rescue, replication and packaging of the AAV virion. Thus, an AAV vector includes at least those sequences required in cis for replication and packaging (e. g., functional ITRs) of the virus. The ITR sequence need not be the wild-type nucleotide sequence, and may be altered, e. g., by the insertion, deletion or substitution of nucleotides, so long as the sequence provides for functional rescue, replication and packaging.

AAV expression vectors are constructed using known techniques to at least provide as operatively linked components in the direction of transcription, control elements including a transcriptional initiation region, the DNA of interest and a transcriptional termination region. The control elements are selected to be functional in a mammalian cell. The resulting construct which contains the operatively linked components is bounded (5'and 3') with functional AAV ITR sequences. Suitable AAV constructs can be designed using techniques well known in the art. See, e. g., U. S. Patent Nos. 5,173,414 and 5,139,941; International Publication Nos. WO 92/01070 (published 23 January 1992) and WO 93/03769 (published 4 March 1993); Lebkowski et al. (1988) Molec. Cell. Biol. 8: 3988-3996; Vincent et al. (1990) Vaccines 90 (Cold Spring Harbor Laboratory Press); Carter, B. J. (1992) Current Opinion in Biòòtechnology 3 : 533539; Muzyczka, N. (1992) Current Topics in Microbiol. and Immunol. 158 : 97- 129; Kotin, R M. (1994) Human Gene Therapy 5: 793-801; Shelling and Smith (1994) Gene Therapy 1: 165-169; and Zhou et al. (1994) J. Exp. Med. 179: 18671875.

### Pharmaceutical Preparations

Formulation of a preparation comprising the polynucleotides used in the present invention, with or without addition of an adjuvant composition, or formulation of the compound used in the invention can be carried out using standard pharmaceutical formulation chemistries and methodologies all of which are readily available to the ordinarily skilled artisan. Where appropriate the compound used in the invention may also be formulated and administered as described below.

For example, compositions containing one or more nucleic acid molecules (e.g., present in a plasmid or viral vector) can be combined with one or more pharmaceutically acceptable excipients or vehicles to provide a liquid preparation.

Auxiliary substances, such as wetting or emulsifying agents, pH buffering substances and the like, may be present in the excipient or vehicle. These excipients, vehicles and auxiliary substances are generally pharmaceutical agents that do not induce an immune response in the individual receiving the composition, and which may be administered without undue toxicity.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable excipient that serves as a stabilizer, particularly for peptide, protein or other like molecules if they are to be included in the vaccine composition. Examples of suitable carriers that also act as stabilizers for peptides include, without limitation, pharmaceutical grades of dextrose, sucrose, lactose, trehalose, mannitol, sorbitol, inositol, dextran, and the like.

Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTONS PHARMACEUTICAL SCIENCES (Mack Pub. Co:, N. J. 1991), incorporated herein by reference.

Certain facilitators of nucleic acid uptake and/or expression ("transfection facilitating agents") can also be included in, e. g., non-viral vector compositions, for example, facilitators such as bupivacaine, cardiotoxin and sucrose, and transfection facilitating vehicles such as liposomal or lipid preparations that are routinely used to deliver nucleic acid molecules. Anionic and neutral liposomes are widely available and well known for delivering nucleic acid molecules (see, e. g., Liposomes : A Practical Approach, (1990) RPC New Ed., IRL Press).

Cationic lipid preparations are also well known vehicles for use in delivery of nucleic acid molecules. Suitable lipid preparations include DOTMA (N- [1- (2, 3dioleyloxy) propyl]-N, N, N-trimethylammonium chloride), available under the tradename Lipofectin, and DOTAP (1, 2-bis (oleyloxy)-3 (trimethylammonio) propane), see, e. g., Felgner et al. (1987) Proc. Natl. Acad. Sci. USA 84: 7413-7416; Malone et al. (1989) Proc. Natl. Acad. Sci. USA 86: 6077-6081; US Patent Nos 5,283,185 and 5,527,928, and International Publication Nos WO 90/11092, WO 91/15501 and WO 95/26356. These cationic lipids may preferably be used in association with a neutral lipid, for example DOPE (dioleyl phosphatidylethanolamine). Still further transfection-facilitating compositions that can be added to the above lipid or liposome preparations include spermine derivatives (see, e. g., International Publication No. WO 93/18759) and membrane-permeabilizing compounds such as GALA, Gramicidine S and cationic bile salts (see, e. g., International Publication No. WO 93/19768).

Alternatively, the nucleic acid molecules used in the present invention may be encapsulated, adsorbed to, or associated with, particulate carriers. Suitable particulate carriers include those derived from polymethyl methacrylate polymers, as well as PLG microparticles derived from poly (lactides) and poly (lactide-co-glycolides). See, e. g., Jeffery et al. (1993) Pharm. Res. 10: 362368. Other particulate systems and polymers can also be used, for example, polymers such as polylysine, polyarginine, polyornithine, spermine, spermidine, as well as conjugates of these molecules.

The formulated vaccine compositions will thus typically include polynucleotide (e. g., a plasmid) containing a sequence encoding an antigen of interest in an amount sufficient to mount an immunological response. An appropriate effective amount can be readily determined by one of skill in the art. Such an amount will fall in a relatively broad range that can be determined through routine trials. In the case of antigens 10 ug to lg may be used, for example 100ug to 0.1g. Immune responses have been obtained using as little as 1 pg of DNA, while in other administrations, up to 2 mg of DNA has been used. It is generally expected that an effective dose of polynucleotides containing the genomic fragments will fall within a range of about 10 pg to 1000 ug. however, doses above and below this range may also be found effective. The compositions may thus contain from about 0.1 % to about 99.9% of the antigen, polynucleotide or compound of the invention. In the case of the compound of the invention 1 ug to 10g is typically administered, preferably 0.1mg to 50mg, and most preferably 1mg to 5mg.

### Administration of Pharmaceutical Preparations

Typically the compound used in the invention (generally in the amounts mentioned above) is applied (topically or transdermally) to an area having a diameter of 1 to 6cm, preferably 2 to 4 cm.

The compound is generally applied at the site where the polynucleotide is administered (or close to there, such as within 2 cm of that site). In another embodiment the compound is applied to a site which is immunologically related to the site where the polynucleotide is administered, such as at a draining lymph node from the site where polynucleotide is administered.

Administration of the above-described pharmaceutical preparations (containing the polynucleotide) can be effected in one or more doses (typically 2, 3, 4 or more doses). The compound used in the invention will be administered 12 to 36 hours after at least one of the administrations of polynucleotide, for example after at least each of 2, 3, 4 or more administrations of polynucleotide.

Delivery maybe be via conventional needle and syringe for the liquid compositions and for liquid suspensions containing particulate compositions. In addition, Various liquid jet injectors are known in the art and may be employed to administer the present compositions. Methods of determining the most effective means and dosages of administration are well known to those of skill in the art and will vary with the delivery vehicle, the composition of the therapy, the target cells, and the subj ect being treated. Single and multiple administrations can be carried out with the dose level and pattern being selected by the attending physician.

Furthermore, the polynucleotides may be combined with other suitable compositions and therapies. For instance, in order to augment an immune response in a subject, the compositions and uses described herein can further include ancillary substances (e. g., adjuvants), such as pharmacological agents, cytokines, or the like. Ancillary substances may be administered, for example, as proteins or other macromolecules at the same time, prior to, or subsequent to, administration of the polynucleotides described herein. The nucleic acid molecule compositions may also be administered directly to the subject or, alternatively, delivered ex vivo, to cells derived from the subject, using methods known to those skilled in the art.

### Coated Particles

In one embodiment, the polynucleotides (e. g., DNA vaccines), adjuvants, and/or compound used in the invention are delivered using carrier particles (e. g., core carriers). Particle mediated methods for delivering such preparations are known in the art. Thus, once prepared and suitably purified, the above-described substances can be coated onto carrier particles (e.g. core carriers) using a variety of techniques known in the art. Carrier particles are selected from materials which have a suitable density in the range of particle sizes typically used for intracellular delivery from an appropriate particle mediated delivery device. The optimum carrier particle size will, of course, depend on the diameter of the target cells. Alternatively, colloidal gold particles can be used wherein the coated colloidal gold is administered (e. g., injected) into tissue (e. g., skin or muscle) and subsequently taken-up by immune-competent cells.

For the purposes of the invention, tungsten, gold, platinum and iridium carrier particles can be used. Tungsten and gold particles are preferred. Tungsten particles are readily available in average sizes of 0.5 to 2.0 um in diameter. Although such particles have optimal density for use in particle acceleration delivery methods, and allow highly efficient coating with DNA, tungsten may potentially be toxic to certain cell types. Gold particles or microcrystalline gold (e. g., gold powder A1570, available from Engelhard Corp., East Newark, NJ) will also find use with the present methods. Gold particles provide uniformity in size (available from Alpha Chemicals in particle sizes of 1-3 um, or available from Degussa, South Plainfield, NJ in a range of particle sizes including 0.95 um) and reduced toxicity.

A number of methods are known and have been described for coating or precipitating DNA or RNA onto gold or tungsten particles. Most such methods generally combine a predetermined amount of gold or tungsten with plasmid DNA, CaCl₂ and spermidine. The resulting solution is vortexed continually during the coating procedure to ensure uniformity of the reaction mixture. After precipitation of the nucleic acid, the coated particles can be transferred to suitable membranes and allowed to dry prior to use, coated onto surfaces of a sample module or cassette, or loaded into a delivery cassette for use in a suitable particle delivery instrument.

Peptide adjuvants (e. g., cytokines), can also be coated onto suitable carrier particles, e. g., gold or tungsten. For example, peptides can be attached to the carrier particle by simply mixing the two components in an empirically determined ratio, by ammonium sulfate precipitation or other solvent precipitation methods familiar to those skilled in the art, or by chemical coupling of the peptide to the carrier particle. The coupling of L-cysteine residues to gold has been previously described (Brown et al., Chemical Society Reviews 9 : 271- 311 (1980)). Other methods include, for example, dissolving the peptide antigen in absolute ethanol, water, or an alcohol/water mixture, adding the solution to a quantity of carrier particles, and then drying the mixture under a stream of air or nitrogen gas while vortexing. Alternatively, the peptide antigens can be dried onto carrier particles by centrifugation under vacuum. Once dried, the coated particles can be resuspended in a suitable solvent (e. g., ethyl acetate or acetone), and triturated (e. g., by sonication) to provide a substantially uniform suspension.

### Administration of Coated Particles

Following their formation, carrier particles coated with either nucleic acid preparations, or peptide or protein adjuvant preparations, or the compound used in the invention are delivered to a subject, for example transdermally, using particle-mediated delivery techniques. Various particle delivery devices suitable for particle-mediated delivery techniques are known in the art, and are all suited for use in the practice of the invention. Current device designs employ an explosive, electric or gaseous discharge to propel the coated core carrier particles toward target cells. The coated particles can themselves be releasably attached to a movable carrier sheet, or removably attached to a surface along which a gas stream passes, lifting the particles from the surface and accelerating them toward the target.

An example of a gaseous discharge device is described in U. S. Patent No. 5,204,253. An explosive-type device is described in U. S. Patent No. 4,945,050. One example of an electric discharge-type particle acceleration apparatus is described in U. S. Patent No. 5,120,657. Another electric discharge apparatus suitable for use herein is described in U. S. Patent No. 5,149,655. The disclosure of all of these patents is incorporated herein by reference in their entireties.

The coated particles are administered to the subject to be treated in a manner compatible with the dosage formulation, and in an amount that will be effective to bring about a desired immune response. The amount of the composition to be delivered which, in the case of nucleic acid molecules is generally in the range of from 0.001 to 100.0 ug, more typically 0.01 to 10.0, ug of nucleic acid molecule per dose, and in the case of antigen peptide or protein molecules or compound of the invention is 1, ug to 5 mg, more typically 1 to 50, ug of peptide, depends on the subject to be treated. The exact amount necessary will vary depending on the age and general condition of the individual being immunized and the particular nucleotide sequence or peptide selected, as well as other factors. An appropriate effective amount can be readily determined by one of skill in the art upon reading the instant specification.

Thus, an effective amount of the antigens herein described, or nucleic acids coding therefor, or compound used in the invention will be sufficient to bring about a suitable immune response in an immunized subject, and will fall in a relatively broad range that can be determined through routine trials..Preferably, the coated particles are delivered to suitable recipient cells in order to bring about an immune response (e. g.; T-cell activation) in the treated subject.

### Particulate Compositions

Alternatively, the antigen, polynucleotide, adjuvant or compound used in the invention can be formulated as a particulate composition. This can be carried out using standard pharmaceutical formulation chemistries and methodologies all of which are readily available to the reasonably skilled artisan.

The particulate composition will comprise an acceptable excipient or vehicle. Auxiliary substances, such as wetting or emulsifying agents, pH buffering substances, and the like, may be present in the excipient or vehicle. These excipients, vehicles and auxiliary substances are generally pharmaceutical agents that do not themselves induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that an antigen composition will contain a pharmaceutically acceptable carrier that serves as a stabilizer, particularly for peptide, protein or other like antigens.

Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, carriers, stabilizers and other auxiliary substances is available in REMINGTONS PHARMACEUTICAL SCIENCES (Mack Pub. Co., N. J. 1991), incorporated herein by reference.

The formulated compositions will include an amount of the polynucleotide or compound of the invention which is sufficient to mount an immunological response, as defined above. An appropriate effective amount can be readily determined by one of skill in the art. Such an amount will fall in a relatively broad range, generally within the range of about 0.1 ug to 25 mg or more, and specific suitable amounts can be determined through routine trials. The compositions may contain from about 0.1 % to about 99.9% of the antigen.

If an adjuvant is included in the composition, or the methods are used to provide a particulate adjuvant composition, the adjuvant will be present in a suitable amount as described above. The compositions are then prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be densified using the techniques described in commonly owned International Publication No. WO 97/48485, incorporated herein by reference.

These methods can be used to obtain polynucleotide particles having a size ranging from about 0.1 to about 250 um, preferably about 10 to about 150 um, and most preferably about 20 to about 60 um; and a particle density ranging from about 0.1 to about 25 g/cm3, and a bulk density of about 0.5 to about 3.0 g/cm3, or greater.

Similarly, particles of antigen, adjuvants or the compound of the invention having a size ranging from about 0.1 to about 250 um, preferably about 0.1 to about 150 um, and most preferably about 20 to about 60 um ; a particle density ranging.. from about 0.1 to about 25 g/cm3, and a bulk density of preferably about 0.5 to about 3.0 g/cm3, and most preferably about 0.8 to about 1.5 g/cm3 can be obtained.

### Administration of Particulate Compositions

Following their formation, the particulate composition (e. g., powder) can be delivered transdermally to vertebrate tissue using a suitable transdermal particle delivery technique. Various particle delivery devices suitable for administering the substance of interest are known in the art, and will find use in the practice of the invention. A particularly preferred transdermal particle delivery system employs a needleless syringe to fire solid particles in controlled doses into and through intact skin and tissue. See, e. g., U. S. Patent No. 5,630,796 to Bellhouse et al. which describes a needleless syringe (also known as "the PowderJect particle delivery device"). Other needleless syringe configurations are known in the art and are described herein.

The particulate compositions can then be administered using a transdermal delivery technique. Preferably, the particulate compositions will be delivered via a powder injection method, e. g., delivered from a needleless syringe such as those described in commonly owned International Publication Nos. WO 94/24263, WO 96/04947, WO 96/12513, and WO 96/20022, all of which are incorporated herein by reference. Delivery of particles from such particle delivery devices is practiced with particles having an approximate size generally ranging from 0.1 to 250 um, preferably ranging from about 10-70 um. Particles larger than about 250 um can also be delivered from the devices, with the upper limitation being the point at which the size of the particles would cause untoward damage to the skin cells. The actual distance which the delivered particles will penetrate a target surface depends upon particle size (e. g., the nominal particle diameter assuming a roughly spherical particle geometry), particle density, the initial velocity at which the particle impacts the surface, and the density and kinematic viscosity of the targeted skin tissue. In this regard, optimal particle densities for use in needleless injection generally range between about 0.1 and 25 g/cm3, preferably between about 0.9 and 1.5 g/cm3, and injection velocities generally range between about 100 and 3,000 m/sec, or greater. With appropriate gas pressure, particles having an average diameter of 10-70 um can be accelerated through the nozzle at velocities approaching the supersonic speeds of a driving gas flow.

If desired, these particle delivery devices (e. g., a needleless syringe) can be provided in a preloaded condition containing a suitable dosage of the particles comprising the antigen of interest and/or the selected adjuvant. The loaded syringe can be packaged in a hermetically sealed container, which may further be labeled as described above.

Compositions containing a prophylactically or therapeutically effective amount of the powdered molecules described herein can be delivered to any suitable target tissue via the above-described particle delivery devices. For example, the compositions can be delivered to muscle, skin, brain, lung, liver, spleen, bone marrow, thymus, heart, lymph, blood, bone cartilage, pancreas, kidney, gall bladder, stomach, intestine, testis, ovary, uterus, rectum, nervous system, eye, gland and connective tissues. For nucleic acid molecules, delivery is preferably to, and the molecules expressed in, terminally differentiated cells; however, the molecules can also be delivered to non-differentiated, or partially differentiated cells such as stem cells of blood and skin fibroblasts.

The powdered compositions are administered to the subject to be treated in a manner compatible with the dosage formulation, and in an amount that will be prophylactically and/or therapeutically effective. The amount of the composition to be delivered, generally in the range of from 0.5 ug/kg to 100 ug/kg of nucleic acid molecule per dose, depends on the subject to be treated.

Doses may be as low as 0.5 ug for 50 kg subject, or approximately 0.01 ug/kg. Doses for other pharmaceuticals, such as physiological active peptides and proteins, generally range from about 0.1 ug to about 20 mg, preferably 10 ug to about 3 mg. The exact amount necessary will vary depending on the age and general condition of the individual to be treated, the severity of the condition being treated, the particular preparation delivered, the site of administration, as well as other factors. An appropriate effective amount can be readily determined by one of skill in the art.

Thus, a "therapeutically effective amount" of the present particulate compositions will be sufficient to bring about treatment or prevention of disease or condition symptoms, and will fall in a relatively broad range that can be determined through routine trials.

### Preferred formulations for delivery of the compound used in the invention

The compound used in the invention is administered in the form of a pharmaceutical formulation which is suitable for the topical or transdermal delivery of drugs. The formulation will comprise a therapeutically effective amount of the compound of the invention and generally also a pharmaceutically acceptable vehicle. Typically, in the formulation, the compound of the invention is present in an amount of about 0.05 to 20 per cent, preferably 0.5 percent to about 10 percent, by weight based on the total weight of said formulation. In a preferred embodiment the formulation contains about 2 to about 7 per by weight of the compound of the invention, for example about 5 per cent.

Typically the formulation is in the form of a cream, ointment or adhesive coating (such as a pressure sensitive adhesive coating or adhesive-coated sheet material). The formulation may additionally comprise substances that enhance skin penetration of drugs.

The formulation is preferably substantially non-irritating. Such a formulation will not cause unacceptable skin irritation in conventional repeat skin irritation tests in albino rabbits such as that described in Draize et al., "Appraisal of the Safety of Chemicals in Food, Drugs and Cosmetics", prepared by the Division of Pharmacology of the Food and Drug Administration, published originally in 1959 by the Association of Food and Drug Officials of the United States, Topeka, Kans. (2nd printing 1965), incorporated herein by reference.

A fatty acid such as isostearic acid, oleic acid or a mixture thereof is incorporated into a formulation of the invention. The total amount of fatty acid present in a formulation is preferably about 3 percent to about 45 percent, preferably about 5 per cent to about 25 per cent by weight based on the total weight of formulation.

The formulation is preferably a cream according containing the compound of the invention and optionally also fatty acid. The cream generally comprises an oil phase and a water phase in admixture.

Optionally, the formulation (in particular when it is a cream or ointment) can contain one or more emollients, emulsifiers, thickeners and/or preservatives.

The emollients are typically long chain alcohols, such as cetyl alcohol, stearyl alcohol and cetearyl alcohol; hydrocarbons such as petrolatum and light mineral oil; or acetylated lanolin. The total amount of emollient in the formulation is preferably about 5 percent to about 30 percent, and more preferably about 5 percent to about 10 percent by weight based on the total weight of the formulation.

The emulsifier is typically a nonionic surface active agent, e.g., polysorbate 60 (available from ICI Americas), sorbitan monostearate, polyglyceryl-4 oleate, and polyoxyethylene(4)lauryl ether or trivalent cationic. Generally the total amount of emulsifier is preferably about 2 percent to about 14 percent, and more preferably about 2 percent to about 6 percent by weight based on the total weight of the formulation.

Pharmaceutically acceptable thickeners, such as Veegum.TM.K (available from R T. Vanderbilt Company, Inc.), and long chain alcohols (i.e. cetyl alcohol, stearyl alcohol or cetearyl alcohol) can be used.. The total amount of thickener present is preferably about 3 percent to about 12 percent by weight based on the total weight of the formulation.

Preservatives such as methylparaben, propylparaben and benzyl alcohol can be present in the formulation. The appropriate amount of such preservative(s) is known to those skilled in the art.

Optionally, an additional solubilizing agent such as benzyl alcohol, lactic acid, acetic acid, stearic acid or hydrochloric acid can be included in the formulation. If an additional solubilizing agent is used, the amount present is preferably about 1 percent to about 12 percent by weight based on the total weight of the cream.

Optionally, the formulation can contain a humectant such as glycerin and skin penetration enhancers such as butyl stearate.

It is known to those skilled in the art that a single ingredient can perform more than one function in a cream, i.e., cetyl alcohol can serve both as an emollient and as a thickener.

Generally, a cream consists of an oil phase and a water phase mixed together to form an emulsion. Preferably, the amount of water present in a cream is about 45 percent to about 85 percent by weight based on the total weight of the cream.

Where the formulation is an ointment a pharmaceutically acceptable ointment base such as petrolatum or polyethylene glycol 400 (available from Union Carbide) in combination with polyethylene glycol 3350 (available from Union Carbide) can be used. The amount of ointment base present in an ointment is preferably about 60 percent to about 95 percent by weight based on the total weight of ointment.

In a preferred embodiment the formulation is a cream which comprises an oil-in-water cream base comprising isotearic acid, cetyl alcohol, stearyl alcohol, white petrolatum, polysorbate 60, sorbiton monostearate, glycerin, xanthum gum, purified water, benzyl alcohol, methylparaban and propyl-paraban. Such a cream may be in the form of Aldara imiquimod cream which contains 5% imiquimod.

In another preferred embodiment the formulation comprises about 1 percent compound used in the invention, about 10 percent of said isostearic acid, about 2 percent benzyl alcohol, about 2.2 percent cetyl alcohol, about 3.1 percent stearyl alcohol, about 2.55 percent polysorbate 60, about 0.45 percent sorbitan monostearate, about 2 percent glycerin, about 0.2 percent methylparaben, about 0.02 percent propylparaben and about 76.48 percent purified water, all percentages being based on the total weight of said formulation.

In another embodiment the formulation comprises about 1 percent compound use in the invention, about 10 percent of said isostearic acid, about 6 percent cetearyl alcohol, about 2.55 percent polysorbate 60, about 0.45 percent sorbitan monostearate, about 2 percent glycerin, about 0.2 percent methylparaben, about 0.02 percent propylparaben and about 77.78 percent purified water, all percentages being based on the total weight of said formulation.

In a further embodiment of the invention, the formulation comprises about 1 percent compound of the invention, about 10 percent of said isostearic acid about 2 percent benzyl alcohol, about 1.7 percent cetyl alcohol, about 2.3 percent stearyl alcohol, about 2.55 percent polysorbate 60, about 0.45 percent sorbitan monostearate, about 2 percent glycerin, about 0.2 percent methylparaben, about 0.02 percent propylparaben and about 77.78 percent purified water, all percentages being based on the total weight of said formulation.

The formulation may comprise about 5 percent compound of the invention, about 25 percent of said isostearic acid, about 2 percent benzyl alcohol, about 2.2 percent cetyl alcohol, about 3.1 percent stearyl alcohol, about 3 percent petrolatum, about 3:4 percent polysorbate 60, about 0.6 percent sorbitan monostearate, about 2 percent glycerin, about 0.2 percent methylparaben, about 0.02 percent propylparaben and about 53.48 percent purified water, all percentages being based on the total weight of said formulation.

Alternatively the formulation may comprise about 1 percent compound of the invention, about 5 percent of said isostearic acid, about 15 percent petrolatum, about 12.8 percent light mineral oil, about 8 percent aluminum stearate, about 4 percent cetyl alcohol, about 3 percent polyglyceryl-4 oleate, about 1 percent acetylated lanolin, about 0.063 percent propylparaben, about 1 percent Veegum K, about 0.12 percent methylparaben and about 49.02 percent purified water, all percentages being based on the total weight of said formulation.

A pressure-sensitive adhesive composition used in the invention generally comprises the compound used in the invention, fatty acid, and a pressure sensitive adhesive polymer. The amount of the compound present in a pressure sensitive adhesive composition is preferably about 0.5 percent to about 9 percent by weight, and more preferably about 3 percent to about 7 percent by weight based on the total weight of the adhesive composition. The amount of fatty acid present is preferably about 10 percent to about 40 percent by weight, more preferably about 15 percent to about 30 percent by weight, and most preferably about 20 percent to about 30 percent by weight, based on the total weight of the adhesive composition.

Preferably, the adhesive polymer utilized in a pressure sensitive adhesive composition of the invention is substantially chemically inert to the compound of the invention. The adhesive polymer is preferably present in an amount of about 55 percent to about 85 percent by weight based on the total weight of the composition. Suitable adhesive polymers include acrylic adhesives that contain, as a major constituent (i.e., at least about 80 percent by weight of all monomers in the polymer), a hydrophobic monomeric acrylic or methacrylic acid ester of an alkyl alcohol, the alkyl alcohol containing 4 to 10 carbon atoms. Examples of suitable monomers are those discussed below in connection with the "A Monomer". These adhesive polymers can further contain minor amounts of other monomers such as the "B Monomers" listed below.

Preferred adhesives include acrylic pressure-sensitive adhesive copolymers containing A and B Monomers as follows: Monomer A is a hydrophobic monomeric acrylic or methacrylic acid ester of an alkyl alcohol, the alkyl alcohol containing 4 to 10 carbon atoms, preferably 6 to 10 carbon atoms, more preferably 6 to 8 carbon atoms, and most preferably 8 carbon atoms. Examples of suitable A Monomers are n-butyl, n-pentyl, n-hexyl, isoheptyl, n-nonyl, n-decyl, isohexyl, 2-ethyloctyl, isooctyl and 2-ethylhexyl acrylates. The most preferred A Monomer is isooctyl acrylate.

Monomer B is a reinforcing monomer selected from the group consisting of acrylic acid; methacrylic acid; alkyl acrylates and methacrylates containing 1 to to 3 carbon atoms in the alkyl group; acrylamide; methacrylamide; lower alkyl-substituted acrylamides (i.e., the alkyl group containing 1 to 4 carbon atoms) such as tertiary-butyl acrylamide; diacetone acrylamide; n-vinyl-2-pyrrolidone; vinyl ethers such as vinyl tertiary-butyl ether; substituted ethylenes such as derivatives of maleic anhydride, dimethyl itaconate and monoethyl formate and vinyl perfluoro-n-butyrate. The preferred B Monomers are acrylic acid, methacrylic acid, the above-described alkyl acrylates and methacrylates, acrylamide, methacrylamide, and the above-described lower alkyl substituted acrylamides. The most preferred B Monomer is acrylamide.

In one embodiment of a pressure-sensitive adhesive composition, the pressure-sensitive adhesive copolymer containing A and B Monomers as set forth above preferably contains the A Monomer in an amount by weight of about 80 percent to about 98 percent of the total weight of all monomers in the copolymer. The A Monomer is more preferably present in an amount by weight of about 88 percent to about 98 percent, and is most preferably present in an amount by weight of about 91 percent to about 98 percent. The B Monomer in such a copolymer is preferably present in the pressure-sensitive adhesive copolymer in an amount by weight of about 2 percent to about 20 percent, more preferably about 2 percent to about 12 percent, and most preferably 2 to 9 percent of the total weight of the monomers in the copolymer.

In another embodiment of a pressure-sensitive adhesive composition, the adhesive copolymer comprises about 60 to about 80 percent by weight (and preferably about 70 to about 80 percent by weight) of the above-mentioned hydrophobic monomeric acrylic or methacrylic acid ester of an alkyl alcohol (i.e., Monomer A described above) based on the total weight of all monomers in the copolymer; about 4 to about 9 percent by weight based on the total weight of all monomers in the copolymer of a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide and N-vinyl-2-pyrrolidone; and about 15 to about 35 percent by weight (and preferably about 15 to about 25 percent by weight) of vinyl acetate based on the total weight of all monomers in the copolymer. In this embodiment the preferred acrylic or methacrylic acid ester is isooctyl acrylate and the preferred reinforcing monomer is acrylamide.

The above described adhesive copolymers are known, and methods of preparation therefor are well known to those skilled in the art, having been described for example, in U.S. Pat. No. 24,906 (Ulrich), the disclosure of which is incorporated herein by reference. The polymerization reaction can be carried out using a free radical initiator such as an organic peroxide (e.g., benzoylperoxide) or an organic azo compound (e.g., 2,2'-azobis(2,4-dimethylpentanenitrile), available under the trade designation "Vazo 52" from DuPont).

Since pressure-sensitive adhesives such as those described above are inherently rubbery and tacky and are suitably heat and light stable, there is no need to add tackifiers or stabilizers. However, such can be added if desired.

Optionally, a pressure sensitive adhesive composition can also contain one or more skin penetration enhancers such as glyceryl monolaurate, ethyl oleate, isopropyl myristate, diisopropyl adipate and N,N-dimethyldodecylamine-N-oxide, either as a single ingredient or as a combination of two or more ingredients. The skin penetration enhancer(s) preferably form a substantially homogeneous mixture with the pressure sensitive adhesive polymer or copolymer. The total amount of skin penetration enhancer(s) present in a pressure sensitive adhesive composition of the invention is preferably about 3 percent to about 25 percent by weight, more preferably about 3 percent to about 10 percent by weight based on the total weight of the adhesive composition.

When the skin penetration enhancer is a single ingredient, it is preferably a skin penetration enhancer such as isopropyl myristate, diisopropyl adipate, ethyl oleate, or glyceryl monolaurate. When a combination skin penetration enhancer is used, it is preferably a combination such as: ethyl oleate with glyceryl monolaurate; ethyl oleate with N,N-dimethyldodecylamine-N-oxide; glyceryl monolaurate with N,N-dimethyldodecylamine-N-oxide; and ethyl oleate with both glyceryl monolaurate and N,N-dimethyldodecylamine-N-oxide.

The pressure-sensitive adhesive compositions described above are preferably coated onto one surface of a suitable backing of sheet material, such as a film, to form a pressure-sensitive adhesive coated sheet material. A pressure-sensitive adhesive coated sheet material can be prepared by knife coating a suitable release liner to a predetermined uniform thickness with a wet adhesive formulation. This adhesive coated release liner is then dried and laminated onto a backing using conventional methods. Suitable release liners include conventional release liners comprising a known sheet material, such as a polyester web, a polyethylene web, or a polystyrene web, or polyethylene-coated paper, coated with a suitable silicone-type coating such as that available under the trade designation Daubert 164Z, from Daubert Co. The backing can be occlusive, non-occlusive or a breathable film as desired. The backing can be any of the conventional materials for pressure-sensitive adhesive tapes, such as polyethylene, particularly low density polyethylene, linear low density polyethylene, high density polyethylene, randomly-oriented nylon fibers, polypropylene, ethylene-vinylacetate copolymer, polyurethane, rayon and the like. Backings that are layered, such as polyethylene-aluminum-polyethylene composites are also suitable. The backing should be substantially non-reactive with the ingredients of the adhesive coating. The presently preferred backing is low density polyethylene.

The pressure-sensitive adhesive coated sheet material can be made in the form of an article such as a tape, a patch, a sheet, a dressing or any other form known to those skilled in the art.

Preferably, an article in the form of a patch is made from an adhesive coated sheet material and applied to the skin of a mammal. The patch is replaced as necessary with a fresh patch to maintain the particular desired therapeutic effect of the compound of the invention

Formulations which are suitable for use in the invention are described in the prior art, for example in US-A-5,238,944 (which discloses preferred formulations for use in the method of the invention), US-A-4,689,338, U.S. Pat. No. 4,751,087 (discloses the use of a combination of ethyl oleate and glyceryl monolaurate as a skin penetration enhancer for nitroglycerine, with all three components being contained in the adhesive layer of a transdermal patch), U.S. Pat. No. 4,411,893 (discloses the use of N,N-dimethyldodecylamine-N-oxide as a skin penetration enhancer in aqueous systems), U.S. Pat. No. 4,722,941 (discloses readily absorbable pharmaceutical compositions that comprise a pharmacologically active agent distributed in a vehicle comprising an absorption-enhancing amount of at least one fatty acid containing 6 to 12 carbon atoms and optionally a fatty acid monoglyceride), U.S. Pat. No. 4,746,515 (discloses a method of using glyceryl monolaurate to enhance the transdermal flux of a transdermally deliverable drug through intact skin).

Below are examples of specific embodiments for carrying out the present invention. The examples are offered for illustrative purposes only, and are not intended to limit the scope of the present invention in any way.

### Examples

### Materials and Method

### Plasmids

For immunization with the Hepatitis B surface antigen (HBsAg) a plasmid containing the HCMV promoter/enhancer was used to drive expression of the HBsAg. For one experiment we also employed a plasmid in which the keratin 14 promoter was used instead of the HCMV promoter. This promoter is expected to have a more restricted pattern of expression and be expressed only in the skin unlike the HCMV promoter that is expressed in many cell types.

For immunization with HSV-2 antigens in some cases single gene plasmids expressing either the gD or gB proteins using the HCMV promoter were used. The single gene plasmids were created by amplification of genomic sequences by PCR and insertion of the PCR fragment into the pTARGET expression vector. In some instances genomic fragments of HSV-2 were used for immunization which we call subgenomic vaccines. These do not use the HCMV promoter but rather use the native promoters from the genes present in the fragment. The genomic fragments are cloned in the Super Cos backbone from Stratagene. The subgenomic vaccine has a genomic segment of approximately 36,000 bases containing nucleotides 110,931-147,530 of the genome.

In some experiments plasmids were used that contained the Cholera toxin (CT) genes A and B subunits. Another plasmid used expressed the HSP70 gene. This gene was cloned from a RT-PCR reaction of RNA obtained from mouse splenocytes, and was cloned into the pTARGET vector.

All DNA constructs were purified from bacterial extracts using purification kits (Qiagen) and purity was assessed by agarose gel electrophoresis of whole or digested plasmids and by determining the A260/A280 ratios.

### Topical Adjuvants

Imiquimod was obtained in the form of Aldara cream from a prescription. To apply, the cream was rubbed onto the abdomens of mice that had been clipped using a cotton swab. The cream is a 5% solution. About 20 ul was used, and so approximately 1 mg was given to each mouse.

### Preparation of DNA vaccines

Precipitation of DNA onto gold particles was achieved using standard procedures for the calcium/speimidine formulation of DNA vaccines. DNA was mixed with 2 micron gold particles in a small centrifuge tube containing 300 ml of 50 mM spermidine. The amount of DNA added is 2 ug per mg gold particles and typically batches of 26 mg gold (52 ug of DNA) were made. The DNA was precipitated onto gold by the addition of a 1/10 volume of 10% CaCl₂ during continuous agitation of the tube on a rotary mixer. DNA-gold complexes were washed three times with absolute ethanol then loaded into Tefzel tubing, dried and cut into 0.5 inch segments for use in the XR-1 device.

Instances where more than one DNA was used was done by first mixing the DNA constructs then precipitation onto gold. This puts the DNAs onto the same particle.

For immunization, DNA vaccines were delivered by the XR-1 device into the abdomen of Balb/C mice. A single shot was given for the immunization. Some experiments employed a prime only, and others had a prime and a boost at 4 weeks. Samples were collected from animals two weeks after the final immunization.

### Antibody ELISA

Serum samples were assayed for antibodies using an ELISA assay. Falcon Pro Bind microtiter plates were coated overnight at 4 °C with antigen in PBS (phosphate buffered saline, BioWhittaker). For HBsAg ELISA the antigen was purified HBsAg (BioDesign) at 0.1 ug per well, and for HSV ELISAs the antigen was 5 ug per well of an infected cell extract (Advanced Biotechnolgies Incorporated). The plates were blocked for 1 hour at RT with 5% dry milk/PBS then washed 3X with wash buffer (10 mM Tris Buffered saline, 0.1% Brij-35) and serum samples diluted in dilution buffer (2% dry milk/PBS/0.05 % Tween 20) were added to the plate and then incubated for 2 hours at RT.

Plates were washed 3X and a biotinylated goat anti-mouse antibody (Southern Biotechnology) diluted 1:8000 in dilution buffer was added to the plate and incubated for 1 hr at RT. Following the incubation, plates were washed 3X, then a Streptavidin-Horseradish peroxidase conjugate (Southern Biotechnology) diluted 1:8000 in PBS was added and the plate incubated a further 1 hr at RT. Plates were washed 3X, then substrate solution added (BioRad) and the reaction was stopped with 1N H₂SO₄. Optical density was read at 450 nm. Endpoint titers for HBsAg were calculated by comparison of the samples with a standard of known titer.

### Cell Culture

Single cell suspensions were obtained from mouse spleens. Spleens were squeezed through a mesh to produce a single cell suspension and cells were then sedimented, and treated with ACK buffer (Bio Whittaker, Walkersville MD) to lyse red blood cells. The cells were then washed twice in RPMI 1640 media supplemented with HEPES, 1 % glutamine (Bio Whittaker), and 5% heat inactivated fetal calf serum (FCS, Harlan, Indianapolis IN). Cells were counted, and resuspended to an appropriate concentration in "Total" media consisting of RPMI 1640 with HEPES and 1% glutamine, supplemented with 5% heat inactivated FCS, 50 mM mercaptoethanol (Gibco-BRL, Long Island NY), gentamycin (Gibco-BRL), 1 mM MEM sodium pyruvate (Gibco-BRL) and MEM non-essential amino acids (Sigma, St. Louis MO). Cell suspensions were then utilized in various immunoassays. For CD8 specific assays cells were cultured *in nitro* in the presence of a peptide corresponding to a known CD8 epitopes. For HBsAg in BALB/C mice the sequence of the peptide was IPQSLDSWWTSL (QCB Inc). For HSV CD8 responses in Balb/C mice the HGPSLYRTF peptide found in ICP27 was used. Peptides were made up in DMSO (10 mg/ml) and diluted to 10 ug/ml in culture medium.

### ELISPOTs

For IFN-g ELISPOTs assays Millipore Multiscreen membrane filtration plates were coated with 50 ul of 15 ug/ml anti-IFN-g antiserum (Pharmingen) in sterile 0.1M carbonate buffer pH 9.6, overnight at 4 °C. Plates were washed 6X with sterile PBS and then blocked with tissue culture medium containing 10% fetal bovine serum (FBS) for 1-2 hr at RT. The medium was removed and spleen cells dispensed into the wells with a total of 1X10⁶ cells per well. For wells in which less than 1X10⁶ cells from immunized animals was added, cells from naive animals were used to bring the total to 1X10⁶. Cells were incubated overnight in a tissue culture incubator in the presence of the peptide as described above. Plates were washed 2X with PBS and 1X with distilled water. This was followed with 3 washes with PBS. Biotinylated anti IFN-g monoclonal antibody (Pharmingen) was added to the plate (50 ul of a 1 ug/ml solution in PBS) and incubated for 2 hr at RT. Plates were washed 6X with PBS then 50 ul of a Streptavidin Alkaline phosphatase conjugate (1:1000 in PBS, Pharmingen) was added and incubated for 2 hr at RT. Plates were washed 6X with PBS and the color substrate (BioRad) was added and the reaction was allowed to proceed until dark spots appeared. The reaction was stopped by washing with water 3X. Plates were air dried and spots counted under a microscope.

### IFN-g ELISA

For the CD8 IFN-g ELISA cells were cultured overnight in round bottom 96 well tissue culture plates in the presence of the peptide. Samples of the supernatant were taken and used for the determination of IFN-g levels. High binding plates (Costar) were coated with 100 ul of 0.5 ug/ml of anti-mouse IFN-g antibody (Pharmingen)in bicarbonate buffer pH 9.6. Plates were blocked for 1 hr at RT with tissue culture medium containing 10% FBS then washed 3X with the TBS wash buffer. Supernatant samples obtained from cultured cells were diluted in tissue culture medium and loaded onto the plate and incubated for 2 hr at RT. Plates were washed 3X with wash buffer and a secondary antibody (0.5 ug/ml of biotinylated rat anti-mouse INF-g in PBS, Pharmingen) was added to the plates and incubated for 1 hr at RT. Plates were washed 3X, and a Streptavidin-horseradish peroxidase conjugate (1:2000 in PBS, Southern Biotechnology) was added for 1 hr at RT. Plates were washed 3X, then substrate solution added (BioRad) and the reaction was stopped with IN H₂SO₄. Optical density was read at 450 nm.

When IFN-g ELISAs were carried out on cells stimulated with the UV-inactivated HSV-2, the supernatants from the proliferation plates (described below) were used as a source of the experimental IFN-g. Otherwise the procedure was the same as described above.

### Proliferation assays

For proliferation cells were plated at a concentration of 3 X 10⁵ cells/well in Costar 96 well plates (Corning Incorporated, Coming NY). UV-inactivated virus (original moi 2), or infected cell protein extract (2 and 0.5 ug per well) was added to triplicate wells and the cells incubated at 37°C in a CO₂ atmosphere for 3 days. Then methyl-³H thymidine (NEN, Boston MD) was added (0.5 mC well) and cells were cultured for a further 18 hr before plates were processed. Stimulation indices were calculated by taking the average cpm of triplicate wells for cells stimulated by antigen, divided by the average of triplicate wells of cells cultured with medium only.

### Example 1

### Initial experiment to investigate potential adjuvant activity of imiquimod

Mice were immunized with a DNA vaccine containing a HBsAg expressing plasmid using either a high dose (lug) or low dose (50 ng) of vaccine. Treatment with Aldara 5% imiquimod cream was either 1 day before immunization (day -1), on the day of immunization (day 0), 1 day following or 2 days following immunization. One group was also treated for three days (day 0, 1 and 2). Mice were given a single dose of vaccine and were sacrificed 2 weeks later to measure immune responses. Results shown in Figure 1 are from the CD8 IFN-g ELISA. From this data there was an indication that the Aldara could boost cellular immune responses. Some effect was found at day 0 but day 1 appeared to be better although this was largely due to two strong responders. The values are the means from 4 individual mice.

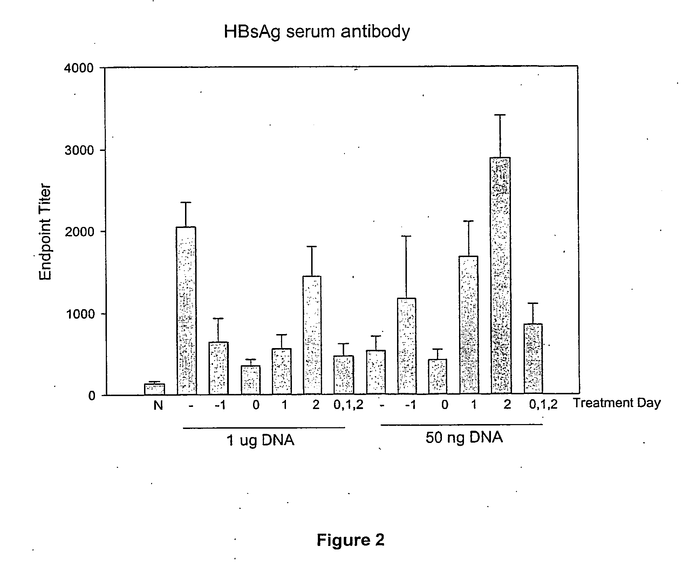

The serum antibody was also tested (Figure 2), but because these are only 2 weeks after prime they tended to be variable.

### Example 2.

### Imiquimod enhances cellular responses when applied one day after immunisation

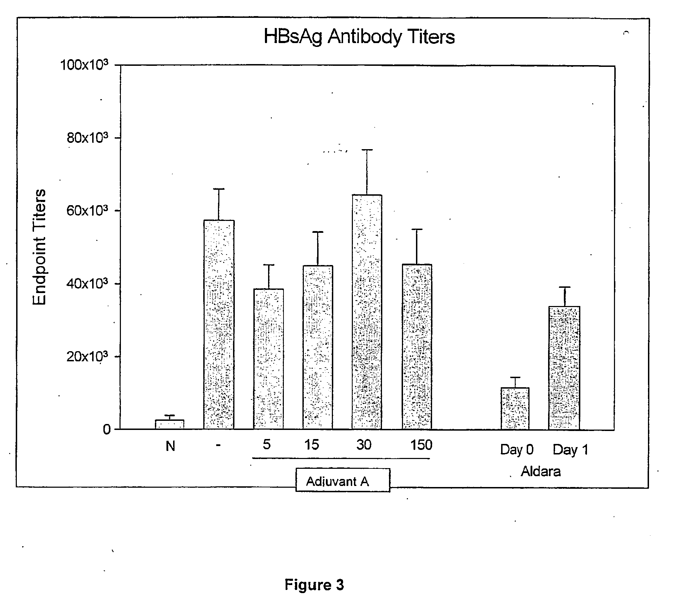

The experiment described in Example 1 was repeated. In addition various doses of another adjuvant (adjuvant A) were tested. The animals were given a prime and boost using a plasmid expressing the HBsAg gene from the HCMV promoter. Antibody levels were slightly affected by adjuvant A but in this instance the effect of imiquimod can be clearly seen and a strong reduction in antibody levels was found (Figure 3).

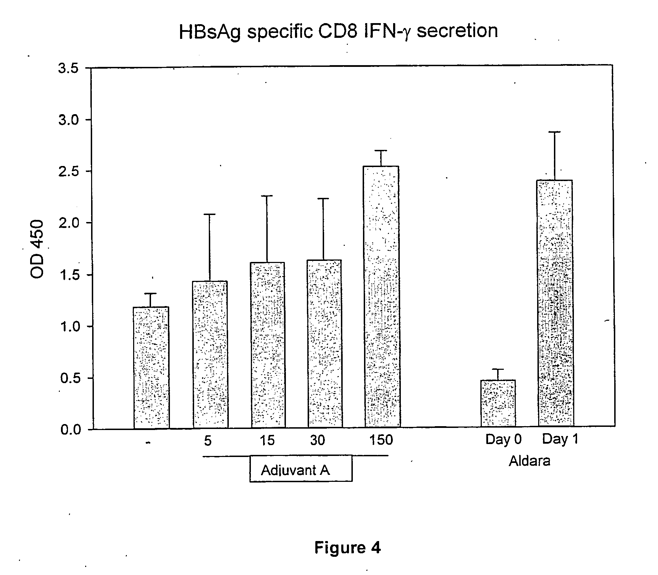

The cellular response shown by both the IFN-g ELISA and ELISPOTs indicated that the treatment with imiquimod enhanced the responses. The animals treated with Aldara one day after immunization showed again the strongest effect (Figure 4).

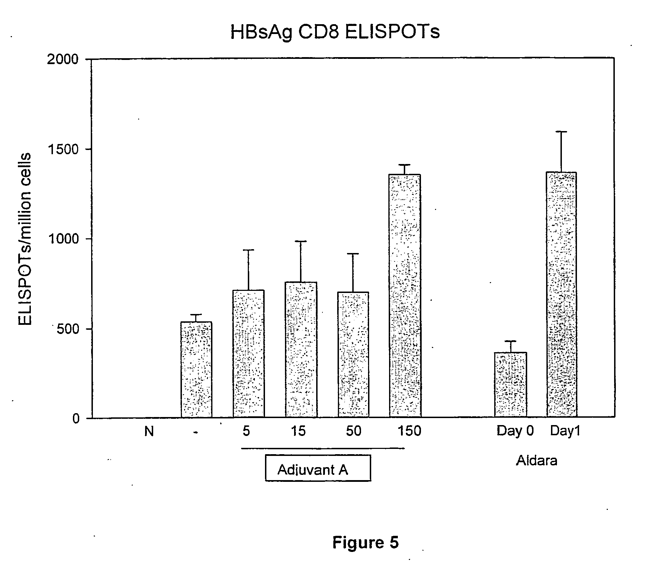

This is confirmed by the data shown in Figure 5. In all the following experiments the Aldara cream was given on Day 1 after immunization.

### Example 3.

### The effect of imiquimod on a variety of different boost and prime strategies.

This experiment includes a plasmid that expresses HBsAg from the keratin 14 promoter. This was used to try and alter the expression of the antigen in different cell types and see if this affects the immune response. Mice were immunized twice. Some were treated with Aldara cream at prime only, some were given Aldara only at boost and one group received Aldara at both prime and boost.

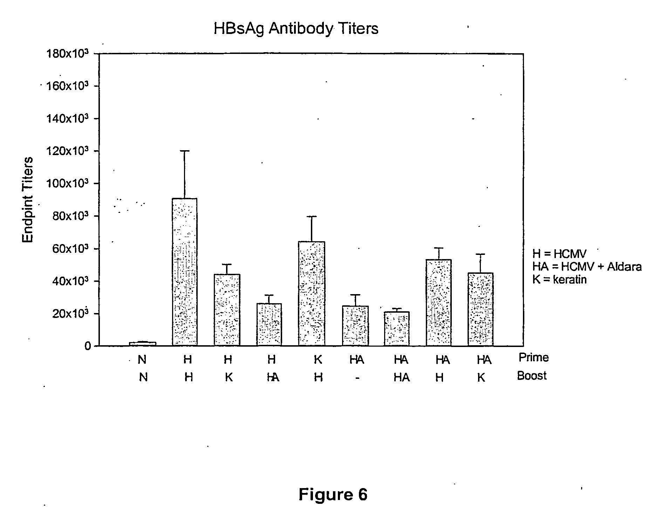

Results are shown in Figure 6. Antibody titers were not enhanced by the treatment and as often found they were in fact reduced. The most pronounced reduction for mice given two immunizations is the group given Aldara cream at prime and at boost. In the Figures the naïve animals are labeled as "N" and animals labeled as HA,- (sixth group) were given only a single immunization.

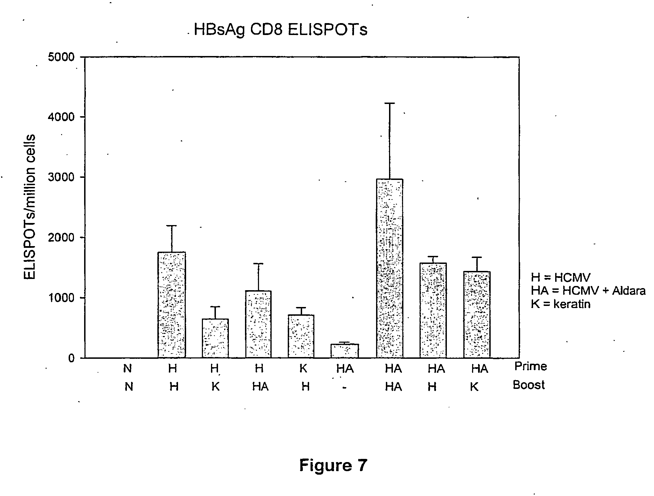

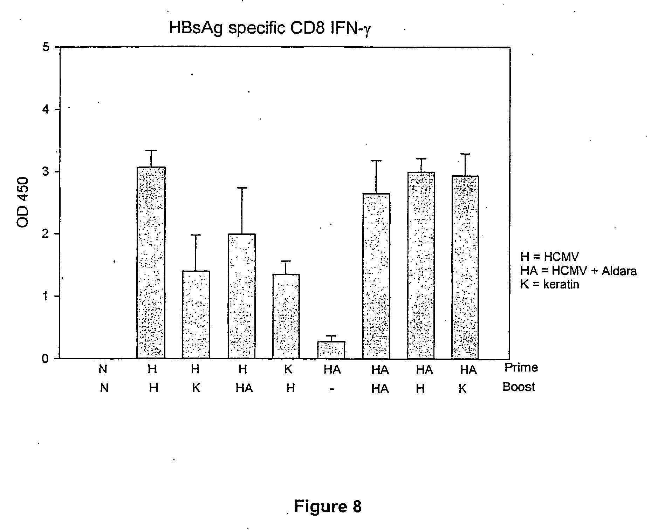

When cellular immune responses were measured (Figure 7 and 8) we did not get the expected pattern because the control group given two immunizations with HCMV plasmid (H,H) had responses considerably higher than found in the past (see previous experiment). The abnormally high controls made it impossible to interpret the Aldara effects although if one considers that the H,H control should be more like the H,K group beside it an effect of the Aldara can be extrapolated. Overall, it appears that the Aldara enhances cellular responses, and inhibits antibody responses.

### Example 4

### Using imiquimod to enhance responses to HSV-2 antigens

Three types of DNA vaccine used in this experiment. A single gene gD plasmid that expresses the full length glycoprotein which would be a membrane protein. A single gene gB plasmid that expresses a truncated form of the protein so that it is secreted from the cell. The third type of vaccine is a subgenomic vaccine (SV) that contains a genomic fragment of HSV-2. Within this fragment is the gene for gD, but not gB. Other genes of interest in this vaccine are the immediate early genes ICP0, 4 22 and 27. The adjuvants used were Aldara cream applied topically, the CT genes co-delivered on the same gold particles as the vaccine and another adjuvant (adjuvant B) given on day 0, 1 and 2.

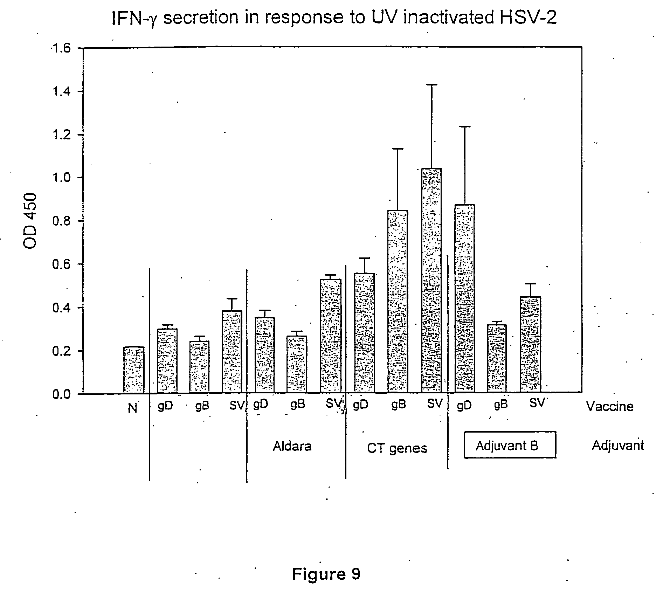

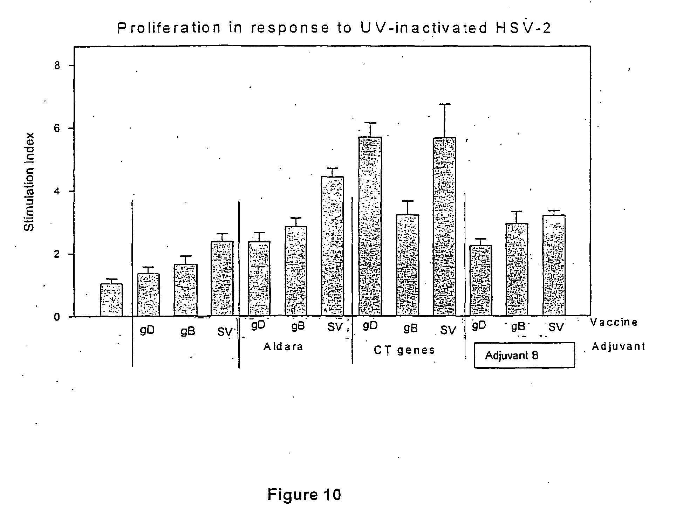

Cellular immune measures (Figures 9 and 10) show that the CT adjuvants are superior to the other adjuvants although some activity for the Aldara cream was apparent.

### Example 5

### Testing a panel of adjuvants

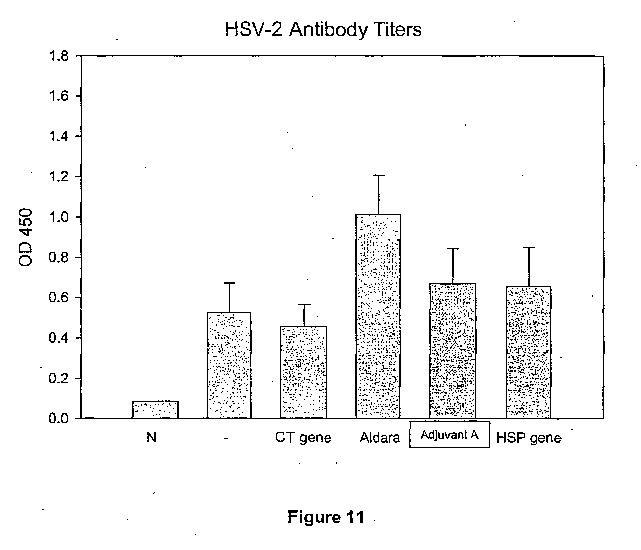

A panel of adjuvants were tested for activity with a HSV-2 vaccine which was the subgenomic vaccine. In this case adjuvant A and the HSP gene were used as well as Aldara cream and the CT genes. All adjuvants were given at a dose in which we had previously seen optimal effects in experiments with the HBsAg vaccines. The Aldara was applied one day after immunization, adjuvant A (150 ng) was given on the day of immunization, the CT and HSP genes were co-delivered with the subgenomic vaccine in 9:1 1 and 20:1 1 ratios of vaccine: adjuvant respctively. Animals were given a prime and boost. Antibody responses are typically low from the vaccine and none of the adjuvants showed a strong ability to boost these (Figure 11).

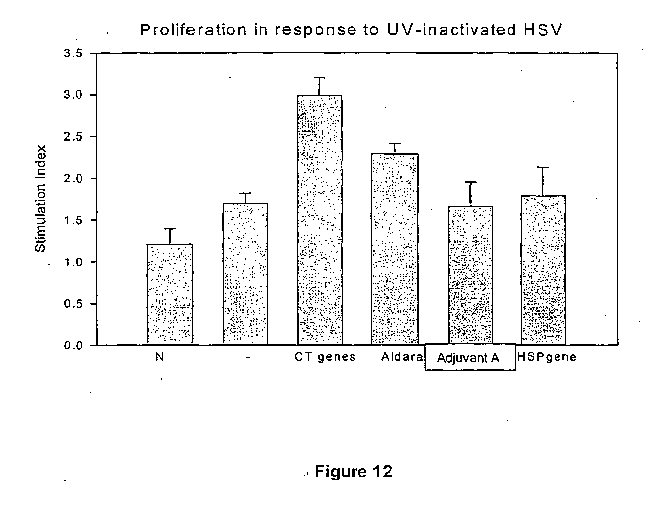

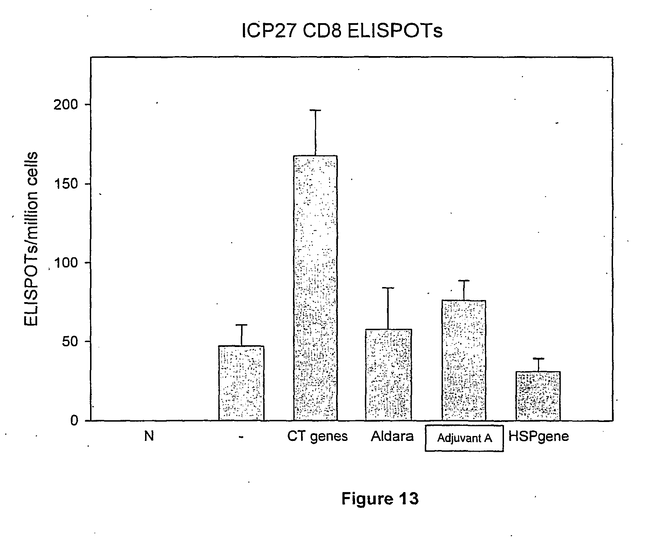

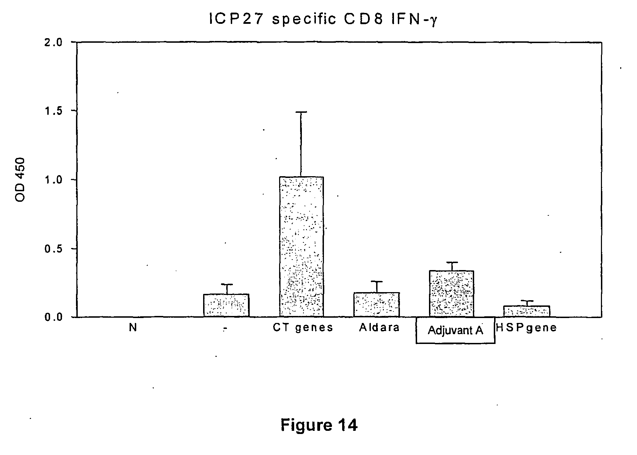

Cellular immune responses were measured by proliferations in response to UV-inactivated HSV-2 as well as specific CD8 responses to the ICP27 protein expressed from the vaccine (Figures 12 to 14).

### Example 6

### Testing antibody subclass

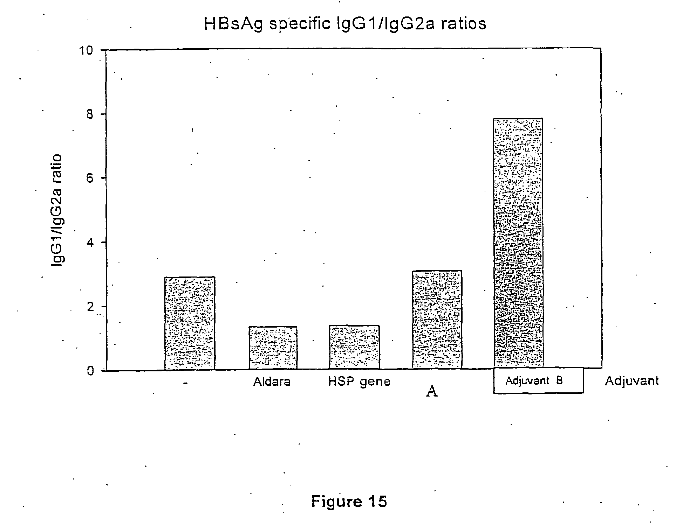

An ELISA was done on serum samples from different experiments to look at the subclass of the HBsAg specific antibody. Unadjuvanted responses from the DNA vaccine show a ratio of IgG1/IgG2a of about 3. A stronger cellular bias of an immune response is indicated by a stronger IgG2a response so the ratio would thus approach 1 or below. Of the adjuvants we have worked with the Aldara is one in which this ratio does drop indicating that it is able to bias the immune response towards a cellular response (Figure 15). PGE2 for example does not have the same effect overall even though it can enhance cellular responses as the Aldara does, but it does not seem to shift the immune response as much. This feature of imiquimod may be of importance when an antibody response is detrimental, or a greatly biased cellular response is needed.

### Example 7

### Further characterisation of the adjuvants activity of imiquimod

Two further experiments were performed to assess the efficacy of imiquimod (Aldara cream) as an adjuvant for particle-mediated immunization with DNA (PMID). Both experiments used a plasmid expressing HBV sAg and cAg as a test vaccine. The dosage of DNA, the formulation of Aldara (neat vs. diluted with control cream), and the time of Aldara administration were varied. Readouts included antibody titers and cytokine ELISPOTs to the two antigens. As expected from the results in previous Examples imiquimod primarily enhanced the IFN-g ELISPOT response with little effect on antibody titers and IL-4 ELISPOTs. The results also showed that delivering Aldara one or seven days after PMID was generally more effective than control cream or Aldara delivered at the time of PMID administration.

In order to test the efficacy of imiquimod as an adjuvant for PMID, HBV sAg-and cAg-encoding DNA was administered using an ND5.5 device and imiquimod was administered as Aldara cream. In the first experiment, different doses of DNA and times of administration of Aldara were tested (Table 1). In the second experiment, a single dose of HBV-encoding DNA (2 µg) was tested along with treatment with Aldara at 1 day post-PMID vaccination, diluted Aldara at the same time as PMID, and Aldara 1 week post-PMID (Table 2).

Groups of 5-10 Balb/c mice were vaccinated on the shaved abdomen with one shot each on day 0 (prime only) or days 0 and 28 (prime and boost). PMID was administered using an ND5.5 device with a payload of 1 mg Au and a 35 bar cylinder. Aldara was administered at the doses and times indicated in the tables above. Imiquimod was administered as Aldara cream (5% imiquimod) obtained from a pharmacist. In experiment one, a small, unmetered amount of Aldara was delivered at the indicated times using a cotton swab to rub the cream in. In experiment two, 20 ml of Aldara was measured and applied to the mice. The control cream was an over the counter hand cream which contained many of the same ingredients as the Aldara base cream. In the group where the Aldara was diluted, a 5:1 mix of control cream and Aldara was emulsified between two syringes using a three-way stopcock. '

Mice were bled retro-orbitally at 4 weeks for prime / boost groups. Blood was collected by intracardiac puncture, and spleens were removed for cellular assays at sacrifice at either week 2 (prime only) or week 6 (boost + 2 weeks). Sera were analyzed for anti-sAg and-anti-cAg antibodies using in-house ELISAs. Spleen cells from individual mice were stimulated with protein or peptide for g-IFN and IL-4 ELISPOT assays. Three sets of antigen were used: 1) intact cAg from Biodesign; 2) a library of overlapping sAg peptides (15 mers offset by 3 amino acids synthesized by Chiron); and 3) an immunodominant peptide from sAg (amino acids 28-39) known to bind the MHC class I molecule L^{d}. Specific spot-forming cells were calculated by subtracting the number of spots generated with cells cultured in medium alone.

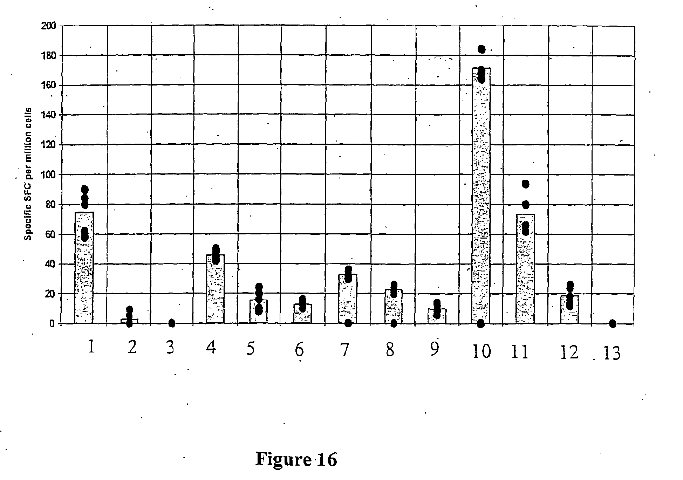

As observed previously, imiquimod did not enhance antibody responses to either HBV antigen (data not shown): In the faust experiment, there was also no effect of imiquimod on IL-4 secretion, as measured by ELISPOT assay (also not shown). As shown in Figure 16, there was an enhancement of the frequency of cells which secreted g-IFN in response to antigen. The response to sAg peptide library (primarily CD4+ cells) is shown but comaparable responses were seen with a class I-restricted peptide which stimulates CD8+ cells (not shown). At the low doses of HBV-encoding DNA, imiquimod enhances g-IFN secretion when administered at all three time points, although administration 24 hours post PMID is the most effective. At the highest dose of DNA, imiquimod administration is slightly inhibitory when delivered right before or right after but augments PMID when given 24 hours after.

In the second experiment, prime / boost regimens were attempted as well as lowering the dose of imiquimod by mixing Aldara with a control cream. It should be noted that as we had no way of measuring imiquimod, we are uncertain as to how effective the mixing was. In this study, the g-IFN ELISPOT responses of the mice given control cream was higher than normal after prime only. Therefore, there was no augmentation of the response by Aldara (data not shown). This is, unfortunately, not consistent with the first study. In the animals that were boosted, however, augmentation of the frequency of g-IFN secreting cells was seen when Aldara was administered at the following times: 24 hours after prime only; 24 hours after both the prime and boost; 24 hours after only the boost; and seven days after the boost. The largest effect occurred with the later doses of Aldara (1 and 7 days post-boost) (Figure 17).

As we have not tested ELISPOTs at longer time points after boost and Aldara treatment, we do not know if this effect is transient. As in experiment one, the CD8+ responses were similar to the CD4+ responses with the exception that Aldara delivered 24 hours after the boost only was not higher than when it was delivered after prime only. Unlike experiment one (prime only studies), augmentation of IL-4 ELISPOT responses was seen after boost, particularly with the group treated 7 days after the boost (data not shown).

These experiments show that imiquimod augments the frequency of g-IFN-secreting cells following PMID vaccination. The optimal time point for this effect seems to be about a day and upto 7 days after PMID.

**Table 1**

| **Group** | **HBV sAg/cAg plasmid (**µ**g)** | **Control Vector (µg)** | **Aldara (time relative to PMID)** |
|---|---|---|---|
| **1** | **2** | **0** | **0** |
| **2** | **0.2** | **1.8** | **0** |
| **3** | **0.02** | **1.98** | **0** |
| **4** | **2** | **0** | **Right before** |
| **5** | **0.2** | **1.8** | **Right before** |
| **6** | **0.02** | **1.98** | **Right before** |
| **7** | **2** | **0** | **Right after** |
| **8** | **0.2** | **1.8** | **Right after** |
| **9** | **0.02** | **1.98** | **Right after** |
| **10** | **2** | **0** | **One day after** |
| **11** | **0.2** | **1.8** | **One day after** |
| **12** | **0.02** | **1.98** | **One day after** |
| **13** | **0** | **2** | **0** |

**Table 2**

| **Group** | **Aldara Treatment (T=Time post vaccination)** | **Day of Sacrifice** |
|---|---|---|
| **1** | **Control cream T=24 hr** | **14 post-prime** |
| **2** | **Aldara neat T=24 hr** | **14 post-prime** |
| **3** | **Aldara diluted 1:5 T=0** | **14 post-prime** |
| **4** | **Aldara T=7 days** | **14 post-prime** |
| **5** | **Naive** | **14 post-prime** |
| **6** | **Control cream T=24 hr** | **14 post-boost** |
| **7** | **Aldara neat T=24 hr at prime only** | **14 post-boost** |
| | | |
| **8** | **Aldara neat T=24 hr at prime and boost** | **14 post-boost** |
| **9** | **Aldara neat T=24 hr at boost only** | **14 post-boost** |
| **10** | **Aldara T=7 days post-boost** | **14 post-boost** |
| **11** | **Naive** | **14 post-boost** |

### Example 8

### Optimisation of p55 gag (p17, p24, p13) to resemble codon usage of highly expressed human genes

A synthetic gene coding for the p55gag antigen of the HIV- clade B strain HXB2(GenBank entry K03455) was optimised for expression in mammalian cells was assembled from overlapping oligonucleotides by PCR. Optimisation involved changing the codon usage pattern of the viral gene to give a codon frequency closer to that found in highly expressed human genes. Codons were assigned using a statistical Visual Basic program called Syngene (an updated version of Calcgene, written by R.S. Hale and G. Thompson, Protein Expression and Purification Vol. 12 pp 185-188, 1998).

The 1528bp gag PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

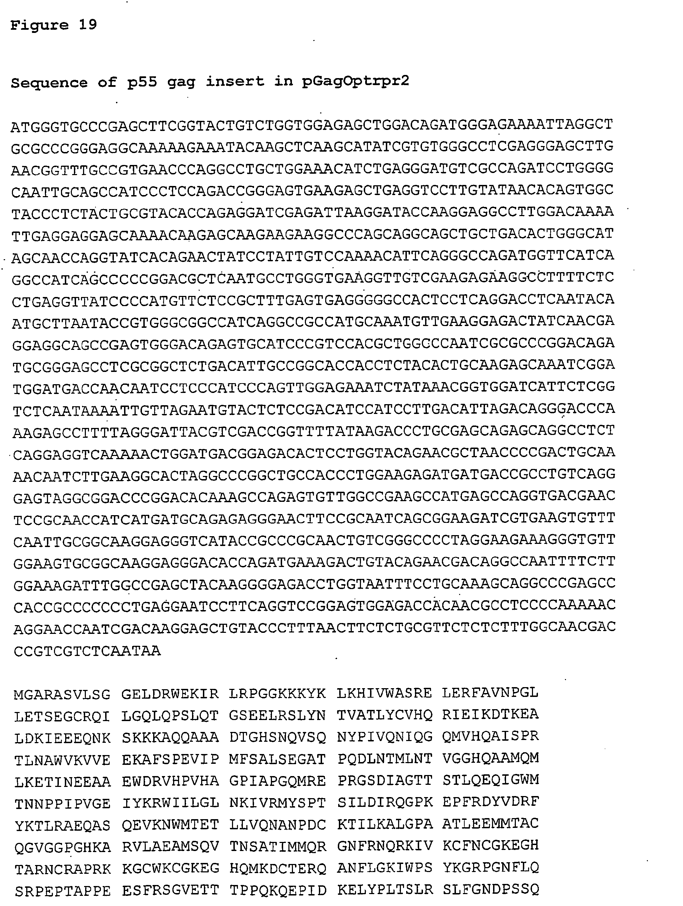

Clones were sequenced and checked for errors. No single clone was 100% correct. Regions of correct sequence from two clones were therefore combined by overlapping PCR using appropriate combinations of the optimisation oligo set to give a full length codon optimised gag gene. This final clone was subsequently found to contain a single nucleotide deletion which resulted in a frame shift and premature termination of translation. The deletion was repaired by cutting out the region of the gene containing the incorrect sequence and cloning in the correct sequence from the equivalent region of another clone. This gave the final codon optimised p55 gag clone: Gagoptrpr2 (see Figure 19).

### Example 9

### Production of a p17/p24 truncated Nef fusion gene

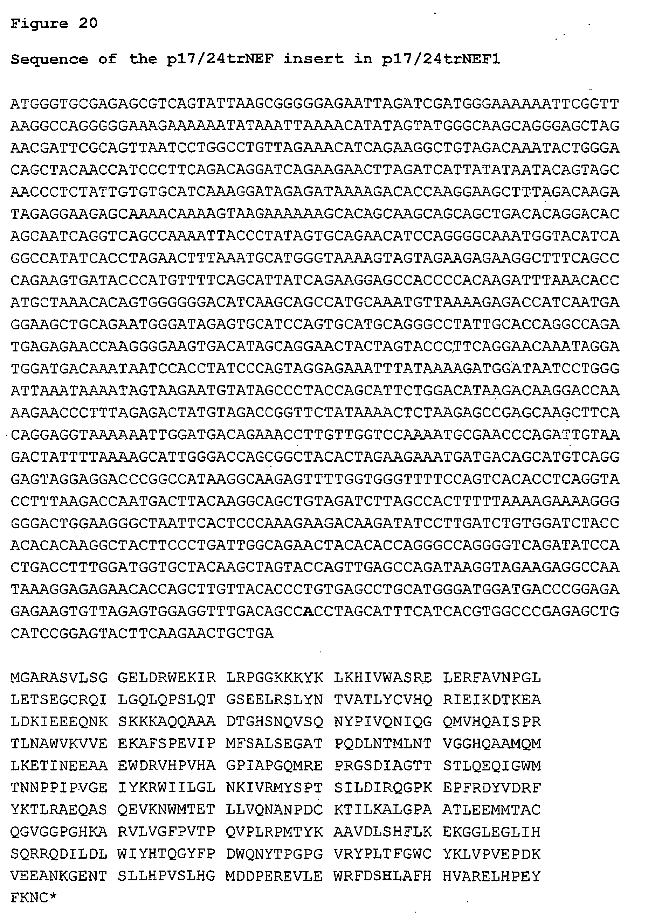

The p17 and p24 portions of the p55gag gene derived from the HIV-1 clade B strain HXB2 was PCR amplified from the plasmid pHXB?Pr (B.Maschera, E Furfine and E.D. Blair 1995 J.Virol 69 5431-5436). pHXB?Pr. 426bp from the 3' end of the HXB2 nef gene were amplified from the same plasmid. Since the HXB2 nef gene contains a premature termination codon two overlapping PCRs were used to repair the codon (TGA [stop] to TGG [Trp]). The p17/p24linker and trNEFlinker PCR products were joined to form the p17p24trNEF fusion gene (figure 20) in a PCR reaction (antisense).

The 1542bp product was gel purified, cut with restriction endonucleases NotI and BamHI and cloned into the NotI BamHI sites of vector WRG7077. This places the gene between the CMV promoter/intron A and the Bovine growth hormone polyadenylation signal.

### Example 10

### Production of an Gag p17/24opt/trNef1 ('Gagopt/Nef) fusion gene.

The p17/p24 portion of the codon optimised p55gag gene derived from the HIV-1 clade B strain HXB2 was PCR amplified from the plasmid pGagOPTipr2. The truncated HXB2 Nef gene with the premature termination codon repaired (TGA [stop] to TGG [Trp]) was amplified by PCR from the plasmid 7077trNef20. The two PCR products were designed to have overlapping ends so that the two genes could be joined in a second PCR.

The 1544bp product was gel purified, cut with restriction endonucleases NotI and BamHI and cloned (see figures) into the NotI BamHI sites of vector WRG7077. This places the gene between the CMV promoter/intron A and the Bovine growth hormone polyadenylation signal.

### Example 11

### Plasmid: p7077-RT3 Clone #A

A synthetic gene coding for the RT portion of the pol gene of HIV-1 clade B strain HXB2, optimised for expression in mammalian cells assembled from overlapping oligonucleotides by PCR. The sequence cloned is equivalent to positions 2550-4222 of the H2 reference sequence (GenBank entry K03455). To ensure expression the cloned sequence has two additional codons at the 5' end not present in the original gene - AUG GGC (Met Gly).

Optimisation involved changing the codon usage pattern of the viral gene to give a codon frequency closer to that found in highly expressed human genes, but excluding rarely used codons. Codons were assigned using a statistical Visual Basic program called Syngene ( an updated version of Calcgene, written by R.S. Hale and G. Thompson, Protein Expression and Purification Vol. 12 pp 185-188, 1998). The final clone was constructed from two intermediate clones, # 16 and #21.

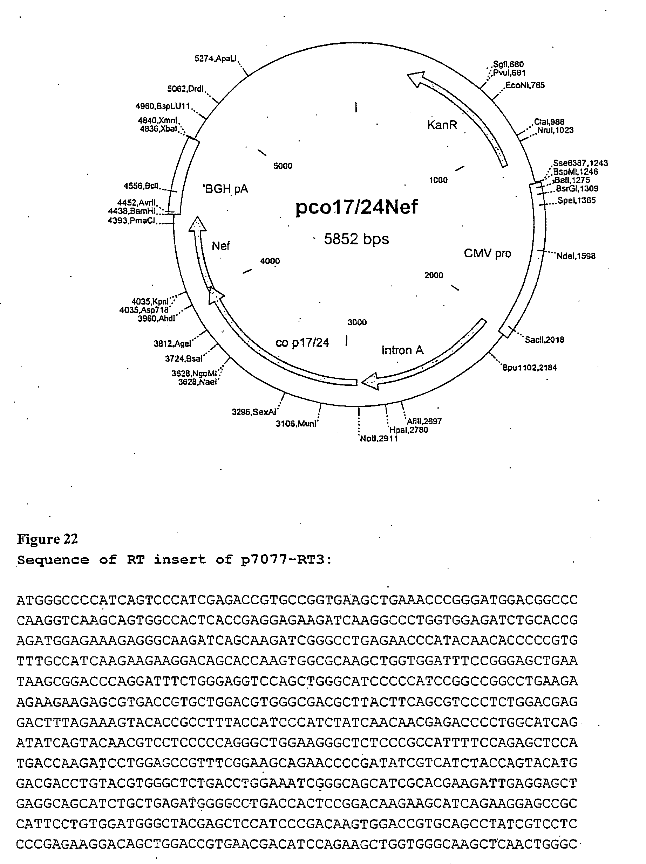

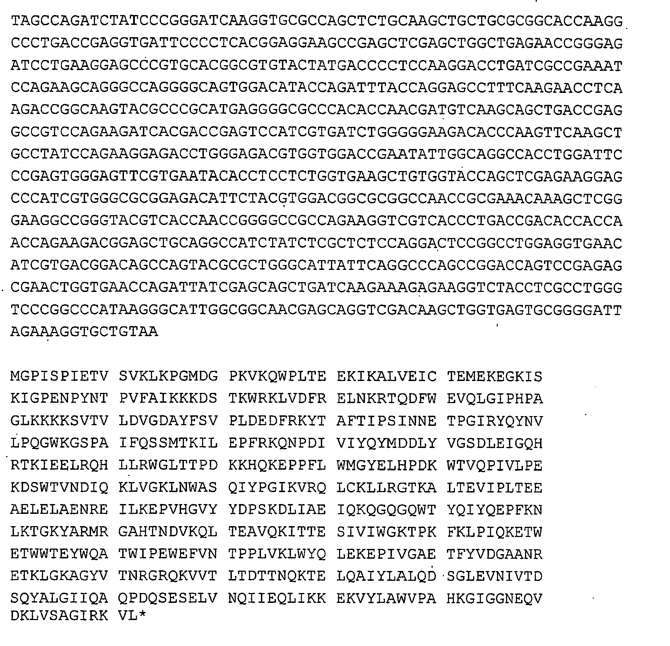

The 1.7kb PCR products were gel purified, cut with NotI and BamHI and PCR cleaned, before being ligated with NotI / BamHI cut pWRG7077. This places the gene between the CMV promoter and bovine growth hormone polyadenylation signal. Clones were sequenced. No clone was 100% correct, but clone #16 was collected by replacing the 403bp KpnI-BamHI fragment containing 3 errors with a correct KpnI-BamHI fragment from clone#21. The final clone was verified by sequencing. (see figure 22).

### Example 12

### Optimised RT

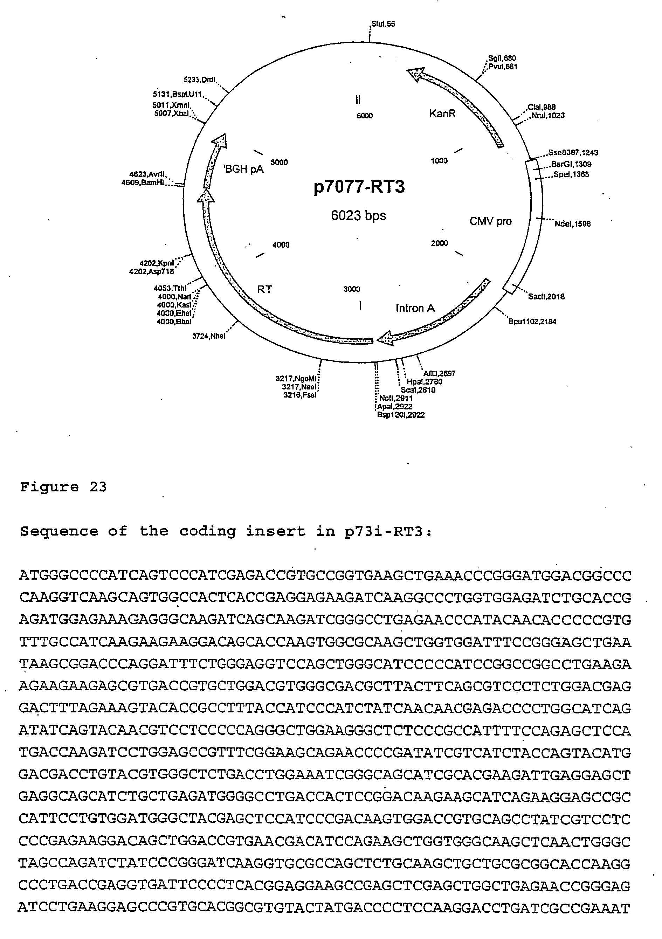

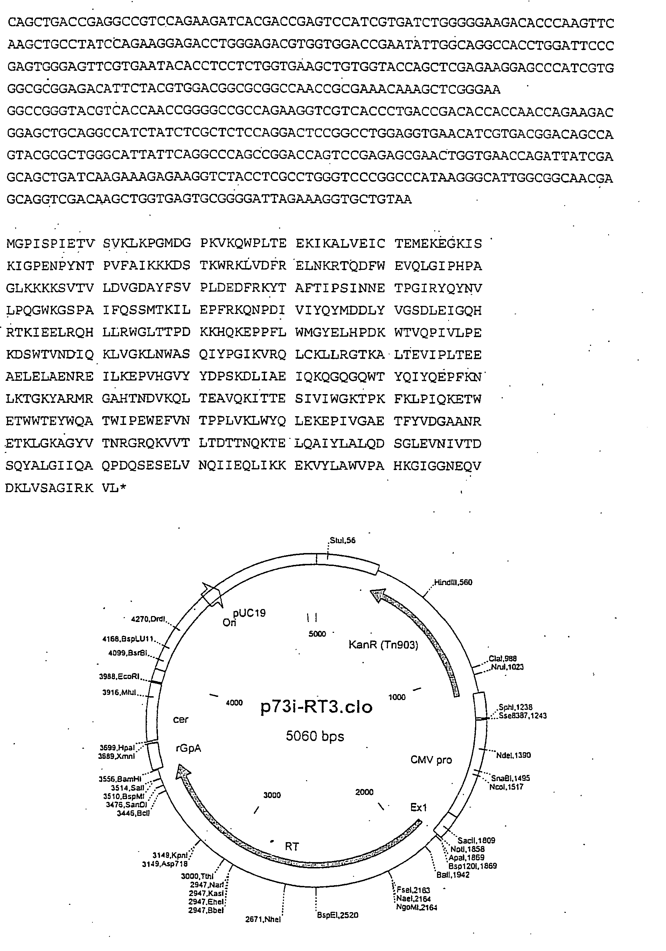

The synthetic gene coding for the RT portion of the pol gene of HIV-1 clade B strain HXB2, optimised for expression in mammalian cells was excised from plasmid p7077-RT3 as a 1697bp NotI / BamHI fragment, gel purified, and cloned into the NotI & BamHI sites of p7313-ie (derived from pspC31) to place the gene downstream of an Iowa length HCMV promoter + exon1, and upstream of a rabbit globin poly-adenylation signal. (R7004 p27) (figure 23)

### Example 13

### Preparation of plasmid-coated 'gold slurry' for 'gene gun' DNA cartridges

Plasmid DNA (approximately 1µg/µl), eg. 100 ug, and 2µm gold particles, eg. 50 mg, (PowderJect), were suspended in 0.05M spermidine, eg. 100 ul, (Sigma). The DNA was precipitated on to the gold particles by addition of 1M CaCl₂, eg. 100µl (American Pharmaceutical Partners, Inc., USA). The DNA/gold complex was incubated for 10 minutes at room temperature, washed 3 times in absolute ethanol, eg. 3 x 1 ml, (previously dried on molecular sieve 3A (BDH)). Samples were resuspended in absolute ethanol containing 0.05mg/ml of polyvinylpyrrolidone (PVP, Sigma), and split into three equal aliquots in 1.5 ml microfuge tubes, (Eppendorf).

The aliquots were for analysis of (a) 'gold slurry', (b) eluate- plasmid eluted from (a) and (c) for preparation of gold/ plasmid coated Tefzel cartridges for the 'gene gun', (see below). For preparation of samples (a) and (b), the tubes containing plasmid DNA / 'gold slurry' in ethanol / PVP were spun for 2 minutes at top speed in an Eppendorf 5418 microfuge, the supernatant was removed and the 'gold slurry' dried for 10 minutes at room temperature. Sample (a) was resuspended to 0.5 - 1.0 ug / ul of plasmid DNA in TE pH 8.0, assuming approx. 50 % coating. For elution, sample (b) was resuspended to 0.5 - 1.0 ug / ul of plasmid DNA in TE pH 8.0 and incubated at 37°C for 30 minutes, shaking vigorously, and then spun for 2 minutes at top speed in an Eppendorf 5418 microfuge and the supernatant, eluate, was removed and stored at -20°C. The exact DNA concentration eluted was determined by spectrophotometric quantitation using a Genequant II (Pharmacia Biotech).

### Example 14

### Preparations of Cartridges for DNA immunisation

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 mm gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with a total of 0.5 mg DNA/caitridge.

### Example 15

### Immune Response to HIV antigens following, DNA vaccination utilising the gene gun.

Mice (n=3/group) were vaccinated with antigens encoded by nucleic acid and located in two vectors. P7077 utilises the HCMV IE promoter including Intron A and exon 1 (fcmv promoter). P73I delivers the same antigen, but contains the HCMV IE promoter (icmv promoter) that is devoid of Intron A, but includes exon 1. Plasmid was delivered to the shaved target site of abdominal skin of F1 (C3H x Balb/c) mice. Mice were given a primary immunisation of 2 x 0.5 µg DNA on day 0, boosted with 2 x 0.5 (µg DNA on day 35 and cellular response were detected on day 40 using IFN - gamma Elispot.

| | | | | |
|---|---|---|---|---|
| . | P73I | | - | empty vector |
| . | P7077 | | - | empty vector |
| . | P7077 | GRN | - | (f CMV promoter) Gag, RT, Nef |
| . | P73I | GRN | - | (i CMV promoter) Gag, RT, Nef |
| . | P73I | GR3N | - | (CMV promoter) Optimised Gag, Optimised RT, Nef |
| . | P7077 | GN | - | (f CMV promoter)Gag, Nef |
| . | P73I | GN | - | (i CMV promoter) Gag, Nef |

### Cytotoxic T Cell Responses

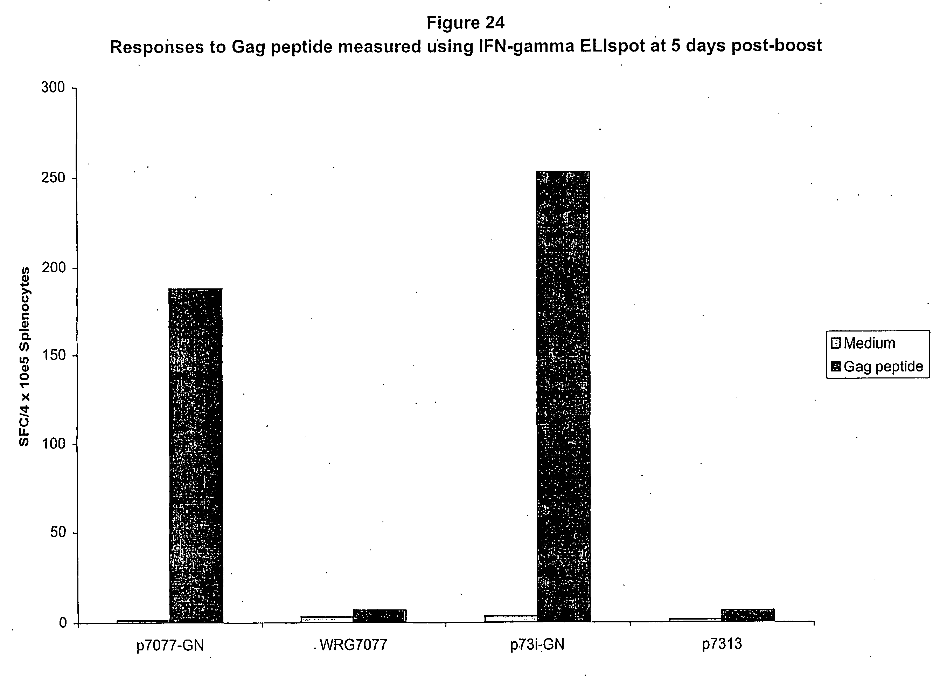

The cytotoxic T cell response was assessed by CD8+ T cell-restricted-IFN-g ELISPOT assay of splenocytes collected 5 days later. Mice were killed by cervical dislocation and spleens were collected into ice-cold PBS. Splenocytes were teased out into phosphate buffered saline (PBS) followed by lysis of red blood cells (1 minute in buffer consisting of 155mM NH₄Cl, 10 mM KHCO₃, 0.1mM EDTA). After two washes in PBS to remove particulate matter the single cell suspension was aliquoted into ELISPOT plates previously coated with capture IFN-g antibody and stimulated with CD8-restricted cognate peptide (Gag, Nef or RT). After overnight culture, IFN-g producing cells were visualised by application of anti-murine IFN-g-biotin labelled antibody (Pharmingen) followed by streptavidin -conjugated alkaline phosphatase and quantitated using image analysis. The result of this experiment are shown in Figures 24 to 26.

### Example 16

### Adjuvant activity of Resiquimod

Resiquimod is delivered topically to the abdominal skin of Balb/c mice on either day -2, -1, 0, 1, 2 or 3 relative to immunization on day 0. Immunization utilizes a DNA vaccine that expresses the Hepatitis B surface antigen so that cellular and antibody responses found after treatment with Resiquimod can be evaluated and compared to a wealth of historical data. The formulation consists of either a 0.05% cream, or a DMSO solution (approximately 5 mg/ml). Ideally both types of formula are tested. One half of the mice are sacrificed 7 days after immunization to measure cellular immune responses (CD8 ELISPOT). The remaining mice are bled 28 days after immunization, boosted with the same schedule as the priming immunization and then sacrificed 2 weeks later to test for antibody and cellular responses. Serum is used to test for antibody titer and to evaluation subclass distribution of IgG antibodies indicating the bias of the immune response.
At a time either before or after immunization the Resiquimod enhances immune responses to the DNA vaccines. The enhancement is likely occur when Resiquimod is delivered 1 day after immunization as found for Aldara, which is a similar compound. Most likely a boost in the cellular immune response is found. Resiquimod may have an ability to enhance antibody responses but this may not occur with the delivery site and schedule of administration that we employ. In theory, one time point (different than the enhancing point) may also find a suppressing effect of the Resiquimod, which may be exploited for other applications. The doses used appear to be in the range where activity of Resiquimod is found *in vivo*, and further refinement of the method will optimize dosing and formulation of the Resiquimod.

### Materials and Methods

### Plasmids

For immunization DNA vaccine plasmids with a well-known immune response are employed. Plasmid WRG7128 is a likely choice which expresses the Hepatitis B surface antigen (HBsAg) using the HCMV promoter/enhancer and generates a cellular and antibody response.

### Topical Adjuvants

Resiquimod is formulated as either a 0.05% cream, or a 5 mg/ml solution in DMSO. To apply the cream, it is rubbed onto the abdomens of mice using a cotton swab. Approximately 20 ml of cream is given by this method. The DMSO solution is spread over the abdomen of the mice using a pipettor that delivers 50ug

### DNA vaccines

Precipitation of DNA onto gold particles is achieved using standard procedures for the calcium/spermidine formulation of DNA vaccines. DNA is mixed with 2 micron gold particles in a small centrifuge tube containing 300 ml of 50 mM spermidine. The amount of DNA added is 2 ug per mg gold particles and typically batches of 26 mg gold (5-2 ug of DNA) were made. The DNA is precipitated onto gold by the addition of a 1/10 volume of 10% CaCl₂ during continuous agitation of the tube on a rotary mixer. DNA-gold complexes are washed three times with absolute ethanol then loaded into Tefzel tubing, dried and cut into 0.5 inch segments for use in the XR-1 device.

For immunization, DNA vaccines are delivered by the XR-1 device into the abdomen of Balb/C mice. A single shot is given for the immunization. Some experiments will employ a prime only, and others will have a prime and a boost at 4 weeks.

### Antibody ELISA

Serum samples are assayed for antibodies using an ELISA assay. Falcon Pro Bind microtiter plates are coated overnight at 4 °C with antigen in PBS (phosphate buffered saline, BioWhittaker). For HBsAg ELISA the antigen is purified HBsAg (BioDesign) at 0.1 ug per well. The plates are blocked for 1 hour at RT with 5% dry milk/PBS then washed 3X with wash buffer (10 mM Tris Buffered saline, 0.1% Brij-35) and serum samples diluted in dilution buffer (2% dry milk/PBS/0.05 % Tween 20) are added to the plate and then incubated for 2 hours at RT. Plates are washed 3X and a biotinylated goat anti-mouse antibody (Southern Biotechnology) diluted 1: 8000 in dilution buffer is added to the plate and incubated for 1 hr at RT.

Following the incubation, plates are washed 3X, then a Streptavidin-Horseradish peroxidase conjugate (Southern Biotechnology) diluted 1:8000 in PBS is added and the plate incubated a further 1 hr at RT. Plates are washed 3X, then substrate solution added (BioRad) and the reaction is stopped with IN H₂SO₄. Optical density is read at 450 nm. Endpoint titers for HBsAg are calculated by comparison of the samples with a standard of known titer. For subclass evaluation the biotinylated anti-mouse antibodies used are either IgG1 or IgG2a specific but the assay remains the same.

### Cell Culture

Single cell suspensions are obtained from mouse spleens. Spleens are squeezed through a mesh to produce a single cell suspension and cells are then sedimented, and treated with ACK buffer (Bio Whittaker, Walkersville MD) to lyse red blood cells. The cells are then washed twice in RPMI 1640 media supplemented with HEPES, 1 % glutamine (Bio Whittaker), and 5% heat inactivated fetal calf serum (FCS, Harlan, Indianapolis IN). Cells are counted, and resuspended to an appropriate concentration in "Total" media consisting of RPMI 1640 with HEPES and 1% glutamine, supplemented with 5% heat inactivated FCS, 50 mM mercaptoethanol (Gibco-BRL, Long Island NY), gentamycin (Gibco-BRL), 1 mM MEM sodium pyruvate (Gibco-BRL) and MEM non-essential amino acids (Sigma, St. Louis MO). For the CD8 specific assays cells are cultured *in vitro* in the presence of a peptide corresponding to a known CD8 epitopes. For HBsAg in BALB/C mice the sequence of the peptide is IPQSLDSWWTSL (QCB Inc). Peptides are made up in DMSO (10 mg/ml) and diluted to 10 mg/ml in culture medium.

### ELISPOTs

For IFN-g ELISPOTs assays Millipore Multiscreen membrane filtration plates are coated with 50 ul of 15 ug/ml anti-IFN-g antiserum (Pharmingen) in sterile 0.1M carbonate buffer pH 9.6, overnight at 4°C. Plates are washed 6X with sterile PBS and then blocked with tissue culture medium containing 10% fetal bovine serum (FBS) for 1-2 hr at RT. The medium is removed and spleen cells dispensed into the wells with a total of 1X10⁶ cells per well. For wells in which less than 1X10⁶ cells from immunized animals is added, cells from naïve animals are used to bring the total to 1X10⁶. Cells are incubated overnight in a tissue culture incubator in the presence of the peptide as described above. Plates are washed 2X with PBS and 1X with distilled water. This is followed with 3 washes with PBS. Biotinylated anti IFN-g monoclonal antibody (Pharmingen) is added to the plate (50 ul of a 1 ug/ml solution in PBS) and incubated for 2 hr at RT. Plates are washed 6X with PBS then 50 ul of a Streptavidin Alkaline phosphatase conjugate (1:1000 in PBS, Pharmingen) is added and incubated for 2 hr at RT. Plates are washed 6X with PBS and the color substrate (BioRad) is added and the reaction is allowed to proceed until dark spots appear. The reaction is stopped by washing with water 3X. Plates are air dried and spots counted under a microscope.

## Claims

1. Use of compound which is an imidazoquinoline amine, imidazopyridine amine, 6,7-fused cycloalkylimidazopyridine amine, 1,2-bridged imidazoquinoline amine, thiazolo- and oxazolo-quinolinamine or pyridinamine, imidazonaphthyridine or tetrahydroimidazonaphthyridine amine in the manufacture of a medicament to enhance an immune response to an antigen, wherein the compound is administered topically or transdermally to the individual 12 to 36 hours after a nucleic acid vaccine.is administered, and wherein the nucleic acid vaccine comprises a nucleotide sequence that encodes an HIV-1 gag protein or fragment containing a gag epitope thereof and a second HIV antigen or a fragment encoding an epitope of said second HIV antigen, operably linked to a heterologous promoter.

2. Use of a nucleotide sequence that encodes an HIV-1 gag protein or fragment containing a gag epitope thereof and a second HIV antigen or a fragment encoding an epitope of said second HIV antigen, operably linked to a heterologous promoter in the manufacture of a nucleic acid vaccine, wherein 12 to 36 hours subsequent to the administration of the nucleic acid vaccine to an individual a compound which is an imidazoquinoline amine, imidazopyridine amine, 6,7-fused cycloalkylimidazopyridine amine, 1,2-bridged imidazoquinoline amine, thiazolo- and oxazolo-quinolinamine or pyridinamine, imidazonaphthyridine or tetr-ahydroimidazonaphthyridine amine is administered topically or transdermally to the individual.

3. Use according to claim 1 or 2 wherein the compound is an imidazoquinoline.

4. Use according to claim 1 or 2 wherein the compound is imiquimod.

5. Use according to any one of the preceding claims wherein the nucleic acid vaccine is administered topically or transdermally.

6. Use according to any one of the preceding claims wherein the nucleic acid vaccine is administered in the form of particles.

7. Use according to any one of the preceding claims wherein the compound is administered in the form of particles.

8. Use according to claim 6 or 7 wherein the nucleic acid vaccine or compound is coated on a core carrier.

9. Use according to any one of claims 6 to 8 wherein the nucleic acid vaccine or compound is administered using a needless syringe.

10. Use according to any one of the preceding claims in which the compound is administered in the form of a cream

11. Use according to any one of the preceding claims wherein the administration of the antigen or polynucleotide is repeated to provide a prime and booster administration.

12. Use according to any one of the preceding claims wherein the second antigen is selected from the group consisting of: Nef, RT or a fragment containing an epitope of Nef or RT.

13. Use according to any one of the preceding claims wherein the gag protein comprises p17.

14. Use according to claim 13 wherein the gag protein additionally comprises p24.

15. Use according to any one of the preceding claims wherein the gag sequence is codon optimised to resemble the codon usage in a highly expressed human gene.

16. Use according to any one claims 12 to 15 wherein the RT sequence or fragment thereof is codon optimised to resemble a highly expressed human gene.

17. Use according to any one of the preceding claims wherein the nucleotide sequence encodes a Nef protein or epitope thereof.

18. Use according to any one of the preceding claims wherein the nucleotide sequence is selected from the group
- Gag (p17,p24) Nef truncate
- Gag (p17,p24) (codon optimised) Nef (truncate)
- Gag (p17,p24) RT Nef (truncate)
- Gag (p17,p24) codon optimised RT Nef (truncate)
- Gag (p17,p24) codon optimised RT codon optimised Nef truncate

19. Use according to any one of the preceding claims wherein the heterologous promoter is the minimal promoter from HCMV IE gene.

20. Use according to claim 19 wherein the 5' of the promoter comprises exon 1.

21. Use according to any one of the preceding claims wherein the nucleic acid sequence is in the form of a double stranded DNA plasmid.

22. Use according to any one of the preceding claims wherein the nucleic acid sequence encodes Gag (or a fragment thereof which comprises an epitope) and RT (or a fragment thereof which comprises an epitope) and Nef (or a fragment thereof which comprises an epitope) in any order.

23. Use according to claim 22 wherein wherein the nucleic acid encodes the proteins, or fragments thereof, in the sequence Nef-RT-Gag, RT-Nef-Gag or RT-Gag-Nef.

24. Use according to any one of the preceding claims wherein at least one of the proteins which is encoded by the nucleic acid is a fusion protein.

25. A product containing (i) a nucleic acid vaccine that comprises a nucleotide sequence that encodes an HIV-1 gag protein or fragment containing a gag epitope thereof and a second HIV antigen or a fragment encoding an epitope of said second HIV antigen, operably linked to a heterologous promoter, and (ii) a compound which is an imidazoquinoline amine, imidazopyridine amine, 6,7-fused cycloalkylimidazopyrdine amine, 1,2-bridged imidazoquinoline amine, thiazolo- and oxazolo-quinolinamine or pyridinamine, imidazonaphthyridine or tetrahydroimidazonaphthyridine amine for sequential use, wherein the compound is administered topically or transdermally 12 to 36 hours after administration of the nucleic acid vaccine.

## Patentansprüche

1. Verwendung einer Verbindung, die ein Imidazochinolinamin, Imidazopyridinamin, 6,7-kondensiertes Cycloalkylimidazopyridinamin, 1,2-verbrücktes Imidazochinolinamin, Thiazolo- und Oxazolochinolinamin oder -pyridinamin, Imidazonaphthyridin- oder Tetrahydroimidazonaphthyridinamin ist, bei der Herstellung eines Medikaments zur Verstärkung einer Immunantwort auf ein Antigen, wobei die Verbindung 12 bis 36 Stunden nach Verabreichung eines Nucleinsäure-Impfstoffs topisch oder transdermal an das Individuum verabreicht wird und wobei der Nucleinsäure-Impfstoff eine Nucleotidsequenz, die ein HIV-1 gag-Protein oder Fragment, das ein gag-Epitop davon enthält, kodiert, und ein zweites HIV-Antigen oder ein Fragment, das ein Epitop des zweiten HIV-Antigens kodiert, das operabel mit einem heterologen Promotor verknüpft ist, enthält.

2. Verwendung einer Nucleotidsequenz, die ein HIV-1 gag-Protein oder Fragment, das ein gag Epitop davon enthält, kodiert, und eines zweiten HIV-Antigens oder eines Fragments, das ein Epitop des zweiten HIV-Antigens kodiert, das operabel mit einem heterologen Promotor verknüpft ist, bei der Herstellung eines Nucleinsäure-Impfstoffs, wobei 12 bis 36 Stunden nach der Verabreichung des Nucleinsäure-Impfstoffs an ein Individuum eine Verbindung, die ein Imidazochinolinamin, Imidazopyridinamin, 6,7-kondensiertes Cycloalkylimidazopyridinamin, 1,2-verbrücktes Imidazochinolinamin, Thiazolo- und Oxazolochinolinamin oder -pyridinamin, Imidazonaphthyridin- oder Tetrahydroimidazonaphthyridinamin ist, topisch oder transdermal an das Individuum verabreicht wird.

3. Verwendung nach Anspruch 1 oder 2, wobei die Verbindung ein Imidazochinolin ist.

4. Verwendung nach Anspruch 1 oder 2, wobei die Verbindung Imiquimod ist.

5. Verwendung nach einem der vorhergehenden Ansprüche, wobei der Nucleinsäure-Impfstoff topisch oder transdermal verabreicht wird.

6. Verwendung nach einem der vorhergehenden Ansprüche, wobei der Nucleinsäure-Impfstoff in der Form von Partikeln verabreicht wird.

7. Verwendung nach einem der vorhergehenden Ansprüche, wobei die Verbindung in der Form von Partikeln verabreicht wird.

8. Verwendung nach Anspruch 6 oder 7, wobei der Nucleinsäure-Impfstoff oder die Verbindung auf einen Träger-Kern aufgebracht ist.

9. Verwendung nach einem der Ansprüche 6 bis 8, wobei der Nucleinsäure-Impfstoff oder die Verbindung unter Verwendung einer nadellosen Spritze verabreicht wird.

10. Verwendung nach einem der vorhergehenden Ansprüche, wobei die Verbindung in der Form einer Creme verabreicht wird.

11. Verwendung nach einem der vorhergehenden Ansprüche, wobei die Verabreichung des Antigens oder Polynucleotids wiederholt wird, um eine Prime-und-Booster-Verabreichung bereitzustellen.

12. Verwendung nach einem der vorhergehenden Ansprüche, wobei das zweite Antigen aus der Gruppe ausgewählt ist, die besteht aus: Nef, RT oder einem Fragment, das ein Epitop von Nef oder RT enthält.

13. Verwendung nach einem der vorhergehenden Ansprüche, wobei das gag-Protein p17 umfasst.

14. Verwendung nach Anspruch 13, wobei das gag-Protein zusätzlich p24 umfasst.

15. Verwendung nach einem der vorhergehenden Ansprüche, wobei die gag-Sequenz Codon-optimiert ist, um dem Codon-Gebrauch in einem hoch exprimierten menschlichen Gen zu gleichen.

16. Verwendung nach einem der Ansprüche 12 bis 15, wobei die RT-Sequenz oder ein Fragment davon Codon-optimiert ist, um einem hoch exprimierten menschlichen Gen zu gleichen.

17. Verwendung nach einem der vorhergehenden Ansprüche, wobei die Nucleotid-Sequenz ein Nef-Protein oder Epitop davon codiert.

18. Verwendung nach einem der vorhergehenden Ansprüche, wobei die Nucleotid-Sequenz aus der folgenden Gruppe ausgewählt ist:
- Gag (p17, p24) Nef-Trunkat
- Gag (p17, p24) (Codon-optimiert) Nef (Trunkat)
- Gag (p17, p24) Nef (Trunkat)
- Gag (p17, p24) (Codon-optimiert) RT Nef (Trunkat)
- Gag (p17, p24) (Codon-optimiert) RT (Codon-optimiert) Nef-Trunkat

19. Verwendung nach einem der vorhergehenden Ansprüche, wobei der heterologe Promotor der Minimalpromotor aus dem HCMV IE-Gen ist.

20. Verwendung nach Anspruch 19, wobei das 5' des Promotors das Exon 1 umfasst.

21. Verwendung nach einem der vorhergehenden Ansprüche, wobei die Nucleinsäuresequenz in der Form eines doppelsträngigen DNA-Plasmids vorliegt.

22. Verwendung nach einem der vorhergehenden Ansprüche, wobei die Nucleinsäuresequenz Gag (oder ein Fragment davon, das ein Epitop einschließt) und RT (oder ein Fragment davon, das ein Epitop einschließt) und Nef (oder ein Fragment davon, das ein Epitop einschließt) in beliebiger Reihenfolge codiert.

23. Verwendung nach Anspruch 22, wobei die Nucleinsäure die Proteine, oder Fragmente davon, in der Sequenz Nef-RT-Gag, RT-Nef-Gag oder RT-Gag-Nef codiert.

24. Verwendung nach einem der vorhergehenden Ansprüche, wobei mindestens eines der Proteine, das von der Nucleinsäure codiert wird, ein Fusionsprotein ist.

25. Ein Produkt, das folgendes enthält (i) einen Nucleinsäure-Impfstoff, der eine Nucleotidsequenz, die ein HIV-1 gag Protein oder Fragment, das ein gag Epitop davon enthält, kodiert, und ein zweites HIV-Antigen oder ein Fragment, das ein Epitop des zweiten HIV-Antigens kodiert, das operabel mit einem heterologen Promotor verknüpft ist, enthält, und (ii) eine Verbindung, die ein Imidazochinolinamin, Imidazopyridinamin, 6,7-kondensiertes Cycloalkylimidazopyridinamin, 1,2-verbrücktes Imidazochinolinamin, Thiazolo- und Oxazolochinolinamin oder -pyridinamin, Imidazonaphthyridin- oder Tetrahydroimidazonaphthyridinamin ist, zur sequenziellen Verwendung, wobei die Verbindung 12 bis 36 Stunden nach Verabreichung des Nucleinsäure-Impfstoffs topisch oder transdermal verabreicht wird.

## Revendications

1. Utilisation d'un composé qui est une imidazoquinoline amine, une imidazopyridine amine, une cycloalkylimidazopyridine amine condensée en 6,7, une imidazoquinoline amine à pont 1,2, une thiazolo- et oxazolo-quinolinamine ou pyridinamine, une imidazonaphtyridine ou tétrahydro-imidazonaphtyridine amine, dans la fabrication d'un médicament destiné à augmenter une réponse immunitaire à un antigène, dans laquelle le composé est administré par voie topique ou transdermique à l'individu 12 à 36 heures après administration d'un vaccin à base d'acide nucléique, et dans laquelle le vaccin à base d'acide nucléique comprend une séquence nucléotidique qui code une protéine gag de VIH-1 ou un fragment de celle-ci contenant un épitope de gag et un second antigène de VIH ou un fragment codant un épitope dudit second antigène de VIH, liée de façon opérationnelle à un promoteur hétérologue.

2. Utilisation d'une séquence nucléotidique qui code une protéine gag de VIH-1 ou un fragment de celle-ci contenant un épitope de gag et un second antigène de VIH ou un fragment codant un épitope dudit second antigène de VIH, liée de façon opérationnelle à un promoteur hétérologue, dans la fabrication d'un vaccin à base d'acide nucléique, dans laquelle, 12 à 36 heures après l'administration du vaccin à base d'acide nucléique à un individu, un composé, qui est une imidazoquinoline amine, une imidazopyridine amine, une cycloalkylimidazopyridine amine condensée en 6,7, une imidazoquinoline amine à pont 1,2, une thiazolo- et oxazolo-quinolinamine ou pyridinamine, une imidazonaphtyridine ou tétrahydro-imidazonaphtyridine amine, est administré par voie topique ou transdermique à l'individu.

3. Utilisation selon la revendication 1 ou 2, dans laquelle le composé est une imidazoquinoline.

4. Utilisation selon la revendication 1 ou 2, dans laquelle le composé est l'imiquimod.

5. Utilisation selon l'une quelconque des revendications précédentes, dans laquelle le vaccin à base d'acide nucléique est administré par voie topique ou transdermique.

6. Utilisation selon l'une quelconque des revendications précédentes, dans laquelle le vaccin à base d'acide nucléique est administré sous forme de particules.

7. Utilisation selon l'une quelconque des revendications précédentes, dans laquelle le composé est administré sous forme de particules.

8. Utilisation selon la revendication 6 ou 7, dans laquelle le vaccin à base d'acide nucléique ou le composé est déposé sur un coeur support.

9. Utilisation selon l'une quelconque des revendications 6 à 8, dans laquelle le vaccin à base d'acide nucléique ou le composé est administré à l'aide d'une seringue sans aiguille.

10. Utilisation selon l'une quelconque des revendications précédentes, dans laquelle le composé est administré sous forme d'une crème.

11. Utilisation selon l'une quelconque des revendications précédentes, dans laquelle l'administration de l'antigène ou du polynucléotide est répétée pour fournir une sensibilisation et une administration de rappel.

12. Utilisation selon l'une quelconque des revendications précédentes, dans laquelle le second antigène est choisi dans l'ensemble consistant en : Nef, RT ou un fragment contenant un épitope de Nef ou RT.

13. Utilisation selon l'une quelconque des revendications précédentes, dans laquelle la protéine gag comprend p17.

14. Utilisation selon la revendication 13, dans laquelle a protéine gag comprend en outre p24.

15. Utilisation selon l'une quelconque des revendications précédentes, dans laquelle la séquence de gag est optimisée en termes de codons pour imiter l'usage des codons dans un gène humain à expression élevée.

16. Utilisation selon l'une quelconque des revendications 12 à 15, dans laquelle la séquence RT ou l'un de ses fragments est optimisée en termes de codons pour ressembler à un gène humain à expression élevée.

17. Utilisation selon l'une quelconque des revendications précédentes, dans laquelle la séquence nucléotidique code une protéine Nef ou un épitope de celle-ci.

18. Utilisation selon l'une quelconque des revendications précédentes, dans laquelle la séquence nucléotidique est choisie dans l'ensemble
- Gag (p17,p24) Nef troncature
- Gag (p17,p24) (codons optimisés) Nef (troncature)
- Gag (p17,p24) RT Nef (troncature)
- Gag (p17,p24) codons optimisés RT Nef (troncature)
- Gag (p17,p24) codons optimisés RT codons optimisés Nef troncature

19. Utilisation selon l'une quelconque des revendications précédentes, dans laquelle le promoteur hétérologue est le promoteur minimal du gène IE de HCMV

20. Utilisation selon la revendication 19, dans laquelle la partie 5' du promoteur comprend l'exon 1.

21. Utilisation selon l'une quelconque des revendications précédentes, dans laquelle la séquence d'acide nucléique est sous la forme d'un plasmide d'ADN bicaténaire.

22. Utilisation selon l'une quelconque des revendications précédentes, dans laquelle la séquence d'acide nucléique code Gag (ou l'un de ses fragments qui comprend un épitope) et RT (ou l'un de ses fragments qui comprend un épitope) et Nef (ou l'un de ses fragments qui comprend un épitope) dans un ordre quelconque.

23. Utilisation selon la revendication 22, dans laquelle l'acide nucléique code les protéines, ou leurs fragments, selon la séquence Nef-RT-Gag, RT-Nef-Gag ou RT-Gag-Nef.

24. Utilisation selon l'une quelconque des revendications précédentes, dans laquelle au moins une des protéines qui est codée par l'acide nucléique est une protéine de fusion.

25. Produit contenant (i) un vaccin à base d'acide nucléique qui comprend une séquence nucléotidique qui code une protéine gag de VIH-1 ou un fragment de celle-ci contenant un épitope de gag et un second antigène de VIH ou un fragment codant un épitope dudit second antigène de VIH, liée de façon opérationnelle à un promoteur hétérologue, et (ii) un composé qui est une imidazoquinoline amine, une imidazopyridine amine, une cycloalkylimidazopyridine amine condensée en 6,7, une imidazoquinoline amine à pont 1,2, une thiazolo- et oxazolo-quinolinamine ou pyridinamine, une imidazonaphtyridine ou tétrahydro-imidazonaphtyridine amine pour une utilisation séquentielle, le composé étant administré par voie topique ou transdermique 12 à 36 heures après administration du vaccin à base d'acide nucléique.


### SEQUENCE LISTING

<110> Braun, Ralph P.
   Thomsen, Lindy
   Van-Wely, Catherine
   Ertl, Peter
<120> Adjuvant
<130> 03-3267-015
<140> US 10/102,622
   <141> 2002-03-19
<160> 12
<170> FastSEQ for Windows Version 4.0
<210> 1
   <211> 12
   <212> PRT
   <213> Artificial Sequence
<220>
   <223> HBsAg in BALB/C mice
<400> 1
   Ile Pro Gln Ser Leu Asp Ser Trp Trp Thr Ser Leu
    1               5                   10
<210> 2
   <211> 9
   <212> PRT
   <213> Artificial Sequence
<220>
   <223> HSV CD8 in BALB/C mice
<400> 2
   His Gly Pro Ser Leu Tyr Arg Thr Phe
    1               5
<210> 3
   <211> 1503
   <212> DNA
   <213> Artificial Sequence
<220>
   <223> nucletoide sequence of the p55 gag insert in pGagOptrpr2
<400> 3
   atgggtgccc gagcttcggt actgtctggt ggagagctgg acagatggga gaaaattagg 60
   ctgcgcccgg gaggcaaaaa gaaatacaag ctcaagcata tcgtgtgggc ctcgagggag 120
   cttgaacggt ttgccgtgaa cccaggcctg ctggaaacat ctgagggatg tcgccagatc 180
   ctggggcaat tgcagccatc cctccagacc gggagtgaag agctgaggtc cttgtataac 240
   acagtggcta ccctctactg cgtacaccag aggatcgaga ttaaggatac caaggaggcc 300
   ttggacaaaa ttgaggagga gcaaaacaag agcaagaaga aggcccagca ggcagctgct 360
   gacactgggc atagcaacca ggtatcacag aactatccta ttgtccaaaa cattcagggc 420
   cagatggttc atcaggccat cagcccccgg acgctcaatg cctgggtgaa ggttgtcgaa 480
   gagaaggcct tttctcctga ggttatcccc atgttctccg ctttgagtga gggggccact 540
   cctcaggacc tcaatacaat gcttaatacc gtgggcggcc atcaggccgc catgcaaatg 600
   ttgaaggaga ctatcaacga ggaggcagcc gagtgggaca gagtgcatcc cgtccacgct 660
   ggcccaatcg cgcccggaca gatgcgggag cctcgcggct ctgacattgc cggcaccacc 720
   tctacactgc aagagcaaat cggatggatg accaacaatc ctcccatccc agttggagaa 780
   atctataaac ggtggatcat tctcggtctc aataaaattg ttagaatgta ctctccgaca 840
   tccatccttg acattagaca gggacccaaa gagcctttta gggattacgt cgaccggttt 900
   tataagaccc tgcgagcaga gcaggcctct caggaggtca aaaactggat gacggagaca 960
   ctcctggtac agaacgctaa ccccgactgc aaaacaatct tgaaggcact aggcccggct 1020
   gccaccctgg aagagatgat gaccgcctgt cagggagtag gcggacccgg acacaaagcc 1080
   agagtgttgg ccgaagccat gagccaggtg acgaactccg caaccatcat gatgcagaga 1140
   gggaacttcc gcaatcagcg gaagatcgtg aagtgtttca attgcggcaa ggagggtcat 1200
   accgcccgca actgtcgggc ccctaggaag aaagggtgtt ggaagtgcgg caaggaggga 1260
   caccagatga aagactgtac agaacgacag gccaattttc ttggaaagat ttggccgagc 1320
   tacaagggga gacctggtaa tttcctgcaa agcaggcccg agcccaccgc cccccctgag 1380
   gaatccttca ggtccggagt ggagaccaca acgcctcccc aaaaacagga accaatcgac 1440
   aaggagctgt accctttaac ttctctgcgt tctctctttg gcaacgaccc gtcgtctcaa 1500
   taa                                                               1503
<210> 4
   <211> 500
   <212> PRT
   <213> Artificial Sequence
<220>
   <223> amino acid sequence of p55 gag insert in pGagOptrpr2
<400> 4
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Val Leu Ala Glu Ala Met Ser
           355                 360                 365
   Gln Val Thr Asn Ser Ala Thr Ile Met Met Gln Arg Gly Asn Phe Arg
       370                 375                 380
   Asn Gln Arg Lys Ile Val Lys Cys Phe Asn Cys Gly Lys Glu Gly His
   385                 390                 395                 400
   Thr Ala Arg Asn Cys Arg Ala Pro Arg Lys Lys Gly Cys Trp Lys Cys
                   405                 410                 415
   Gly Lys Glu Gly His Gln Met Lys Asp Cys Thr Glu Arg Gln Ala Asn
               420                 425                 430
   Phe Leu Gly Lys Ile Trp Pro Ser Tyr Lys Gly Arg Pro Gly Asn Phe
           435                 440                 445
   Leu Gln Ser Arg Pro Glu Pro Thr Ala Pro Pro Glu Glu Ser Phe Arg
       450                 455                 460
   Ser Gly Val Glu Thr Thr Thr Pro Pro Gln Lys Gln Glu Pro Ile Asp
   465                 470                 475                 480
   Lys Glu Leu Tyr Pro Leu Thr Ser Leu Arg Ser Leu Phe Gly Asn Asp
                   485                 490                 495
   Pro Ser Ser Gln
               500
<210> 5
   <211> 1515
   <212> DNA
   <213> Artificial Sequence
<220>
   <223> nucleotide sequence of the p17/24trNEF insert.in p17/24trNEF1
<400> 5
   atgggtgcga gagcgtcagt attaagcggg ggagaattag atcgatggga aaaaattcgg 60
   ttaaggccag ggggaaagaa aaaatataaa ttaaaacata tagtatgggc aagcagggag 120
   ctagaacgat tcgcagttaa tcctggcctg ttagaaacat cagaaggctg tagacaaata 180
   ctgggacagc tacaaccatc ccttcagaca ggatcagaag aacttagatc attatataat 240
   acagtagcaa ccctctattg tgtgcatcaa aggatagaga taaaagacac caaggaagct 300
   ttagacaaga tagaggaaga gcaaaacaaa agtaagaaaa aagcacagca agcagcagct 360
   gacacaggac acagcaatca ggtcagccaa aattacccta tagtgcagaa catccagggg 420
   caaatggtac atcaggccat atcacctaga actttaaatg catgggtaaa agtagtagaa 480
   gagaaggctt tcagcccaga agtgataccc atgttttcag cattatcaga aggagccacc 540
   ccacaagatt taaacaccat gctaaacaca gtggggggac atcaagcagc catgcaaatg 600
   ttaaaagaga ccatcaatga ggaagctgca gaatgggata gagtgcatcc agtgcatgca 660
   gggcctattg caccaggcca gatgagagaa ccaaggggaa gtgacatagc aggaactact 720
   agtacccttc aggaacaaat aggatggatg acaaataatc cacctatccc agtaggagaa 780
   atttataaaa gatggataat cctgggatta aataaaatag taagaatgta tagccctacc 840
   agcattctgg acataagaca aggaccaaaa gaacccttta gagactatgt agaccggttc 900
   tataaaactc taagagccga gcaagcttca caggaggtaa aaaattggat gacagaaacc 960
   ttgttggtcc aaaatgcgaa cccagattgt aagactattt taaaagcatt gggaccagcg 1020
   gctacactag aagaaatgat gacagcatgt cagggagtag gaggacccgg ccataaggca 1080
   agagttttgg tgggttttcc agtcacacct caggtacctt taagaccaat gacttacaag 1140
   gcagctgtag atcttagcca ctttttaaaa gaaaaggggg gactggaagg gctaattcac 1200
   tcccaaagaa gacaagatat ccttgatctg tggatctacc acacacaagg ctacttccct 1260
   gattggcaga actacacacc agggccaggg gtcagatatc cactgacctt tggatggtgc 1320
   tacaagctag taccagttga gccagataag gtagaagagg ccaataaagg agagaacacc 1380
   agcttgttac accctgtgag cctgcatggg atggatgacc cggagagaga agtgttagag 1440
   tggaggtttg acagccacct agcatttcat cacgtggccc gagagctgca tccggagtac 1500
   ttcaagaact gctga                                                  1515
<210> 6
   <211> 504
   <212> PRT
   <213> Artificial Sequence
<220>
   <223> amino acid sequence of the p17/24trNEF insert in p17/24trNEF1
<400> 6
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                           245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Val Leu Val Gly Phe Pro Val
           355                 360                 365
   Thr Pro Gln Val Pro Leu Arg Pro Met Thr Tyr Lys Ala Ala Val Asp
       370                 375                 380
   Leu Ser His Phe Leu Lys Glu Lys Gly Gly Leu Glu Gly Leu Ile His
   385                 390                 395                 400
   Ser Gln Arg Arg Gln Asp Ile Leu Asp Leu Trp Ile Tyr His Thr Gln
                   405                 410                 415
   Gly Tyr Phe Pro Asp Trp Gln Asn Tyr Thr Pro Gly Pro Gly Val Arg
               420                 425                 430
   Tyr Pro Leu Thr Phe Gly Trp Cys Tyr Lys Leu Val Pro Val Glu Pro
           435                 440                 445
   Asp Lys Val Glu Glu Ala Asn Lys Gly Glu Asn Thr Ser Leu Leu His
       450                 455                 460
   Pro Val Ser Leu His Gly Met Asp Asp Pro Glu Arg Glu Val Leu Glu
   465                 470                 475                 480
   Trp Arg Phe Asp Ser His Leu Ala Phe His His Val Ala Arg Glu Leu
                   485                 490                 495
   His Pro Glu Tyr Phe Lys Asn Cys
               500
<210> 7
   <211> 1518
   <212> DNA
   <213> Artificial Sequence
<220>
   <223> nucleotide sequence of the p17/24opt/trNef insert in p17/24opt/trNef1
<400> 7
   atgggtgccc gagcttcggt actgtctggt ggagagctgg acagatggga gaaaattagg 60
   ctgcgcccgg gaggcaaaaa gaaatacaag ctcaagcata tcgtgtgggc ctcgagggag 120
   cttgaacggt ttgccgtgaa cccaggcctg ctggaaacat ctgagggatg tcgccagatc 180
   ctggggcaat tgcagccatc cctccagacc gggagtgaag agctgaggtc cttgtataac 240
   acagtggcta ccctctactg cgtacaccag aggatcgaga ttaaggatac caaggaggcc 300
   ttggacaaaa ttgaggagga gcaaaacaag agcaagaaga aggcccagca ggcagctgct 360
   gacactgggc atagcaacca ggtatcacag aactatccta ttgtccaaaa cattcagggc 420
   cagatggttc atcaggccat cagcccccgg acgctcaatg cctgggtgaa ggttgtcgaa 480
   gagaaggcct tttctcctga ggttatcccc atgttctccg ctttgagtga gggggccact 540
   cctcaggacc tcaatacaat gcttaatacc gtgggcggcc atcaggccgc catgcaaatg 600
   ttgaaggaga ctatcaacga ggaggcagcc gagtgggaca gagtgcatcc cgtccacgct 660
   ggcccaatcg cgcccggaca gatgcgggag cctcgcggct ctgacattgc cggcaccacc 720
   tctacactgc aagagcaaat cggatggatg accaacaatc ctcccatccc agttggagaa 780
   atctataaac ggtggatcat tctcggtctc aataaaattg ttagaatgta ctctccgaca 840
   tccatccttg acattagaca gggacccaaa gagcctttta gggattacgt cgaccggttt 900
   tataagaccc tgcgagcaga gcaggcctct caggaggtca aaaactggat gacggagaca 960
   ctcctggtac agaacgctaa ccccgactgc aaaacaatct tgaaggcact aggcccggct 1020
   gccaccctgg aagagatgat gaccgcctgt cagggagtag gcggacccgg acacaaagcc 1080
   agagtgttga tggtgggttt tccagtcaca cctcaggtac ctttaagacc aatgacttac 1140
   aaggcagctg tagatcttag ccacttttta aaagaaaagg ggggactgga agggctaatt 1200
   cactcccaaa gaagacaaga tatccttgat ctgtggatct accacacaca aggctacttc 1260
   cctgattggc agaactacac accagggcca ggggtcagat atccactgac ctttggatgg 1320
   tgctacaagc tagtaccagt tgagccagat aaggtagaag aggccaataa aggagagaac 1380
   accagcttgt tacaccctgt gagcctgcat gggatggatg acccggagag agaagtgtta 1440
   gagtggaggt ttgacagcca cctagcattt catcacgtgg cccgagagct gcatccggag 1500
   tacttcaaga actgctga                                               1518
<210> 8
   <211> 505
   <212> PRT
   <213> Artificial Sequence
<220>
   <223> amino acid sequence of the p17/24opt/trNef insert in p17/24opt/trNefl
<400> 8
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Val Leu Met Val Gly Phe Pro
           355                 360                 365
   Val Thr Pro Gln Val Pro Leu Arg Pro Met Thr Tyr Lys Ala Ala Val
       370                 375                 380
   Asp Leu Ser His Phe Leu Lys Glu Lys Gly Gly Leu Glu Gly Leu Ile
   385                 390                 395                 400
   His Ser Gln Arg Arg Gln Asp Ile Leu Asp Leu Trp Ile Tyr His Thr
                   405                 410                 415
   Gln Gly Tyr Phe Pro Asp Trp Gln Asn Tyr Thr Pro Gly Pro Gly Val
               420                 425                 430
   Arg Tyr Pro Leu Thr Phe Gly Trp Cys Tyr Lys Leu Val Pro Val Glu
           435                 440                 445
   Pro Asp Lys Val Glu Glu Ala Asn Lys Gly Glu Asn Thr Ser Leu Leu
       450                 455                 460
   His Pro Val Ser Leu His Gly Met Asp Asp Pro Glu Arg Glu Val Leu
   465                 470                 475                 480
   Glu Trp Arg Phe Asp Ser His Leu Ala Phe His His Val Ala Arg Glu
                   485                 490                 495
   Leu His Pro Glu Tyr Phe Lys Asn Cys
               500                 505
<210> 9
   <211> 1689
   <212> DNA
   <213> Artificial Sequence
<220>
   <223> nucleotide sequence of RT insert of p707-7-RT3
<400> 9
   atgggcccca tcagtcccat cgagaccgtg ccggtgaagc tgaaacccgg gatggacggc 60
   cccaaggtca agcagtggcc actcaccgag gagaagatca aggccctggt ggagatctgc 120
   accgagatgg agaaagaggg caagatcagc aagatcgggc ctgagaaccc atacaacacc 180
   cccgtgtttg ccatcaagaa gaaggacagc accaagtggc gcaagctggt ggatttccgg 240
   gagctgaata agcggaccca ggatttctgg gaggtccagc tgggcatccc ccatccggcc 300
   ggcctgaaga agaagaagag cgtgaccgtg ctggacgtgg gcgacgctta cttcagcgtc 360
   cctctggacg aggactttag aaagtacacc gcctttacca tcccatctat caacaacgag 420
   acccctggca tcagatatca gtacaacgtc ctcccccagg gctggaaggg ctctcccgcc 480
   attttccaga gctccatgac caagatcctg gagccgtttc ggaagcagaa ccccgatatc 540
   gtcatctacc agtacatgga cgacctgtac gtgggctctg acctggaaat cgggcagcat 600
   cgcacgaaga ttgaggagct gaggcagcat ctgctgagat ggggcctgac cactccggac 660
   aagaagcatc agaaggagcc gccattcctg tggatgggct acgagctcca tcccgacaag 720
   tggaccgtgc agcctatcgt cctccccgag aaggacagct ggaccgtgaa cgacatccag 780
   aagctggtgg gcaagctcaa ctgggctagc cagatctatc ccgggatcaa ggtgcgccag 840
   ctctgcaagc tgctgcgcgg caccaaggcc ctgaccgagg tgattcccct cacggaggaa 900
   gccgagctcg agctggctga gaaccgggag atcctgaagg agcccgtgca cggcgtgtac 960
   tatgacccct ccaaggacct gatcgccgaa atccagaagc agggccaggg gcagtggaca 1020
   taccagattt accaggagcc tttcaagaac ctcaagaccg gcaagtacgc ccgcatgagg 1080
   ggcgcccaca ccaacgatgt caagcagctg accgaggccg tccagaagat cacgaccgag 1140
   tccatcgtga tctgggggaa gacacccaag ttcaagctgc ctatccagaa ggagacctgg 1200
   gagacgtggt ggaccgaata ttggcaggcc acctggattc ccgagtggga gttcgtgaat 1260
   acacctcctc tggtgaagct gtggtaccag ctcgagaagg agcccatcgt gggcgcggag 1320
   acattctacg tggacggcgc ggccaaccgc gaaacaaagc tcgggaaggc cgggtacgtc 1380
   accaaccggg gccgccagaa ggtcgtcacc ctgaccgaca ccaccaacca gaagacggag 1440
   ctgcaggcca tctatctcgc tctccaggac tccggcctgg aggtgaacat cgtgacggac 1500
   agccagtacg cgctgggcat tattcaggcc cagccggacc agtccgagag cgaactggtg 1560
   aaccagatta tcgagcagct gatcaagaaa gagaaggtct acctcgcctg ggtcccggcc 1620
   cataagggca ttggcggcaa cgagcaggtc gacaagctgg tgagtgcggg gattagaaag 1680
   gtgctgtaa                                                         1689
<210> 10
   <211> 562
   <212> PRT
   <213> Artificial Sequence
<220>
   <223> amino acid sequence of RT insert of p7077-RT3
<400> 10
   Met Gly Pro Ile Ser Pro Ile Glu Thr Val Ser Val Lys Leu Lys Pro
   1               5                   10                  15
   Gly Met Asp Gly Pro Lys Val Lys Gln Trp Pro Leu Thr Glu Glu Lys
               20                  25                  30
   Ile Lys Ala Leu Val Glu Ile Cys Thr Glu Met Glu Lys Glu Gly Lys
           35                  40                  45
   Ile Ser Lys Ile Gly Pro Glu Asn Pro Tyr Asn Thr Pro Val Phe Ala
       50                  55                  60
   Ile Lys Lys Lys Asp Ser Thr Lys Trp Arg Lys Leu Val Asp Phe Arg
   65                  70                  75                  80
   Glu Leu Asn Lys Arg Thr Gln Asp Phe Trp Glu Val Gln Leu Gly Ile
                   85                  90                  95
   Pro His Pro Ala Gly Leu Lys Lys Lys Lys Ser Val Thr Val Leu Asp
               100                 105                 110
   Val Gly Asp Ala Tyr Phe Ser Val Pro Leu Asp Glu Asp Phe Arg Lys
           115                 120                 125
   Tyr Thr Ala Phe Thr Ile Pro Ser Ile Asn Asn Glu Thr Pro Gly Ile
       130                 135                 140
   Arg Tyr Gln Tyr Asn Val Leu Pro Gln Gly Trp Lys Gly Ser Pro Ala
   145                 150                 155                 160
   Ile Phe Gln Ser Ser Met Thr Lys Ile Leu Glu Pro Phe Arg Lys Gln
                   165                 170                 175
   Asn Pro Asp Ile Val Ile Tyr Gln Tyr Met Asp Asp Leu Tyr Val Gly
               180                 185                 190
   Ser Asp Leu Glu Ile Gly Gln His Arg Thr Lys Ile Glu Glu Leu Arg
           195                 200                 205
   Gln His Leu Leu Arg Trp Gly Leu Thr Thr Pro Asp Lys Lys His Gln
       210                 215                 220
   Lys Glu Pro Pro Phe Leu Trp Met Gly Tyr Glu Leu His Pro Asp Lys
   225                 230                 235                 240
   Trp Thr Val Gln Pro Ile Val Leu Pro Glu Lys Asp Ser Trp Thr Val
                   245                 250                 255
   Asn Asp Ile Gln Lys Leu Val Gly Lys Leu Asn Trp Ala Ser Gln Ile
               260                 265                 270
   Tyr Pro Gly Ile Lys Val Arg Gln Leu Cys Lys Leu Leu Arg Gly Thr
           275                 280                 285
   Lys Ala Leu Thr Glu Val Ile Pro Leu Thr Glu Glu Ala Glu Leu Glu
       290                 295                 300
   Leu Ala Glu Asn Arg Glu Ile Leu Lys Glu Pro Val His Gly Val Tyr
   305                 310                 315                 320
   Tyr Asp Pro Ser Lys Asp Leu Ile Ala Glu Ile Gln Lys Gln Gly Gln
                   325                 330                 335
   Gly Gln Trp Thr Tyr Gln Ile Tyr Gln Glu Pro Phe Lys Asn Leu Lys
               340                 345                 350
   Thr Gly Lys Tyr Ala Arg Met Arg Gly Ala His Thr Asn Asp Val Lys
           355                 360                 365
   Gln Leu Thr Glu Ala Val Gln Lys Ile Thr Thr Glu Ser Ile Val Ile
       370                 375                 380
   Trp Gly Lys Thr Pro Lys Phe Lys Leu Pro Ile Gln Lys Glu Thr Trp
   385                 390                 395                 400
   Glu Thr Trp Trp Thr Glu Tyr Trp Gln Ala Thr Trp Ile Pro Glu Trp
                   405                 410                 415
   Glu Phe Val Asn Thr Pro Pro Leu Val Lys Leu Trp Tyr Gln Leu Glu
               420                 425                 430
   Lys Glu Pro Ile Val Gly Ala Glu Thr Phe Tyr Val Asp Gly Ala Ala
           435                 440                 445
   Asn Arg Glu Thr Lys Leu Gly Lys Ala Gly Tyr Val Thr Asn Arg Gly
       450                 455                 460
   Arg Gln Lys Val Val Thr Leu Thr Asp Thr Thr Asn Gln Lys Thr Glu
   465                 470                 475                 480
   Leu Gln Ala Ile Tyr Leu Ala Leu Gln Asp Ser Gly Leu Glu Val Asn
                   485                 490                 495
   Ile Val Thr Asp Ser Gln Tyr Ala Leu Gly Ile Ile Gln Ala Gln Pro
               500                 505                 510
   Asp Gln Ser Glu Ser Glu Leu Val Asn Gln Ile Ile Glu Gln Leu Ile
           515                 520                 525
   Lys Lys Glu Lys Val Tyr Leu Ala Trp Val Pro Ala His Lys Gly Ile
       530                 535                 540
   Gly Gly Asn Glu Gln Val Asp Lys Leu Val Ser Ala Gly Ile Arg Lys
   545                 550                 555                 560
   Val Leu
<210> 11
   <211> 1689
   <212> DNA
   <213> Artificial Sequence
<220>
   <223> nucleotide sequence of the coding insert in p73i-RT3
<400> 11
   atgggcccca tcagtcccat cgagaccgtg ccggtgaagc tgaaacccgg gatggacggc 60
   cccaaggtca agcagtggcc actcaccgag gagaagatca aggccctggt ggagatctgc 120
   accgagatgg agaaagaggg caagatcagc aagatcgggc ctgagaaccc atacaacacc 180
   cccgtgtttg ccatcaagaa gaaggacagc accaagtggc gcaagctggt ggatttccgg 240
   gagctgaata agcggaccca ggatttctgg gaggtccagc tgggcatccc ccatccggcc 300
   ggcctgaaga agaagaagag cgtgaccgtg ctggacgtgg gcgacgctta cttcagcgtc 360
   cctctggacg aggactttag aaagtacacc gcctttacca tcccatctat caacaacgag 420
   acccctggca tcagatatca gtacaacgtc ctcccccagg gctggaaggg ctctcccgcc 480
   attttccaga gctccatgac caagatcctg gagccgtttc ggaagcagaa ccccgatatc 540
   gtcatctacc agtacatgga cgacctgtac gtgggctctg acctggaaat cgggcagcat 600
   cgcacgaaga ttgaggagct gaggcagcat ctgctgagat ggggcctgac cactccggac 660
   aagaagcatc agaaggagcc gccattcctg tggatgggct acgagctcca tcccgacaag 720
   tggaccgtgc agcctatcgt cctccccgag aaggacagct ggaccgtgaa cgacatccag 780
   aagctggtgg gcaagctcaa ctgggctagc cagatctatc ccgggatcaa ggtgcgccag 840
   ctctgcaagc tgctgcgcgg caccaaggcc ctgaccgagg tgattcccct cacggaggaa 900
   gccgagctcg agctggctga gaaccgggag atcctgaagg agcccgtgca cggcgtgtac 960
   tatgacccct ccaaggacct gatcgccgaa atccagaagc agggccaggg gcagtggaca 1020
   taccagattt accaggagcc tttcaagaac ctcaagaccg gcaagtacgc ccgcatgagg 1080
   ggcgcccaca ccaacgatgt caagcagctg accgaggccg tccagaagat cacgaccgag 1140
   tccatcgtga tctgggggaa gacacccaag ttcaagctgc ctatccagaa ggagacctgg 1200
   gagacgtggt ggaccgaata ttggcaggcc acctggattc ccgagtggga gttcgtgaat 1260
   acacctcctc tggtgaagct gtggtaccag ctcgagaagg agcccatcgt gggcgcggag 1320
   acattctacg tggacggcgc ggccaaccgc gaaacaaagc tcgggaaggc cgggtacgtc 1380
   accaaccggg gccgccagaa ggtcgtcacc ctgaccgaca ccaccaacca gaagacggag 1440
   ctgcaggcca tctatctcgc tctccaggac tccggcctgg aggtgaacat cgtgacggac 1500
   agccagtacg cgctgggcat tattcaggcc cagccggacc agtccgagag cgaactggtg 1560
   aaccagatta tcgagcagct gatcaagaaa gagaaggtct acctcgcctg ggtcccggcc 1620
   cataagggca ttggcggcaa cgagcaggtc gacaagctgg tgagtgcggg gattagaaag 1680
   gtgctgtaa                                                         1689
<210> 12
   <211> 561
   <212> PRT
   <213> Artificial Sequence
<220>
   <223> amino acid sequence of the coding insert in p73i-RT3
<400> 12.
   Met Gly Pro Ile Ser Pro Ile Glu Thr Val Ser Val Lys Leu Lys Pro
   1               5                   10                  15
   Gly Met Asp Gly Pro Lys Val Lys Gln Pro Leu Thr Glu Glu Lys Ile
               20                  25                  30
   Lys Ala Leu Val Glu Ile Cys Thr Glu Met Glu Lys Glu Gly Lys Ile
           35                  40                  45
   Ser Lys Ile Gly Pro Glu Asn Pro Tyr Asn Thr Pro Val Phe Ala Ile
       50                  55                  60
   Lys Lys Lys Asp Ser Thr Lys Trp Arg Lys Leu Val Asp Phe Arg Glu
   65                  70                  75                  80
   Leu Asn Lys Arg Thr Gln Asp Phe Trp Glu Val Gln Leu Gly Ile Pro
                   85                  90                  95
   His Pro Ala Gly Leu Lys Lys Lys Lys Ser Val Thr Val Leu Asp Val
               100                 105                 110
   Gly Asp Ala Tyr Phe Ser Val Pro Leu Asp Glu Asp Phe Arg Lys Tyr
           115                 120                 125
   Thr Ala Phe Thr Ile Pro Ser Ile Asn Asn Glu Thr Pro Gly Ile Arg
       130                 135                 140
   Tyr Gln Tyr Asn Val Leu Pro Gln Gly Trp Lys Gly Ser Pro Ala Ile
   145                 150                 155                 160
   Phe Gln Ser Ser Met Thr Lys Ile Leu Glu Pro Phe Arg Lys Gln Asn
                   165                 170                 175
   Pro Asp Ile Val Ile Tyr Gln Tyr Met Asp Asp Leu Tyr Val Gly Ser
               180                 185                 190
   Asp Leu Glu Ile Gly Gln His Thr Arg Lys Ile Glu Glu Leu Arg Gln
           195                 200                 205
   His Leu Leu Arg Trp Gly Leu Thr Thr Pro Asp Lys Lys His Gln Lys
       210                 215                 220
   Glu Pro Pro Phe Leu Trp Met Gly Tyr Glu Leu His Pro Asp Lys Trp
   225                 230                 235                 240
   Thr Val Gln Pro Ile Val Leu Pro Glu Lys Asp Ser Trp Thr Val Asn
                   245                 250                 255
   Asp Ile Gln Lys Leu Val Gly Lys Leu Asn Trp Ala Ser Gln Ile Tyr
               260                 265                 270
   Pro Gly Ile Lys Val Arg Gln Leu Cys Lys Leu Leu Arg Gly Thr Lys
   275 280 285
   Ala Leu Thr Glu Val Ile Pro Leu Thr Glu Glu Ala Glu Leu Glu Leu
   290 295 300
   Ala Glu Asn Arg Glu Ile Leu Lys Glu Pro Val His Gly Val Tyr Tyr
   305 310 315 320
   Asp Pro Ser Lys Asp Leu Ile Ala Glu Ile Gln Lys Gln Gly Gln Gly
   325 330 335
   Gln Trp Thr Tyr Gln Ile Tyr Gln Glu Pro Phe Lys Asn Leu Lys Thr
   340 345 350
   Gly Lys Tyr Ala Arg Met Arg Gly Ala His Thr Asn Asp Val Lys Gln
   355 360 365
   Leu Thr Glu Ala Val Gln Lys Ile Thr Thr Glu Ser Ile Val Ile Trp
   370 375 380
   Gly Lys Thr Pro Lys Phe Lys Leu Pro Ile Gln Lys Glu Thr Trp Glu
   385 390 395 400
   Thr Trp Trp Thr Glu Tyr Trp Gln Ala Thr Trp Ile Pro Glu Trp Glu
   405 410 415
   Phe Val Asn Thr Pro Pro Leu Val Lys Leu Trp Tyr Gln Leu Glu Lys
   420 425 430
   Glu Pro Ile Val Gly Ala Glu Thr Phe Tyr Val Asp Gly Ala Ala Asn
   435 440 445
   Arg Glu Thr Lys Leu Gly Lys Ala Gly Tyr Val Thr Asn Arg Gly Arg
   450 455 460
   Gln Lys Val Val Thr Leu Thr Asp Thr Thr Asn Gln Lys Thr Glu Leu
   465 470 475 480
   Gln Ala Ile Tyr Leu Ala Leu Gln Asp Ser Gly Leu Glu Val Asn Ile
   485 490 495
   Val Thr Asp Ser Gln Tyr Ala Leu Gly Ile Ile Gln Ala Gln Pro Asp
   500 505 510
   Gln Ser Glu Ser Glu Leu Val Asn Gln Ile Ile Glu Gln Leu Ile Lys
   515 520 525
   Lys Glu Lys Val Tyr Leu Ala Trp Val Pro Ala His Lys Gly Ile Gly
   530 535 540
   Gly Asn Glu Gln Val Asp Lys Leu Val Ser Ala Gly Ile Arg Lys Val
   545 550 555 560
   Leu